# EUROPEAN PATENT APPLICATION

(11) **EP 3 995 513 A1**
(43) Date of publication of application: **11.05.2022**
(21) Application number: 20834320.2
(22) Date of filing: 24.06.2020
(51) Int. Cl.: C07K 19/00, C12N 15/62, C12N 15/63, A61K 38/36, A61P 7/04

(54) **IMPROVED FIX FUSION PROTEIN AND CONJUGATE AND USE THEREOF**

(30) Priority: 02.07.2019 CN 201910591280
(71) Applicant: Jiangsu Gensciences Inc., Nantong, Jiangsu 226000 (CN)
(72) Inventor: SU, Hongsheng, Beijing 100176 (CN); CHEN, Xian, Beijing 100176 (CN); MO, Weichuan, Beijing 100176 (CN); ZHU, Luyan, Beijing 100176 (CN); YAN, Haixia, Beijing 101116 (CN); REN, Zijia, Zhengzhou, Henan 451162 (CN); WANG, Yali, Beijing 100176 (CN)
(74) Representative: Bosia, Alessandra
(86) International application number: PCT/CN2020/097911
(87) International publication number: WO 2021/000767

(57) **Abstract**

Provided are a blood coagulation factor IX fusion protein with a prolonged circulation half-life, a conjugate thereof, a pharmaceutical composition containing same and the use of same in treating hemorrhagic diseases (such as hemophilia B).

## Description

### CROSS REFERENCE TO RELATED APPLICATIONS

This application claims the priority of Chinese Patent Application No. 201910591280.0, filed with the China National Intellectual Property Administration on July 02, 2019.

### FIELD

The present invention relates to a biologically active fusion protein with extended half-life *in vivo* and a conjugate thereof with a polymer, and specifically relates to human coagulation factor IX with extended half-life *in vivo,* a pharmaceutical composition containing the same, and the use of the same in the treatment of hemorrhagic diseases (such as hemophilia B).

### BACKGROUND

Hemophilia, a severe coagulation dysfunction caused by the deficiency of certain coagulation factors in the blood, is a set of hereditary hemorrhagic diseases, including hemophilia A (factor VIII, AHG deficiency) and hemophilia B ( factor IX, PTC deficiency).

Hemophilia B is a serious coagulation dysfunction disease caused by the deficiency of human coagulation factor IX (hFIX). The gene encoding hFIX (*hFIX*) is located at the far end of the long arm of human X chromosome, the Xq27.1 region, with a full length of 34 kb, containing 8 exons and 7 introns. The cDNAof hFIX has a full length of 2802 bp, of which the coding region is 1383 bp (Genbank accession number: NM_000133). The severe patients regularly have an activity of factor IX lower than 1% of normal, who often suffered from spontaneous bleeding which leads to muscle hematoma or joint deformity.

Recombinant formulations for the prevention and treatment of hemophilia are increasingly recognized by the medical system and patients for their safety. For hemophilia B, infusion of factor IX preparation (which is usually recombinant factor IX protein expressed *in vitro* at present) is the only effective treatment method to supplement the coagulation factor IX level in the patient's body. However, since peptide drugs generally have characteristics such as short half-life, poor physical and chemical stability, and easy degradation by various proteases in the body, these drugs usually require multiple injections within a day, resulting in frequent medication, which brings a greater physical, mental and economic burden to patients, limiting the patient's medication compliance. Therefore, there is an urgent need for new drugs in this field to extend the circulation half-life in plasma and increase the systemic drug exposure.

### SUMMARY

After years of research, the inventors invent a fusion protein of coagulation factor IX and Fc, which can be further modified by a group to be a conjugate, including PEG, and has a significantly extended circulation half-life compared with FIX polypeptides.

Specifically, the first aspect of the present invention provides a fusion protein of coagulation factor IX (FIX), comprising a coagulation factor IX active moiety and a fusion partner (FP) capable of extending the half-life of the fusion protein, wherein the coagulation factor IX active moiety and the fusion partner are linked directly or through a first linker L1; preferably, the first linker L1 is a peptide containing 1, 2, 3, 4, 5, 6, 7, 8, 9, 10, or more amino acids; more preferably, the first linker L1 comprises a flexible peptide and/or a rigid unit, and the flexible peptide is a peptide containing glycine (Gly, G), serine (Ser, S), alanine (Ala, A) and/or threonine (Thr, T), for example, the flexible peptide is (GS)ₘ(GGS)ₙ(GGGS)ₒ(GGGGS)ₚ, where m, n, o and p are independently selected from integers from 0 to 50, such as 0, 1, 2, 3, 4, 5, 6, 7, 8, 9, 10, 11, 12, 13, 14, 15, 16, 17, 18, 19 or 20, preferably m, n, o and p are not all 0.

In one embodiment of the present invention, the coagulation factor IX active moiety is derived from human, such as full-length or truncated human coagulation factor IX; the full-length or truncated human coagulation factor IX may contain one or more amino acid mutations, provided that it still retains the FIX activity, for example, the coagulation factor IX active moiety comprises the amino acid sequence shown in SEQ ID NO: 1, or has at least 80%, 85%, 88%, 89%, 90%, 91%, 92%, 93%, 94%, 95%, 96%, 97%, 98%, 99% or higher identity to the amino acid sequence shown in SEQ ID NO: 1.

In another embodiment, the rigid unit is a peptide containing 1, 2, 3, 4, 5, 6, 7, 8, 9, 10 or more proline (Pro, P), preferably, the rigid unit comprises an amino acid sequence selected from the group consisting of:
VAPPPALPAPVRLPGPA (SEQ ID NO: 3),
VAPPPALPAVAPPPALPA (SEQ ID NO: 4),
VAPPPALPAVAPPPALPAVAPPPALPAPVRLPGPA (SEQ ID NO: 5),
VAPPPALPAPVRLPGPAVAPPPALPAVAPPPALPA (SEQ ID NO: 6), and
VAPPPALPAVAPPPALPAGSVAPPPALPAVAPPPALPA (SEQ ID NO: 7).

Preferably, the first linker L1 is selected from the group consisting of:
GGGGSGGGGSGGGGSGGGGSGGGGSVAPPPALPAPVRLPGPA (SEQ ID NO: 8),
GGGGSGGGGSGGGGSGGGGSGGGGSVAPPPALPAVAPPPALPA (SEQ ID NO: 9), 
GGGGSGGGGSGGGGSGGGGSGGGGSVAPPPALPAVAPPPALPAVAPPPALPAPVRLPGP
A (SEQ ID NO: 10),
 
GGGGSGGGGSGGGGSGGGGSGGGGSVAPPPALPAPVRLPGPAVAPPPALPAVAPPPALP
A (SEQ ID NO: 11),
 and

GGGGSGGGGSGGGGSGGGGSGGGGSVAPPPALPAVAPPPALPAGSVAPPPALPAVAPPPA
LPA (SEQ ID NO: 12).

In yet another embodiment, the fusion partner is a full-length, truncated or variant form of immunoglobulin Fc fragment, albumin, transferrin or XTEN; the fusion partner is, for example, derived from human, for example, the Fc fragment is derived from a human immunoglobulin Fc fragment; preferably, the Fc fragment is composed of one to four domains selected from the group consisting of CH1 domain, CH2 domain, CH3 domain and CH4 domain; preferably, the Fc fragment is derived from the Fc fragment of IgG, IgA, IgD, IgE, or IgM, more preferably IgG Fc fragment; more preferably, the Fc fragment is derived from the Fc fragment of IgG1, IgG2, IgG3, or IgG4, and more preferably, the IgG Fc fragment has reduced ADCC effect and/or CDC effect and/or enhanced binding affinity to FcRn.

In yet another embodiment, wherein the fusion partner (FP) is also linked directly or via a second linker L2 to a peptide P containing a Sortase recognition site, and the Sortase is, for example, Sortase A or Sortase B; preferably, the P contains the core recognition site LPXTG of Sortase A, wherein X is any amino acid, and the sequence of P is, such as LPETG, LPETGG or LPETGGG; more preferably, the amino acid sequence of P further contains an affinity tag linked to the Sortase recognition site, and the amino acid sequence of P is, for example, LPETGGHHHHHH or LPETGGWSHPQFEK.

In yet another embodiment, the second linker L2 is a peptide containing 1, 2, 3, 4, 5, 6, 7, 8, 9, 10 or more amino acids; preferably, the second linker L2 is a flexible peptide fragment containing glycine (Gly, G), serine (Ser, S), alanine (Ala, A) and/or threonine (Thr, T), such as (GS)_{w}(GGS)ₓ(GGGS)_{y}(GGGGS)_{z}, where w, x, y and z are independently selected from integers from 0 to 50, such as 0, 1, 2, 3, 4, 5, 6, 7, 8, 9, 10, 11, 12, 13, 14, 15, 16, 17, 18, 19 or 20, preferably w, x, y and z are not all 0; preferably, the second linker L2 is selected from the group consisting of GGGGS (SEQ ID NO: 14), GGGGSGGGGS (SEQ ID NO: 15) and GSGGSGGGGS (SEQ ID NO: 16).

In another embodiment, the fusion protein has a structure of FIX-L1-FP-L2-P, wherein FIX, L1, L2, FP, and P have the same definitions as in the above embodiments, and wherein either or both of L1 and L2 may not be present.

In yet another embodiment, the fusion protein comprises the sequence shown in SEQ ID NO: 18, 20, 22, 24, 26 or 28, or encoded by the nucleic acid sequence shown in SEQ ID NO: 19, 21, 23, 25, 27, or 29.

Further, the present invention also provides a nucleic acid molecule encoding the fusion protein of any one of the above embodiments and an expression vector comprising the nucleic acid molecule.

The second aspect of the present invention provides a conjugate, wherein the conjugate is formed by linking a hydrophilic polymer to the end of the fusion protein according to any embodiment of the first aspect. In the conjugate, one, two or more hydrophilic polymers are attached to the end of the fusion protein according to any embodiment of the first aspect, preferably, the hydrophilic polymer is attached to the Sortase recognition site via a transpeptidation reaction; in particular, the fusion protein may be in a form of monomer or dimer.

In one embodiment, the one, two or more hydrophilic polymers are each independently selected from polysaccharide and polyalkylene glycol, such as polypropylene glycol and polyethylene glycol; the polyalkylene glycol may be end-capped, for example, capped with alkoxy group such as methoxy group; and/or the polyalkylene glycol is linear or branched, for example, the polyalkylene glycol is branched, such as branched polyethylene glycol, especially branched polyethylene glycol capped with methoxy group; the molecular weight of the polyalkylene glycol may be ≥1, ≥10, ≥20, ≥30, ≥40, ≥50, ≥60, ≥70, ≥80, ≥90, ≥100, ≥110, ≥120, ≥130, ≥140, ≥150, or ≥160 kDa, for example, 5, 10, 20, 30, 40, 50, 60, 70, 80, 90, or 100 kDa, or a value between any two of the values; and preferably the molecular weight of the hydrophilic polymer is 20 kDa or 40 kDa.

The third aspect of the present invention provides a method for producing the conjugate of the second aspect, comprising contacting the fusion protein of the first aspect of the present invention with Sortase (such as Sortase A or Sortase B) and the hydrophilic polymer, wherein one end of the hydrophilic polymer has an amino group that can be amidated by Sortase.

In one embodiment, the N-terminus of the hydrophilic polymer contains poly-Gly, such as GGGAA.

The fourth aspect of the present invention provides a pharmaceutical composition comprising an effective amount of the fusion protein described in the first aspect and/or the conjugate described in the second aspect, and optionally a pharmaceutically acceptable carrier.

In one embodiment, the pharmaceutical composition is used for the prevention and/or treatment of a hemorrhagic disease, wherein the hemorrhagic disease is preferably selected from hemorrhagic diseases in patients with congenital or acquired deficiency of FIX, and spontaneous or surgical bleeding in patients with hemophilia B.

In yet another embodiment, the pharmaceutical composition is in a form of liquid preparation or lyophilized preparation.

The fifth aspect of the present invention provides use of the fusion protein, conjugate or pharmaceutical composition of the aforementioned aspects for the manufacture of a medicament for the prevention and/or treatment of a hemorrhagic disease, wherein the hemorrhagic disease is preferably selected from hemorrhagic diseases in patients with congenital or acquired deficiency of FIX, and spontaneous or surgical bleeding in patients with hemophilia B.

The sixth aspect of the present invention provides a method for preventing and/or treating a hemorrhagic disease, comprising administering the fusion protein, conjugate or pharmaceutical composition of the aforementioned aspects to a subject in need thereof. The hemorrhagic disease is preferably selected from hemorrhagic diseases in patients with congenital or acquired deficiency of FIX, and spontaneous or surgical bleeding in patients with hemophilia B.

The seventh aspect of the present invention provides a kit comprising the fusion protein, conjugate or pharmaceutical composition of the aforementioned aspects, and optionally instructions for use.

The eighth and ninth aspects of the present invention respectively provide a nucleic acid molecule encoding the fusion protein of the first aspect and an expression vector comprising the nucleic acid molecule.

### BRIEF DESCRIPTION OF DRAWINGS

FIG. 1A shows the structure of the FIX/PACE (Furin) bicistronic expression vector.
FIG. 1B shows the plasmid map of pcDNA3.1-Hyg-VKGC.
FIG. 2 shows the detection results of SDS-PAGE (A) and SEC-HPLC (B) of the purified FIX-C1-Fc-L2-P fusion protein.
FIG. 3 shows the detection results of the *in vitro* activity of each FIX-L1-Fc-L2-P fusion protein and FIX-C1-Fc-L2-P conjugate.
FIG. 4 shows the SEC-HPLC detection result of the 40kDa PEG conjugate after the conjugation reaction.
FIG. 5 shows the SEC-HPLC detection results of the conjugate after purification and recovery.
FIG. 6 shows the PK detection results of the conjugate FIX-C1-Fc-L2-PEG in hemophilia B model mice, and the table below shows the T1/2 (elimination phase half-life), Cmax, AUC0-inf (area under the curve), MRT (mean residence time) and CL (clearance rate).

### DETAILED DESCRIPTION

In order to make the objectives, technical solutions, and advantages of the present invention clearer, the following further describes the present invention in detail with reference to the drawings and embodiments. Obviously, the embodiments to be described merely are a part rather than all of the embodiments of present invention.

### Definitions

Coagulation factor IX, also called factor IX, FIX, is a vitamin K-dependent coagulation factor with structural similarities to factor VII, prothrombin, factor X, and protein C.

In the present invention, the term "coagulation factor IX active moiety" refers to the moiety of the fusion protein that exhibits coagulation factor IX activity. The term "coagulation factor IX" can refer to its natural wild-type protein (the wild-type amino acid sequence is shown in SEQ ID NO:1, and the reference wild-type nucleic acid coding sequence can be found in Genbank accession number: NM_000133, specifically as shown in SEQ ID NO: 2), and also encompasses its variant forms, for example, a variant protein generated by one or more (such as 2, 3, 4 or 5) amino acid substitutions, deletions or insertions, while retaining the activity of coagulation factor IX.








 (SEQ
ID NO: 1).

ATGCAGCGCGTGAACATGATCATGGCAGAATCACCAGGCCTCATCACCATCTGCCTT
TTAGGATATCTACTCAGTGCTGAATGTACAGTTTTTCTTGATCATGAAAACGCCAAC
AAAATTCTGAATCGGCCAAAGAGGTATAATTCAGGTAAATTGGAAGAGTTTGTTCA
AGGGAACCTTGAGAGAGAATGTATGGAAGAAAAGTGTAGTTTTGAAGAAGCACGA
GAAGTTTTTGAAAACACTGAAAGAACAACTGAATTTTGGAAGCAGTATGTTGATGG
AGATCAGTGTGAGTCCAATCCATGTTTAAATGGCGGCAGTTGCAAGGATGACATTAA
TTCCTATGAATGTTGGTGTCCCTTTGGATTTGAAGGAAAGAACTGTGAATTAGATGT
AACATGTAACATTAAGAATGGCAGATGCGAGCAGTTTTGTAAAAATAGTGCTGATAA
CAAGGTGGTTTGCTCCTGTACTGAGGGATATCGACTTGCAGAAAACCAGAAGTCCT
GTGAACCAGCAGTGCCATTTCCATGTGGAAGAGTTTCTGTTTCACAAACTTCTAAG

CTCACCCGTGCTGAGACTGTTTTTCCTGATGTGGACTATGTAAATTCTACTGAAGCT
GAAACCATTTTGGATAACATCACTCAAAGCACCCAATCATTTAATGACTTCACTCGG
GTTGTTGGTGGAGAAGATGCCAAACCAGGTCAATTCCCTTGGCAGGTTGTTTTGAA
TGGTAAAGTTGATGCATTCTGTGGAGGCTCTATCGTTAATGAAAAATGGATTGTAAC
TGCTGCCCACTGTGTTGAAACTGGTGTTAAAATTACAGTTGTCGCAGGTGAACATA
ATATTGAGGAGACAGAACATACAGAGCAAAAGCGAAATGTGATTCGAATTATTCCTC
ACCACAACTACAATGCAGCTATTAATAAGTACAACCATGACATTGCCCTTCTGGAAC
TGGACGAACCCTTAGTGCTAAACAGCTACGTTACACCTATTTGCATTGCTGACAAGG
AATACACGAACATCTTCCTCAAATTTGGATCTGGCTATGTAAGTGGCTGGGGAAGAG
TCTTCCACAAAGGGAGATCAGCTTTAGTTCTTCAGTACCTTAGAGTTCCACTTGTTG
ACCGAGCCACATGTCTTCGATCTACAAAGTTCACCATCTATAACAACATGTTCTGTG
CTGGCTTCCATGAAGGAGGTAGAGATTCATGTCAAGGAGATAGTGGGGGACCCCAT
GTTACTGAAGTGGAAGGGACCAGTTTCTTAACTGGAATTATTAGCTGGGGTGAAGA
GTGTGCAATGAAAGGCAAATATGGAATATATACCAAGGTATCCCGGTATGTCAACTG
GATTAAGGAAAAAACAAAGCTCACTTAA (SEQ ID NO: 2).

In one embodiment, the variant protein is at least 90% identical to the sequence of SEQ ID NO: 1. In another embodiment, the variant protein is at least 95%, such as 96%, 97%, 98%, or 99% identical to the sequence of SEQ ID NO: 1. As used herein, unless otherwise specified, any specific position mentioned refers to the corresponding position in SEQ ID NO: 1.

The present invention is not limited to the sequences described herein. FIX variants are, for example, disclosed in US5521070 (wherein the tyrosine in the first position is substituted by alanine) and WO2007/135182 (wherein one or more natural amino acid residues in FIX are substituted by cysteine residues). The references are incorporated herein by reference in their entirety. Therefore, FIX variants are well known in the art, and the present invention encompasses those variant forms known or will be developed or discovered in the future.

### Fusion protein

The term "fusion partner", FP, refers to a polypeptide that is fused with a target polypeptide (a polypeptide whose circulation half-life is desired to be extended). The fusion partner can affect the function properties of the fusion protein through a variety of different mechanisms, such as extending the half-life of the target polypeptide *in vivo.*

In one embodiment, the fusion partner delays the clearance of FIX *in vivo* by interacting with the neonatal Fc receptor (FcRn). In another embodiment, the fusion partner is an immunoglobulin Fc fragment, albumin, transferrin, XTEN, or a portion thereof.

The Fc fragment of immunoglobulin is safe to be used as a pharmaceutical carrier because it is a biodegradable polypeptide that can be metabolized in the body. In addition, compared with the entire immunoglobulin molecule, the Fc region of immunoglobulin has a relatively low molecular weight, which is beneficial to the preparation, purification and production of the conjugate. Since the immunoglobulin Fc fragment does not contain Fab fragment (its amino acid sequence varies according to the antibody subclass and is therefore highly heterogeneous), it is expected that the immunoglobulin Fc region can greatly increase the homogeneity of the substance and have low antigenicity.

The term "Fc region/fragment of immunoglobulin" refers to a protein containing heavy chain constant region 2 (CH2) and heavy chain constant region 3 (CH3) of immunoglobulin, but not variable regions of heavy chain and light chain of an immunoglobulin. Furthermore, the Fc fragment used in the present invention may further contain the hinge region in the heavy chain constant region, and/or part or all of the heavy chain constant region 1 (CH1) and/or the light chain constant region 1 (CL1) without variable regions of heavy chain and light chain, as long as it has a physiological function that is basically similar to or better than that of natural form.

In some embodiments, the immunoglobulin Fc fragment of the present invention may comprise 1) CH1 domain, CH2 domain, CH3 domain and CH4 domain; 2) CH1 domain and CH2 domain; 3) CH1 domain and CH3 domain; 4) CH2 domain and CH3 domain; 5) the combination of one or more domains with (part or all of) the immunoglobulin hinge region; or 6) a dimer of any domains of heavy chain constant region and light chain constant region.

In addition, the immunoglobulin Fc fragment may be an Fc region derived from IgG, IgA, IgD, IgE, and IgM, or prepared by a combination or hybrid thereof. Preferably, it is derived from IgG or IgM (because they are two of the most abundant proteins in human blood). In one embodiment, considering that IgG antibodies have a longer half-life, the IgG Fc domain is preferred.

The Fc fragment can also be modified to improve other functions, for example, to change its ability to induce immune responses by mutations, such as the ability to bind to complement and/or to some Fc receptors, because this is unnecessary for the use of Fc to extend the half-life. It is known that the mutations at positions 234, 235, and 237 in the Fc region of IgG usually lead to reduced binding to FcyRI receptors, and may also lead to reduced binding to FcyRIIa and FcyRIII receptors, but these mutations do not change its binding to FcRn receptors, and can therefore still promote a long circulation half-life through the endocytic recirculation pathway. Preferably, the modified IgG Fc fragment of the fusion protein of the present invention contains one or more of the following mutations, mutations L234A, L235E and G237A lead to decreased affinity for certain Fc receptors, and A330S and P331S lead to decreased C1q-mediated complement binding.

In one embodiment, the coagulation factor IX fusion protein is further linked to a fragment containing a Sortase recognition site, i.e., the "peptide P" described in the present invention.

The term "Sortase", specifically including Sortase A and Sortase B, was first discovered to have the function of anchoring bacterial surface proteins on the cell wall. The "sorting signal" of the surface proteins was identified to consist of three key parts: the LPXTG sequence, the hydrophobic sequence and a tail of positively charged residues in most cases. The LPXTG sequence is very conservative and can be recognized by Sortase, then the cysteine (Cys) of the Sortase acts as a nucleophilic group to attack the peptide bond between the threonine and glycine of the C-terminal motif LPXTG of the substrate (such as surface protein), causing the peptide bond to cleave (acylation), which in turn produces an acylase intermediate, and finally the peptide is covalently linked to the cell wall or fimbriae subunit to function (deacylation)

In one embodiment of the present invention, Sortase A is utilized for site-directed coupling of PEG. In a specific embodiment, the Sortase A core recognition sites LPETG, LPETGG, and LPETGGG are used. In another specific embodiment, different affinity tags can be added to the recognition site, such as LPETGGHHHHHH, LPETGGWSHPQFEK, etc.

In another embodiment, the coagulation factor IX fusion protein of the present invention is produced by recombinant methods, comprising, for example, expressing the protein in a suitable prokaryotic or eukaryotic host cell, and isolating the coagulation factor IX fusion protein of the present invention by conventional techniques. For example, the nucleotide sequence encoding the peptide can be synthesized by chemical synthesis method first, and then the sequence can be cloned into a suitable expression vector for expression under the control of a suitable promoter. Alternatively, mutagenesis methods such as PCR mutagenesis can be employed to obtain the nucleotide sequence encoding coagulation factor IX from wild-type coagulation factor IX, and then the sequence and the sequence of other elements for constructing the fusion protein can be cloned to a suitable expression vector for expression under the control of a suitable promoter. These technologies are completely within the abilities of those of ordinary skill in the art, and there are numerous teachings in the prior art.

Suitable eukaryotic host cells comprise mammalian cells, such as CHO, COS, HEK 293, BHK, SK-Hep and HepG2. The cells are preferably growing under conditions suitable for expressing the coagulation factor IX fusion protein of the present invention. As for the reagents and conditions used to produce or isolate the coagulation factor IX fusion protein of the present invention, there are no special restrictions, and any system known in the art or commercially available can be applied. In a preferred embodiment, the coagulation factor IX fusion protein is obtained by methods described in the art.

There are a variety of expression vectors that can be used to prepare the coagulation factor IX fusion protein, which can be selected from eukaryotic and prokaryotic expression vectors. Prokaryotic expression vectors may include, for example, plasmids such as pRSET, pET, and pBAD, in which promoters that can be used include, for example, lac, trc, trp, recA, or araBAD. Eukaryotic expression vectors include: (i) vectors used for expression in yeast, such as pAO, pPIC, pYES, and pMET, in which promoters such as AOX1, GAP, GAL1, AUG1, etc. can be used; (ii) vectors used for expression in insect cells, such as pMT, pAc[delta], plB, pMIB, pBAC, etc., in which promoters such as PH, p10, MT, Ac5, OplE2, gp64, polh, etc. can be used; and (iii) vectors used for expression in mammalian cells, such as pSVL, pCMV, pRc/RSV, pcDNA3, pBPV, etc., and vectors derived from viral systems such as vaccinia virus, adeno-associated virus, herpes virus, retrovirus, and the like, in which promoters such as CMV, SV40, EF-1, UbC, RSV, ADV, BPV and β-actin can be used. In a preferred embodiment, the coagulation factor IX fusion protein is expressed in a prokaryotic or eukaryotic cell system, and codon-optimized sequences are used.

In a preferred embodiment, the sequence for expressing the coagulation factor IX fusion protein contains a propeptide and/or a signal peptide to facilitate the secretion of the coagulation factor IX fusion protein from the cell to the outside of the cell for separation and purification; in order to improve the cleavage efficiency of the propeptide, the yield of FIX and the correct processing, the expression vector was optimized; in a specific embodiment, a FIX/PACE (Furin) bicistronic expression vector was constructed.

In another preferred embodiment, the sequence expressing the coagulation factor IX fusion protein does not contain a propeptide and/or a signal peptide, and instead of being secreted outside the cell, the coagulation factor IX fusion protein is obtained by lysing the cell for separation and purification.

In an embodiment, in the fusion protein of the present invention, the coagulation factor IX protein and the Fc fragment are directly linked. In another embodiment, the coagulation factor IX protein and the Fc fragment are linked through a first linker L1. In another embodiment, the Fc fragment and the fragment containing the Sortase recognition sequence are directly linked. In another embodiment, the Fc fragment and the fragment containing the Sortase recognition sequence are linked through a second linker L2.

In preferred embodiments, the first linker L1 is a peptide containing 1, 2, 3, 4, 5, 6, 7, 8, 9, 10, or more amino acids; more preferably, the first linker L1 comprises a flexible peptide and/or a rigid unit, and the flexible peptide is a peptide containing glycine (Gly, G), serine (Ser, S), alanine (Ala, A) and/or threonine (Thr, T), for example, the flexible peptide is (GS)ₘ(GGS)ₙ(GGGS)ₒ(GGGGS)ₚ, where m, n, o and p are independently selected from integers from 0 to 50, such as 0, 1, 2, 3, 4, 5, 6, 7, 8, 9, 10, 11, 12, 13, 14, 15, 16, 17, 18, 19 or 20, preferably m, n, o and p are not all 0.

The rigid unit is a peptide containing one or more prolines (Pro, P). Proline has a special cyclic structure, which strengthens the restriction on the structure of the peptide chain. The inventors unexpectedly discovered that the inclusion of a rigid unit in the linker can greatly improve the activity of the fusion protein. Preferably, the rigid unit comprises an amino acid sequence selected from the group consisting of:
VAPPPALPAPVRLPGPA (SEQ ID NO: 3),
VAPPPALPAVAPPPALPA (SEQ ID NO: 4),
VAPPPALPAVAPPPALPAVAPPPALPAPVRLPGPA (SEQ ID NO: 5),
VAPPPALPAPVRLPGPAVAPPPALPAVAPPPALPA (SEQ ID NO: 6), and
VAPPPALPAVAPPPALPAGSVAPPPALPAVAPPPALPA (SEQ ID NO: 7).

In another preferred embodiment, the sequence of the first linker L1 is selected from the group consisting of:
GGGGSGGGGSGGGGSGGGGSGGGGSVAPPPALPAPVRLPGPA (SEQ ID NO: 8),
GGGGSGGGGSGGGGSGGGGSGGGGSVAPPPALPAVAPPPALPA (SEQ ID NO: 9), 
GGGGSGGGGSGGGGSGGGGSGGGGSVAPPPALPAVAPPPALPAVAPPPALPAPVRLPGP
A (SEQ ID NO: 10),
 
GGGGSGGGGSGGGGSGGGGSGGGGSVAPPPALPAPVRLPGPAVAPPPALPAVAPPPALP
A (SEQ ID NO: 11),
 and

GGGGSGGGGSGGGGSGGGGSGGGGSVAPPPALPAVAPPPALPAGSVAPPPALPAVAPPPA
LPA (SEQ ID NO: 12).

The second linker L2 is a peptide containing 1, 2, 3, 4, 5, 6, 7, 8, 9, 10 or more amino acids, and is mainly used to separate Fc and the Sortase recognition site to facilitate the recognition and action of Sortase; more preferably, the second linker L2 is a flexible peptide containing glycine (Gly, G), serine (Ser, S), alanine (Ala, A) and/or threonine (Thr, T), such as (GS)_{w}(GGS)ₓ(GGGS)_{y}(GGGGS)_{z}, where w, x, y and z are independently selected from integers from 0 to 50, such as 0, 1, 2, 3, 4, 5, 6, 7, 8, 9, 10, 11, 12, 13, 14, 15, 16, 17, 18, 19 or 20, preferably w, x, y and z are not all 0.

In a preferred embodiment, the sequence of the second linker L2 is selected from the group consisting of GGGGS (SEQ ID NO: 14), GGGGSGGGGS (SEQ ID NO: 15) and GSGGSGGGGS (SEQ ID NO: 16).

Coagulation factor IX fusion protein conjugate or conjugate of coagulation factor IX fusion protein The term "conjugate" refers to a product formed by a polypeptide or a polypeptide variant covalently or non-covalently linked to the hydrophilic polymer modification group described herein, wherein the hydrophilic polymer and the polypeptide can be linked at any position, for example, the appropriate position at the N-terminal, C-terminal or middle part of the polypeptide. Generally, the term "polymer" used herein has the meaning commonly understood by those of ordinary skill in the art, and refers to both the polymer itself and its terminal-modified derivatives, unless explicitly stated otherwise.

In addition, for polymer such as polyethylene glycol, there are many ways to determine their molecular weight. Since polymers are composed of molecules with different degrees of polymerization within a certain distribution range, the molecular weights of the polymers are generally represented by their average molecular weight, specifically, number-average molecular weight or weight-average molecular weight. The number-average molecular weight refers to the sum of the product of the number fraction of each molecule with different molecular weight and its corresponding molecular weight; and the weight-average molecular weight refers to the sum of the product of the weight fraction of each different molecule and its corresponding molecular weight. Although the number-average molecular weight and weight-average molecular weight may have some deviations when the degree of polymerization of the polymers differs greatly, for polymers with a narrow distribution range, the two tend to be equal. For polymers such as polyethylene glycol mentioned herein, when referring to its molecular weight, it can be either weight-average molecular weight or number-average molecular weight.

The coagulation factor IX fusion protein of the present invention can be conjugated with one or more hydrophilic polymers to form a coagulation factor IX fusion protein conjugate. The polymer is preferably physiologically acceptable, which includes those soluble in an aqueous solution or suspension and without negative effects such as side effects on mammals after administration of the coagulation factor IX fusion protein conjugate in a pharmaceutically effective amount. The polymers that can be used in the present invention are not particularly limited. The polymers generally preferably have 2 to about 3000 repeating units. The polymer group can be selected from natural or synthetic polymers, examples of which include, but are not limited to, for example, polysaccharides, polyalkylene glycols such as polyethylene glycol (PEG), polypropylene glycol (PPG), polyethylene oxide (PEO), copolymer of ethylene glycol and propylene glycol, polyvinyl alcohol and any combination thereof. In a preferred embodiment, the coagulation factor IX fusion protein conjugate of the present invention is conjugated with one or more PEG groups for modification.

In the present invention, the polymer is not limited to a particular structure, it can be linear (such as alkoxy PEG or bifunctional PEG), branched or multi-armed (such as bifurcated PEG or PEG linked to polyol core), dendritic or may have degradable linkages. In addition, the internal structure of the polymer can be organized in any number of different patterns, which can be selected from homopolymers, alternating copolymers, random copolymers, block copolymers, alternating terpolymers, random terpolymers, block terpolymers, and the like. The polymer may also include poly (alkylene oxide) polymers, polymaleic acid, poly (D, L-alanine), and the like.

In some embodiments, the polymer is polyethylene glycol (PEG) or derivatives thereof such as methoxy polyethylene glycol (mPEG). Herein, unless specifically indicated, the polyethylene glycols (PEG) include those either with hydroxyl groups as the terminal group or with other groups as the terminal group. The other groups include, but are not limited to, alkoxy, cycloalkoxy, cycloalkyloxy, alkenyl, aryloxy or aralkyloxy. These PEG molecular types are known in the prior art and are routinely used in polypeptide modification. The PEG side chain can be linear, branched, bifurcated or composed of multiple arms. Different polyethylene glycols can have different polymer chain lengths and polymer structures.

In the present invention, there is no particular limitation on the molecular weight of PEG, and its molecular weight can range from 0.1 to 200 kDa, such as 1 to 150 kDa, 2 to 100 kDa, 3 to 80 kDa, 4 to 50 kDa, or 5 to 40 kDa. Other useful PEG include, for example, those disclosed in WO 03/040211, US 6,566,506, US 6,864,350, and US 6,455,639. In particular, the PEG has a general formula HO-CH₂CH₂O-(CH₂CH₂O)ₙ-CH₂CH₂-OH, wherein the range of n is about 5 to 4000. As mentioned above, the PEG of the present invention includes PEG with other terminal groups, such as methoxy PEG, branched PEG, bifurcated PEG, and the like. Suitable branched PEGs can be prepared as described in US Patent No. 5,932,462, the entire disclosure of which is incorporated herein by reference. The bifurcated PEG refers to a PEG that has a branch near one end of the polymer chain, and the main chain of the bifurcated PEG can be linear or branched.

It is known to those skilled in the art that in a bioactive molecule conjugated with a polymer group, as the molecular weight of the polymer group increases, the biological activity of the conjugate usually gradually decreases. It is also known to those skilled in the art that as the molecular weight of the polymer group increases, the biological half-life and/or plasma half-life of the conjugate increases accordingly.

In order to provide a stable therapeutic effect over a long period, and to reduce the frequency of administration to improve patient compliance, it is desirable to extend the biological half-life of the coagulation factor IX fusion protein conjugate as much as possible while retaining significant activity of the coagulation factor IX receptor agonist. Therefore, the present invention provides the coagulation factor IX fusion protein conjugate with extended biological half-life and significant activity of the coagulation factor IX receptor agonist.

In a specific embodiment, in the coagulation factor IX fusion protein conjugate of the present invention, the molecular weight of the one or more polymer groups (such as PEG) is ≥1, ≥10, ≥ 20, ≥30, ≥40, ≥50, ≥60, ≥70, ≥80, ≥90, ≥100, ≥110, ≥120, ≥130, ≥140, ≥150, or ≥160 kDa, for example, 5, 10, 20, 30, 40, 50, 60, 70, 80, 90, or 100 kDa, or a value between any two of the above values. It should be noted that when describing the molecular weight of the conjugated polymer group in a coagulation factor IX fusion protein conjugate, if there are multiple conjugated polymer groups in the conjugate, the sum of the molecular weights of all conjugated polymer groups in the conjugate is calculated, unless otherwise specified.

The polymer used in the present invention is known in the prior art, and it can be obtained through a variety of ways, including, for example, commercial routes, or it can be prepared by oneself according to methods known in the art. The present invention is not limited to polymers made by any specific method.

After the conjugation reaction, the conjugate can be separated by a suitable method, including, for example, ultrafiltration, dialysis, or chromatography, etc., all of which are within the abilities of those of ordinary skill in the art.

### Pharmaceutical composition

The coagulation factor IX fusion protein or coagulation factor IX fusion protein conjugate of the present invention can have multiple applications, including, for example, for use in prevention and/or treatment of hemorrhagic diseases. Therefore, the present invention also provides a pharmaceutical composition for preventing and/or treating hemorrhagic diseases, which comprises a therapeutically effective amount of the fusion protein or conjugate of the present invention, and optionally a pharmaceutically acceptable carrier. Preferably, the pharmaceutical composition can be used for the prevention and/or treatment of hemorrhagic diseases, more preferably for the prevention and/or treatment of hemorrhagic diseases in patients with congenital or acquired deficiency of FIX, and spontaneous or surgical bleeding in patients with hemophilia B.

The therapeutically effective amount of the fusion protein or conjugate of the present invention depends on the route of administration, the type of subjects, and the physical characteristics of the specific mammal considered. These factors and their relationship with determining the amount are well known to those skilled in the medicinal field. The amount and method of administration can be adjusted to achieve optimal efficacy to deliver the peptide to the subject, but depending on factors well known to those skilled in the medicinal field such as body weight, diet, concomitant medication, and other factors.

The pharmaceutical composition of the present invention can be administered in combination therapy, that is, in combination with one or more other agents, wherein the pharmaceutical composition and the other agents are administered together or sequentially. In other embodiments, the other agents may be administered before, during, or after the administration of one or more of the fusion protein, conjugate, or pharmaceutical composition thereof of the present invention.

As used herein, "pharmaceutically acceptable carrier" or "physiologically acceptable carrier" can be used interchangeably, including one or more of any and all physiologically compatible salts, solvents, dispersion media, coatings, antibacterial and antifungal agents, isotonic and absorption delaying agents, etc. In some embodiments, the carrier is suitable for intravenous, intramuscular, subcutaneous, parenteral, spinal or epidermal administration (e.g., by injection or infusion). Depending on the route of administration, the therapeutic agent may be coated with certain materials to protect the therapeutic agent from the action of acids and other natural conditions that may inactivate the therapeutic agent.

When administered, the pharmaceutical preparation of the present invention is administered in a pharmaceutically acceptable amount in a pharmaceutically acceptable composition. The term "pharmaceutically acceptable" means a non-toxic substance that does not interfere with the biologically active efficacy of the active components. Such preparations generally contain salts, buffers, preservatives, compatible carriers, and optionally other therapeutic agents, such as supplementary immune enhancers, including adjuvants, chemokines and cytokines. When used in medicaments, the salt should be pharmaceutically acceptable. However non-pharmaceutically acceptable salts can be conveniently used to prepare pharmaceutically acceptable salts thereof.

If necessary, the coagulation factor IX fusion protein or coagulation factor IX fusion protein conjugate of the present invention can be combined with pharmaceutically acceptable carriers. The term "pharmaceutically acceptable carriers" used herein refers to one or more compatible solid or liquid fillers, diluents, or encapsulating substances, which are suitable for administration to mammals such as human. The term "carrier" means organic or inorganic, natural or synthetic components, which are combined with the active components to facilitate application. The components of the pharmaceutical composition can also be blended in a form in which there are no interactions that can significantly disrupt the therapeutic effect of the desired drug.

Preferably, the pharmaceutical composition of the present invention may contain a buffer system, and preferably the buffer system is an acetate buffer solution with a pH of about 3.0 to about 6.0, or a phosphate buffer solution with a pH of about 5.0 to about 9.0. In some specific embodiments, suitable buffers include acetate, citrate, borate, and phosphate.

Optionally, the pharmaceutical composition may also contain suitable preservatives, such as benzalkonium chloride, chloro-tert-butanol, parabens and thimerosal.

The pharmaceutical composition can be conveniently presented in a unit dosage form and can be prepared by any known method in the pharmaceutical field. The method comprises the step of combining the active agent with a carrier, which contains one or more accessory ingredients. Generally, the composition is prepared by intimately combining the active compound with one or both of a liquid carrier and a finely divided solid carrier, followed by shaping the product if necessary.

Pharmaceutical compositions suitable for parenteral administration may be sterile aqueous or nonaqueous formulations containing one or more fusion proteins or conjugates. In some embodiments, the formulation is isotonic with the blood of the subject. This preparation can be formulated according to known methods using suitable dispersing or wetting agents and suspending agents. The sterile injection preparation can also be a sterile injection solution or suspension in a non-toxic parenterally acceptable diluent or solvent, for example, a solution in 1,3-butanediol. Acceptable carriers and solvents that can be used include water, Ringer's solution, and isotonic sodium chloride solution. In addition, sterile fixed oils are conventionally used as solvents or suspension media. For this reason, any mild fixed oil can be used, including synthetic mono- or di-glycerides. Also, fatty acids such as oleic acid can be used as the injectable formulations. Carrier formulations suitable for oral, subcutaneous, intravenous, intramuscular, etc. administration can be obtained by referring to reference books in the prior art.

The fusion protein or conjugate of the present invention can be prepared with carriers that protect it from rapid release, such as a controlled release formulation, including implants, transdermal patches, and microencapsulated delivery systems. Biodegradable and biocompatible polymers can be used, such as ethylene vinyl acetate, polyanhydrides, polyglycolic acid, collagen, polyorthoesters, and polylactic acid. Many methods for preparing such formulations are known in the prior art.

The pharmaceutical composition of the present invention can be administered by any conventional route, including injection or gradual infusion over time. For example, the administration can be oral, intravenous, intraperitoneal, intramuscular, intracavity, intratumor, or transdermal.

The pharmaceutical composition of the present invention is administered in an effective amount. An "effective amount" is the amount of any fusion protein or conjugate provided herein, alone or with further doses and/or other therapeutic agents, producing a desired response.

Of course, such an amount will depend on the specific disease to be treated, the severity of the disease, individual patient parameters (including age, physical condition, height and body weight), duration of treatment, the nature of the concurrent treatment (if any), the specific route of administration, and similar factors within the knowledge of medical and health workers. These factors are well known to those skilled in the art, and can be known only by using routine experiments. It is generally preferred to use the maximum dose of each component or combination thereof, that is to say the highest safe dose based on reasonable medical judgment. However, those skilled in the art can understand that patients may require lower doses or allowable doses for medical, psychological, or basically any other reasons.

The pharmaceutical composition used in the foregoing method is preferably sterile and contains an effective amount of the fusion protein or conjugate alone or in combination with another preparation in a weight unit or volume unit suitable for administration to patients to produce the desired response, such as a decrease in blood glucose.

The dosage of the fusion protein or conjugate administered to the subject can be selected according to different parameters, especially according to the mode of administration and the state of the subject. Other factors include the required treatment period. If the response in the subject at the initial dose applied is insufficient, a higher dose (or an effective higher dose achieved by a different, more localized delivery route) within the patient's tolerance can be applied.

In some embodiments, the dosage range of the coagulation factor IX fusion protein and/or coagulation factor IX fusion protein conjugate may be 30 mg/kg of body weight/day to 0.00001 mg/kg of body weight/day, or 3 mg/kg/day to 0.0001 mg/kg/day, or 0.3 mg/kg/day to 0.01 mg/kg/day.

In some embodiments, the pharmaceutical composition of the present invention contains 0.20-5 mg/ml of the coagulation factor IX fusion protein and/or 4-40 mg/ml of the coagulation factor IX fusion protein conjugate, preferably 0.20-5mg/ml of the coagulation factor IX fusion protein and/or 4-40 mg/ml of the coagulation factor IX fusion protein conjugate, more preferably 0.5-2 mg/ml of the coagulation factor IX fusion protein and/or 10-20 mg/ml of the coagulation factor IX fusion protein conjugate. Generally, the dosage of the coagulation factor IX fusion protein or coagulation factor IX fusion protein conjugate of the present invention can range from about 10 µg/kg of the patient's body weight to about 100,000 µg/kg of the patient's body weight. In some embodiments, the dosage may range from about 0.1 mg/kg to about 20 mg/kg. In some embodiments, the dosage may range from about 0.1 mg/kg to 5 mg/kg, 0.1 mg/kg to 10 mg/kg, or 0.1 mg/kg to 15 mg/kg. In some embodiments, the dosage may range from about 1 mg/kg to 5 mg/kg, 5 mg/kg to 10 mg/kg, 10 mg/kg to 15 mg/kg, or 15 mg/kg to 20 mg/kg. In some embodiments, the dosage is about 0.1 mg/kg, 0.5 mg/kg, 1 mg/kg, 2 mg/kg, 3 mg/kg, 5 mg/kg, 7 mg/kg, 10 mg/kg, 12 mg/kg, 15 mg/kg, 17 mg/kg, 20 mg/kg, 25 mg/kg, or 30 mg/kg. In some embodiments, the dosage is about 1 mg/kg, 3 mg/kg, 5 mg/kg, or 6 mg/kg. Based on the property of the composition, the dose can be delivered continuously (for example, by a continuous pump) or at periodic intervals. In some embodiments, when administered intravenously, the dosage of the coagulation factor IX fusion protein or coagulation factor IX fusion protein conjugate of the present invention may be 0.1 to 20 mg/kg or any value therein. The ideal time interval for multiple administrations of a particular composition can be determined by those skilled in the art without undue experimentation. Other dosage regimens of the provided composition are known to those skilled in the art, where the dosage, administration schedule, administration site, administration mode, etc. may be different from the foregoing. In one embodiment, the dosage is administered intravenously. In another embodiment, the dosage regimen is a single intravenous administration.

The present invention also provides a kit comprising the coagulation factor IX fusion protein or coagulation factor IX fusion protein conjugate (for example in the pharmaceutical composition) and instructions for use. The kit may additionally contain at least one of other agents, such as one or more of other agents preventing and/or treating hemorrhagic diseases. In another embodiment, the kit may include a carrier that is compartmentalized to tightly hold one or more container devices or a series of container devices (such as test tubes, tubes, flasks, bottles, syringes, etc.). The components of the kit can be packaged in an aqueous medium or in a lyophilized form.

The composition may be provided in a lyophilized form or in an aqueous medium.

Preferably, the subject is a vertebrate, more preferably a mammal, most preferably human, but the subject can also be other animals, such as domestic animals (e.g., dogs, cats, etc.), livestock (e.g., cattle, sheep and goats, pigs, horses, etc.) or experimental animals (e.g., monkeys, rats, mice, rabbits, guinea pigs, etc.).

The term "improved circulation half-life" or "extended circulation half-life" means that the molecule of the present invention has an altered circulation half-life, preferably an increased circulation half-life compared to the wild-type factor IX molecule. The circulation half-life is preferably increased by at least 10%, preferably at least 15%, preferably at least 20%, preferably at least 25%, preferably at least 30%, preferably at least 35%, preferably at least 40%, preferably at least 45%, preferably at least 50%, preferably at least 55%, preferably at least 60%, preferably at least 65%, preferably at least 70%, preferably at least 75%, preferably at least 80%, preferably at least 85%, preferably at least 90%, preferably at least 95%, preferably at least 100%, more preferably at least 125%, more preferably at least 150%, more preferably at least 175%, more preferably at least 200%, and more preferably at least 250% or 300%. Even more preferably, the molecule has a circulation half-life that is increased by at least 400%, 500%, 600%, or even 700%.

The present invention will be further illustrated by the following examples, which should not be construed as further limitations in any way. The entire contents of all references cited in this application (including article references, granted patents, published patent applications and copending patent applications) are expressly incorporated herein by reference. In the following examples, if not specifically noted, the reagents and materials used are commercially available products with at least analytical purity or equivalent levels.

### Examples

### Example 1 Preparation of Fusion Protein FIX-L1-Fc-L2-P

### Construction of expression vector

The FIX fusion proteins constructed in this example have a common molecular structure of FIX-L1-Fc-L2-P, wherein FIX represents a natural coagulation factor IX derived from human, the sequence of which is shown in SEQ ID NO: 1.

L1, representing a first linker between FIX and Fc, is a flexible peptide or a linker peptide composed of a flexible peptide and a rigid structure based on multiple prolines (Pro, P). In this example, a total of 6 sequences was used as L1, expressed as C1, C2, C3, C4, C5 and GS, respectively, and their specific sequences are as follows:
C1: GGGGSGGGGSGGGGSGGGGSGGGGSVAPPPALPAPVRLPGPA (SEQ ID NO: 8);
C2: GGGGSGGGGSGGGGSGGGGSGGGGSVAPPPALPAVAPPPALPA (SEQ ID NO: 9); 
C3:GGGGSGGGGSGGGGSGGGGSGGGGSVAPPPALPAVAPPPALPAVAPPPALPAPVRLP
GPA (SEQ ID NO: 10);
 
C4:GGGGSGGGGSGGGGSGGGGSGGGGSVAPPPALPAPVRLPGPAVAPPPALPAVAPPPA
LPA (SEQ ID NO: 11);
 
C5:GGGGSGGGGSGGGGSGGGGSGGGGSVAPPPALPAVAPPPALPAGSVAPPPALPAVAP
PPALPA (SEQ ID NO: 12);
 and
GS: GGGGSGGGGSGGGGSGGGGSGGGGS (SEQ ID NO: 13).

Fc is derived from human IgG1 carrying N297A point mutation which has reduced ADCC and CDC activities (see Lund J. et al. Multiple binding sites on the CH2 domain of IgG for mouse Fc gamma R11. Mol Immunol. 1992 Jan; 29(1):53-9.).

L2 is a second linker, which is mainly used to separate Fc and the Sortase recognition site. Specifically, the sequence of L2 in this example is GSGGSGGGS (SEQ ID NO: 16).

P is an amino acid fragment containing the Sortase recognition site. Table 1 Summary of all the FIX-L1-Fc-L2-P fusion proteins constructed in this example

**Table 1**

| Protein name | FIX | L1 | Fc | L2 | P |
|---|---|---|---|---|---|
| FIX-C1-Fc-L2-P | SEQ ID NO: 1 | C1 | Human IgG1, N297A | GSGGSGGGGS | LPETGG |
| FIX-C2-Fc-L2-P | SEQ ID NO: 1 | C2 | Human IgG1, N297A | GSGGSGGGGS | LPETGG |
| FIX-GS-Fc-L2-P | SEQ ID NO: 1 | GS | Human IgG1, N297A | GSGGSGGGGS | LPETGG |
| FIX-C3-Fc-L2-P | SEQ ID NO: 1 | C3 | Human IgG1, N297A | GSGGSGGGGS | LPETGG |
| FIX-C4-Fc-L2-P | SEQ ID NO: 1 | C4 | Human IgG1, N297A | GSGGSGGGGS | LPETGG |
| FIX-C5-Fc-L2-P | SEQ ID NO: 1 | C5 | Human IgG1, N297A | GSGGSGGGGS | LPETGG |

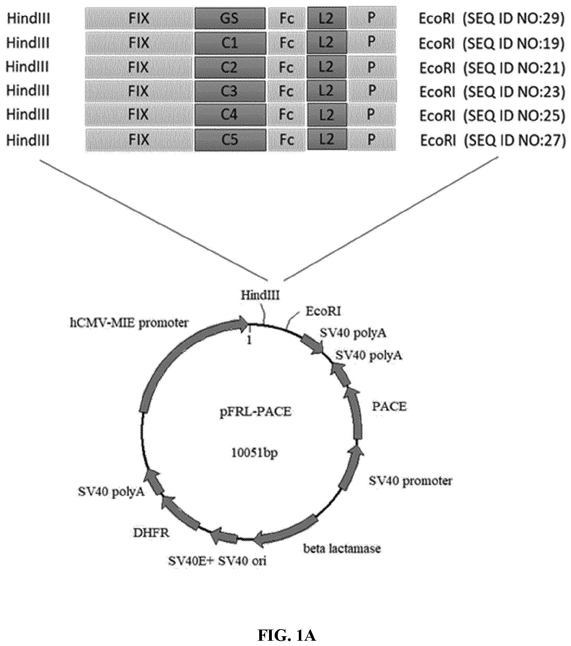

Most of the recombinant FIX expressed in CHO cells exists as a precursor protein, and only about 30% of the propeptide is cleaved from precursor protein to form mature FIX. The processing of mature FIX requires the participation of PACE/furin enzyme, which is a subtilisin-like calcium-dependent serine propeptidase that is located in the trans-Golgi apparatus and can cleave the C-terminus of R46 in the FIX precursor protein to catalyze the conversion of FIX precursor protein into mature FIX. In order to improve the yield and ensure the correct processing of FIX, in this example, a FIX/PACE (Furin) bicistronic expression vector is further constructed, the structure of which is shown in FIG. 1A.

### Construction of FIX/PACE (Furin) bicistronic expression vector

### Construction of expression vector pFRL-rPS expressing PACE

The sequence of PACE/Furin refers to GenBank accession number NP_002560.1. The coding sequence of PACE/Furin amino acids 1-715 (SEQ ID NO: 17) was first codon-optimized for the expression host. After full gene synthesis, the sequence was cloned into pcDNA3.1 (+) vector, and the obtained plasmid was named pcDNA3.1-P-SOL (Shanghai Generay Biotech Co., Ltd.). A complete gene expression cassette including the promoter SV40, PACE/Furin gene and Poly A was obtained through PCR amplification with primers F1 and R1 (see below for the sequence) using the plasmid pcDNA3.1-P-SOL as a template. By the BglII restriction sites at both ends, the fragment was inserted into the pFRL-DHFR vector that was single digested with BamHI. The positive clones were selected and verified by sequencing, and the vector was named pFRL-rPS.
F1: 5'-TCAGGAAGATCTCGCGAATTAATTCTGTGGAATGTGT-3' (SEQ ID NO: 30);
R1: 5'-TATCCTAGATCTGCTGGCACGACAGGTTTCCCGAC T- 3' (SEQ ID NO: 31).

The sequence of PACE/Furin amino acid 1-715 is as follow:

MELRPWLLWVVAATGTLVLLAADAQGQKVFTNTWAVRIPGGPAVANSVARKHGFLNL
GQIFGDYYHFWHRGVTKRSLSPHRPRHSRLQREPQVQWLEQQVAKRRTKRDVYQEPT
DPKFPQQWYLSGVTQRDLNVKAAWAQGYTGHGIVVSILDDGIEKNHPDLAGNYDPG
ASFDVNDQDPDPQPRYTQMNDNRHGTRCAGEVAAVANNGVCGVGVAYNARIGGVRM
LDGEVTDAVEARSLGLNPNHIHIYSASWGPEDDGKTVDGPARLAEEAFFRGVSQGRGG
LGSIFVWASGNGGREHDSCNCDGYTNSIYTLSISSATQFGNVPWYSEACSSTLATTYSS
GNQNEKQIVTTDLRQKCTESHTGTSASAPLAAGIIALTLEANKNLTWRDMQHLVVQTS
KPAHLNANDWATNGVGRKVSHSYGYGLLDAGAMVALAQNWTTVAPQRKCIIDILTEP
KDIGKRLEVRKTVTACLGEPNHITRLEHAQARLTLSYNRRGDLAIHLVSPMGTRSTLLA
ARPHDYSADGFNDWAFMTTHSWDEDPSGEWVLEIENTSEANNYGTLTKFTLVLYGTA
PEGLPVPPESSGCKTLTSSQACVVCEEGFSLHQKSCVQHCPPGFAPQVLDTHYSTENDV
ETIRASVCAPCHASCATCQGPALTDCLSCPSHASLDPVEQTCSRQSQSSRESPPQQQPPR
LPPEVEAGQRLRAGLLPSHLPE (SEQ ID NO: 17).

### b. Construction of bicistronic expression vectors expressing FIX-L1-Fc-L2-P

### b1) pFRL-rPS-FIX-C1-Fc-L2-P

The nucleic acid sequence encoding FIX-C1-Fc-L2-P (that is, the sequence of L1 in FIX-L1-Fc-L2-P is C1, and so on) was codon-optimized (the amino acid sequence is shown in SEQ ID NO: 18, and the codon-optimized nucleic acid coding sequence is shown in SEQ ID NO: 19). After full gene synthesis by Shanghai Generay Biotech Co., Ltd., the fragment was inserted into pFRL-rPS through the HindIII/EcoRI double restriction site to construct the bicistronic expression vector pFRL-rPS-FIX-C1-Fc-L2-P co-expressing FIX-C1-Fc-L2-P/Furin.

Amino acid sequence of FIX-C1-Fc-L2-P:

MQRVNMIMAESPGLITICLLGYLLSAECTVFLDHENANKILNRPKRYNSGKLEEFVQGNL
ERECMEEKCSFEEAREVFENTERTTEFWKQYVDGDQCESNPCLNGGSCKDDINSYECWC
PFGFEGKNCELDVTCNIKNGRCEQFCKNSADNKVVCSCTEGYRLAENQKSCEPAVPFPC
GRVSVSQTSKLTRAETVFPDVDYVNSTEAETILDNITQSTQSFNDFTRVVGGEDAKPGQFP
WQVVLNGKVDAFCGGSIVNEKWIVTAAHCVETGVKITVVAGEHNIEETEHTEQKRNVIR
IIPHHNYNAAINKYNHDIALLELDEPLVLNSYVTPICIADKEYTNIFLKFGSGYVSGWGRV
FHKGRSALVLQYLRVPLVDRATCLRSTKFTIYNNMFCAGFHEGGRDSCQGDSGGPHVTE
VEGTSFLTGIISWGEECAMKGKYGIYTKVSRYVNWIKEKTKLTGGGGSGGGGSGGGGSG
GGGSGGGGSVAPPPALPAPVRLPGPADKTHTCPPCPAPELLGGPSVFLFPPKPKDTLMISRT
PEVTCVVVDVSHEDPEVKFNWYVDGVEVHNAKTKPREEQYASTYRVVSVLTVLHQDW
LNGKEYKCKVSNKALPAPIEKTISKAKGQPREPQVYTLPPSRDELTKNQVSLTCLVKGFYP
SDIAVEWESNGQPENNYKTTPPVLDSDGSFFLYSKLTVDKSRWQQGNVFSCSVMHEALH
NHYTQKSLSLSPGKGSGGSGGGGSLPETGG (SEQ ID NO: 18).

Nucleotide sequence of FIX-C1-Fc-L2-P:

ATGCAGCGCGTCAATATGATTATGGCCGAGTCTCCAGGCCTCATTACCATTTGTCTGCT
GGGCTACCTGCTGTCCGCAGAGTGTACCGTCTTCCTCGACCACGAGAACGCAAATAA
GATTCTCAATAGGCCTAAGCGCTACAATTCCGGAAAGCTCGAAGAGTTTGTCCAGGGA
AATCTGGAGCGCGAGTGTATGGAGGAAAAGTGTAGCTTTGAAGAAGCTAGAGAGGTG
TTTGAGAATACTGAGCGCACTACTGAGTTTTGGAAGCAGTATGTGGATGGCGATCAGT
GCGAGTCCAATCCATGCCTGAATGGCGGATCTTGCAAGGATGATATTAATTCTTACGAG
TGTTGGTGCCCATTCGGCTTTGAGGGAAAGAATTGCGAACTGGATGTCACATGTAACA
TCAAGAACGGCAGATGCGAGCAGTTTTGTAAGAACAGCGCCGATAATAAGGTGGTGT
GTAGTTGTACTGAGGGATACCGCCTCGCAGAGAATCAGAAGTCCTGTGAGCCCGCTG
TGCCATTTCCTTGTGGCCGGGTCAGCGTCAGCCAGACTTCTAAGCTGACACGCGCCG
AGACCGTCTTTCCAGATGTGGATTATGTCAACTCTACTGAGGCCGAGACCATTCTCGAT
AATATTACTCAGTCTACACAGTCCTTTAACGACTTCACTCGGGTGGTGGGCGGAGAGG
ACGCAAAGCCTGGCCAGTTTCCTTGGCAGGTCGTGCTCAACGGAAAGGTGGACGCAT
TTTGTGGCGGATCTATCGTGAATGAGAAGTGGATTGTGACCGCCGCTCACTGCGTCGA
GACAGGCGTCAAGATTACCGTGGTCGCTGGAGAGCACAACATTGAGGAAACAGAGC
ACACCGAGCAGAAGCGCAATGTGATTCGCATCATCCCACACCACAACTACAACGCCG

CTATTAATAAGTACAACCACGACATCGCCCTGTTAGAACTAGATGAGCCTCTGGTCCTG
AATAGTTACGTCACTCCTATTTGTATCGCCGATAAAGAATACACTAACATCTTTCTCAA
GTTCGGCTCTGGCTATGTATCTGGATGGGGACGGGTGTTTCACAAGGGACGGTCCGCA
CTCGTGCTACAATACCTCCGGGTGCCACTGGTGGATCGGGCAACATGCCTCCGGTCTA
CCAAGTTTACTATATACAATAACATGTTTTGCGCCGGCTTTCACGAGGGCGGACGGGAT
AGTTGCCAGGGCGATAGTGGAGGCCCTCATGTCACAGAGGTGGAGGGAACTAGCTTT
CTGACAGGCATTATCTCTTGGGGCGAAGAGTGCGCTATGAAGGGAAAGTACGGCATCT
ATACTAAGGTCTCGCGTTACGTCAATTGGATCAAGGAAAAGACAAAGCTGACAGGCG
GCGGAGGGAGCGGCGGCGGAGGCTCTGGCGGCGGCGGATCTGGCGGAGGCGGCTCT
GGCGGAGGCGGATCCGTCGCTCCACCACCTGCACTCCCCGCCCCTGTCAGACTGCCT
GGACCCGCTGATAAGACCCACACATGTCCACCTTGCCCAGCACCTGAGCTGCTGGGC
GGACCTAGCGTGTTTCTGTTCCCACCAAAGCCAAAGGACACCCTCATGATTAGTAGAA
CCCCAGAGGTGACTTGCGTCGTGGTCGATGTCAGTCACGAGGACCCCGAGGTGAAGT
TTAATTGGTATGTAGATGGCGTCGAGGTGCACAACGCTAAGACCAAGCCACGCGAAG
AGCAGTACGCATCTACTTACCGGGTCGTCTCTGTGCTGACTGTGCTGCACCAGGATTG
GCTCAATGGCAAAGAGTACAAGTGTAAGGTCAGTAATAAGGCCCTCCCCGCACCTATT
GAAAAGACTATTAGCAAGGCAAAGGGCCAGCCTAGGGAGCCCCAGGTCTACACCCTG
CCCCCTTCCCGCGATGAGCTGACTAAGAATCAGGTCTCTCTCACTTGCCTCGTGAAGG
GCTTTTACCCATCCGACATTGCCGTGGAGTGGGAGTCCAACGGACAGCCAGAGAATA
ACTACAAGACAACTCCTCCCGTGCTCGATAGCGACGGTTCATTCTTCCTGTACTCCAA
GCTCACCGTTGATAAGTCTCGGTGGCAGCAGGGAAATGTGTTCTCTTGTTCCGTCATG
CACGAGGCACTGCACAATCACTACACCCAGAAGTCCCTGTCCCTCAGCCCTGGCAAA
GGCAGCGGCGGATCTGGAGGCGGCGGATCCCTCCCAGAGACCGGCGGATGA (SEQ ID
NO: 19).

### b2) pFRL-rPS-FIX-C2-Fc-L2-P

Using the coding sequence of FIX-C1-Fc-L2-P as a template, C2 sequence was added to the 5' end of the primer, and the Fc-L2-P sequence was amplified by PCR to obtain the C2-Fc-L2-P sequence. The sequences of primers F2 and R2 used for amplification are as follows:
Forward primer F2:
   5'- 
TTCGCGGATCCGTCGCTCCACCACCTGCACTCCCCGCCGTGGCTCCTCCACCTGCTTT
GCCCGCTGATAAGACCCACACATGTCCAC-3' (SEQ ID NO: 32),
Reverse primer R2:
   5'-

TAACCGGAATTCATTATCCGCCGGTCTCTGGGAGAGATCCGCCGCCTCCAGATCCGCC
GCTGCCGCCAGGGCTGAGGGACAGGGACT-3' (SEQ ID NO: 33).

Next, through the BamHI/EcoRI double restriction site, the C1-Fc-L2-P sequence in the vector pFRL-rPS-FIX-C1-Fc-L2-P was replaced with the amplified product C2-Fc-L2-P, thereby obtaining the bicistronic expression vector pFRL-rPS-FIX-C2-Fc-L2-P co-expressing FIX-C2-Fc-L2-P/Furin.

Amino acid sequence of FIX-C2-Fc-L2-P:

MQRVNMIMAESPGLITICLLGYLLSAECTVFLDHENANKILNRPKRYNSGKLEEFVQGNL
ERECMEEKCSFEEAREVFENTERTTEFWKQYVDGDQCESNPCLNGGSCKDDINSYECWC
PFGFEGKNCELDVTCNIKNGRCEQFCKNSADNKVVCSCTEGYRLAENQKSCEPAVPFPC
GRVSVSQTSKLTRAETVFPDVDYVNSTEAETILDNITQSTQSFNDFTRVVGGEDAKPGQFP
WQVVLNGKVDAFCGGSIVNEKWIVTAAHCVETGVKITVVAGEHNIEETEHTEQKRNVIR
IIPHHNYNAAINKYNHDIALLELDEPLVLNSYVTPICIADKEYTNIFLKFGSGYVSGWGRV
FHKGRSALVLQYLRVPLVDRATCLRSTKFTIYNNMFCAGFHEGGRDSCQGDSGGPHVTE
VEGTSFLTGIISWGEECAMKGKYGIYTKVSRYVNWIKEKTKLTGGGGSGGGGSGGGGSG
GGGSGGGGSVAPPPALPAVAPPPALPADKTHTCPPCPAPELLGGPSVFLFPPKPKDTLMISR
TPEVTCVVVDVSHEDPEVKFNWYVDGVEVHNAKTKPREEQYASTYRVVSVLTVLHQD
WLNGKEYKCKVSNKALPAPIEKTISKAKGQPREPQVYTLPPSRDELTKNQVSLTCLVKGF
YPSDIAVEWESNGQPENNYKTTPPVLDSDGSFFLYSKLTVDKSRWQQGNVFSCSVMHEA
LHNHYTQKSLSLSPGGSGGSGGGGSLPETGG (SEQ ID NO: 20).

Nucleotide sequence of FIX-C2-Fc-L2-P:

ATGCAGCGCGTCAATATGATTATGGCCGAGTCTCCAGGCCTCATTACCATTTGTCTGCT
GGGCTACCTGCTGTCCGCAGAGTGTACCGTCTTCCTCGACCACGAGAACGCAAATAA
GATTCTCAATAGGCCTAAGCGCTACAATTCCGGAAAGCTCGAAGAGTTTGTCCAGGGA

AATCTGGAGCGCGAGTGTATGGAGGAAAAGTGTAGCTTTGAAGAAGCTAGAGAGGTG
TTTGAGAATACTGAGCGCACTACTGAGTTTTGGAAGCAGTATGTGGATGGCGATCAGT
GCGAGTCCAATCCATGCCTGAATGGCGGATCTTGCAAGGATGATATTAATTCTTACGAG
TGTTGGTGCCCATTCGGCTTTGAGGGAAAGAATTGCGAACTGGATGTCACATGTAACA
TCAAGAACGGCAGATGCGAGCAGTTTTGTAAGAACAGCGCCGATAATAAGGTGGTGT
GTAGTTGTACTGAGGGATACCGCCTCGCAGAGAATCAGAAGTCCTGTGAGCCCGCTG
TGCCATTTCCTTGTGGCCGGGTCAGCGTCAGCCAGACTTCTAAGCTGACACGCGCCG
AGACCGTCTTTCCAGATGTGGATTATGTCAACTCTACTGAGGCCGAGACCATTCTCGAT
AATATTACTCAGTCTACACAGTCCTTTAACGACTTCACTCGGGTGGTGGGCGGAGAGG
ACGCAAAGCCTGGCCAGTTTCCTTGGCAGGTCGTGCTCAACGGAAAGGTGGACGCAT
TTTGTGGCGGATCTATCGTGAATGAGAAGTGGATTGTGACCGCCGCTCACTGCGTCGA
GACAGGCGTCAAGATTACCGTGGTCGCTGGAGAGCACAACATTGAGGAAACAGAGC
ACACCGAGCAGAAGCGCAATGTGATTCGCATCATCCCACACCACAACTACAACGCCG
CTATTAATAAGTACAACCACGACATCGCCCTGTTAGAACTAGATGAGCCTCTGGTCCTG
AATAGTTACGTCACTCCTATTTGTATCGCCGATAAAGAATACACTAACATCTTTCTCAA
GTTCGGCTCTGGCTATGTATCTGGATGGGGACGGGTGTTTCACAAGGGACGGTCCGCA
CTCGTGCTACAATACCTCCGGGTGCCACTGGTGGATCGGGCAACATGCCTCCGGTCTA
CCAAGTTTACTATATACAATAACATGTTTTGCGCCGGCTTTCACGAGGGCGGACGGGAT
AGTTGCCAGGGCGATAGTGGAGGCCCTCATGTCACAGAGGTGGAGGGAACTAGCTTT
CTGACAGGCATTATCTCTTGGGGCGAAGAGTGCGCTATGAAGGGAAAGTACGGCATCT
ATACTAAGGTCTCGCGTTACGTCAATTGGATCAAGGAAAAGACAAAGCTGACAGGCG
GCGGAGGGAGCGGCGGCGGAGGCTCTGGCGGCGGCGGATCTGGCGGAGGCGGCTCT
GGCGGAGGCGGATCCGTCGCTCCACCACCTGCACTCCCCGCCGTGGCTCCTCCACCT
GCTTTGCCCGCTGATAAGACCCACACATGTCCACCTTGCCCAGCACCTGAGCTGCTGG
GCGGACCTAGCGTGTTTCTGTTCCCACCAAAGCCAAAGGACACCCTCATGATTAGTAG
AACCCCAGAGGTGACTTGCGTCGTGGTCGATGTCAGTCACGAGGACCCCGAGGTGAA
GTTTAATTGGTATGTAGATGGCGTCGAGGTGCACAACGCTAAGACCAAGCCACGCGA
AGAGCAGTACGCATCTACTTACCGGGTCGTCTCTGTGCTGACTGTGCTGCACCAGGAT
TGGCTCAATGGCAAAGAGTACAAGTGTAAGGTCAGTAATAAGGCCCTCCCCGCACCT
ATTGAAAAGACTATTAGCAAGGCAAAGGGCCAGCCTAGGGAGCCCCAGGTCTACACC
CTGCCCCCTTCCCGCGATGAGCTGACTAAGAATCAGGTCTCTCTCACTTGCCTCGTGA

AGGGCTTTTACCCATCCGACATTGCCGTGGAGTGGGAGTCCAACGGACAGCCAGAGA
ATAACTACAAGACAACTCCTCCCGTGCTCGATAGCGACGGTTCATTCTTCCTGTACTCC
AAGCTCACCGTTGATAAGTCTCGGTGGCAGCAGGGAAATGTGTTCTCTTGTTCCGTCA
TGCACGAGGCACTGCACAATCACTACACCCAGAAGTCCCTGTCCCTCAGCCCTGGCG
GCAGCGGCGGATCTGGAGGCGGCGGATCTCTCCCAGAGACCGGCGGATAA (SEQ ID
NO: 21).

### b3) Construction of pFRL-rPS-FIX-C3-Fc-L2-P

The vector construction method is the same as for pFRL-rPS-FIX-C2-Fc-L2-P. The PCR primers for amplifying the coding sequence of C3-Fc-L2-P include forward primers F3: 5'-TGGCTCCTCCACCTGCTTTGCCCGCTGTCGCTCCACCACCTGCACTCC-3' (SEQ ID NO: 34) and F4: 5'- TAGATCTGTCGCTCCACCACCTGCACTCCC CGCCGTGGCTCCTCCACCTGCTTTGCC-3' (SEQ ID NO: 35), and reverse primer R3: 5'-TGAATTCT TATCATCCGCCGGTCTCTGGGAG-3' (SEQ ID NO: 36). The C3-Fc-L2-P sequence was obtained by 2 rounds of PCR amplification.

Amino acid sequence of FIX-C3-Fc-L2-P:

MQRVNMIMAESPGLITICLLGYLLSAECTVFLDHENANKILNRPKRYNSGKLEEFVQGNL
ERECMEEKCSFEEAREVFENTERTTEFWKQYVDGDQCESNPCLNGGSCKDDINSYECWC
PFGFEGKNCELDVTCNIKNGRCEQFCKNSADNKVVCSCTEGYRLAENQKSCEPAVPFPC
GRVSVSQTSKLTRAETVFPDVDYVNSTEAETILDNITQSTQSFNDFTRVVGGEDAKPGQFP
WQVVLNGKVDAFCGGSIVNEKWIVTAAHCVETGVKITVVAGEHNIEETEHTEQKRNVIR
IIPHHNYNAAINKYNHDIALLELDEPLVLNSYVTPICIADKEYTNIFLKFGSGYVSGWGRV
FHKGRSALVLQYLRVPLVDRATCLRSTKFTIYNNMFCAGFHEGGRDSCQGDSGGPHVTE
VEGTSFLTGIISWGEECAMKGKYGIYTKVSRYVNWIKEKTKLTGGGGSGGGGSGGGGSG
GGGSGGGGSVAPPPALPAVAPPPALPAVAPPPALPAPVRLPGPADKTHTCPPCPAPELLGGPS
VFLFPPKPKDTLMISRTPEVTCVVVDVSHEDPEVKFNWYVDGVEVHNAKTKPREEQYAS
TYRVVSVLTVLHQDWLNGKEYKCKVSNKALPAPIEKTISKAKGQPREPQVYTLPPSRDEL
TKNQVSLTCLVKGFYPSDIAVEWESNGQPENNYKTTPPVLDSDGSFFLYSKLTVDKSRWQ
QGNVFSCSVMHEALHNHYTQKSLSLSPGKGSGGSGGGGSLPETGG (SEQ ID NO: 22).

Nucleotide sequence of FIX-C3-Fc-L2-P:

ATGCAGCGCGTCAATATGATTATGGCCGAGTCTCCAGGCCTCATTACCATTTGTCTGCT
GGGCTACCTGCTGTCCGCAGAGTGTACCGTCTTCCTCGACCACGAGAACGCAAATAA
GATTCTCAATAGGCCTAAGCGCTACAATTCCGGAAAGCTCGAAGAGTTTGTCCAGGGA
AATCTGGAGCGCGAGTGTATGGAGGAAAAGTGTAGCTTTGAAGAAGCTAGAGAGGTG
TTTGAGAATACTGAGCGCACTACTGAGTTTTGGAAGCAGTATGTGGATGGCGATCAGT
GCGAGTCCAATCCATGCCTGAATGGCGGATCTTGCAAGGATGATATTAATTCTTACGAG
TGTTGGTGCCCATTCGGCTTTGAGGGAAAGAATTGCGAACTGGATGTCACATGTAACA
TCAAGAACGGCAGATGCGAGCAGTTTTGTAAGAACAGCGCCGATAATAAGGTGGTGT
GTAGTTGTACTGAGGGATACCGCCTCGCAGAGAATCAGAAGTCCTGTGAGCCCGCTG
TGCCATTTCCTTGTGGCCGGGTCAGCGTCAGCCAGACTTCTAAGCTGACACGCGCCG
AGACCGTCTTTCCAGATGTGGATTATGTCAACTCTACTGAGGCCGAGACCATTCTCGAT
AATATTACTCAGTCTACACAGTCCTTTAACGACTTCACTCGGGTGGTGGGCGGAGAGG
ACGCAAAGCCTGGCCAGTTTCCTTGGCAGGTCGTGCTCAACGGAAAGGTGGACGCAT
TTTGTGGCGGATCTATCGTGAATGAGAAGTGGATTGTGACCGCCGCTCACTGCGTCGA
GACAGGCGTCAAGATTACCGTGGTCGCTGGAGAGCACAACATTGAGGAAACAGAGC
ACACCGAGCAGAAGCGCAATGTGATTCGCATCATCCCACACCACAACTACAACGCCG
CTATTAATAAGTACAACCACGACATCGCCCTGTTAGAACTAGATGAGCCTCTGGTCCTG
AATAGTTACGTCACTCCTATTTGTATCGCCGATAAAGAATACACTAACATCTTTCTCAA
GTTCGGCTCTGGCTATGTATCTGGATGGGGACGGGTGTTTCACAAGGGACGGTCCGCA
CTCGTGCTACAATACCTCCGGGTGCCACTGGTGGATCGGGCAACATGCCTCCGGTCTA
CCAAGTTTACTATATACAATAACATGTTTTGCGCCGGCTTTCACGAGGGCGGACGGGAT
AGTTGCCAGGGCGATAGTGGAGGCCCTCATGTCACAGAGGTGGAGGGAACTAGCTTT
CTGACAGGCATTATCTCTTGGGGCGAAGAGTGCGCTATGAAGGGAAAGTACGGCATCT
ATACTAAGGTCTCGCGTTACGTCAATTGGATCAAGGAAAAGACAAAGCTGACAGGCG
GCGGAGGGAGCGGCGGCGGAGGCTCTGGCGGCGGCGGATCTGGCGGAGGCGGCTCT
GGCGGAGGCGGATCTGTCGCTCCACCACCTGCACTCCCCGCCGTGGCTCCTCCACCT
GCTTTGCCCGCTGTCGCTCCACCACCTGCACTCCCCGCCCCTGTCAGACTGCCTGGAC
CCGCTGATAAGACCCACACATGTCCACCTTGCCCAGCACCTGAGCTGCTGGGCGGAC
CTAGCGTGTTTCTGTTCCCACCAAAGCCAAAGGACACCCTCATGATTAGTAGAACCCC
AGAGGTGACTTGCGTCGTGGTCGATGTCAGTCACGAGGACCCCGAGGTGAAGTTTAA
TTGGTATGTAGATGGCGTCGAGGTGCACAACGCTAAGACCAAGCCACGCGAAGAGCA

GTACGCATCTACTTACCGGGTCGTCTCTGTGCTGACTGTGCTGCACCAGGATTGGCTC
AATGGCAAAGAGTACAAGTGTAAGGTCAGTAATAAGGCCCTCCCCGCACCTATTGAA
AAGACTATTAGCAAGGCAAAGGGCCAGCCTAGGGAGCCCCAGGTCTACACCCTGCCC
CCTTCCCGCGATGAGCTGACTAAGAATCAGGTCTCTCTCACTTGCCTCGTGAAGGGCT
TTTACCCATCCGACATTGCCGTGGAGTGGGAGTCCAACGGACAGCCAGAGAATAACT
ACAAGACAACTCCTCCCGTGCTCGATAGCGACGGTTCATTCTTCCTGTACTCCAAGCT
CACCGTTGATAAGTCTCGGTGGCAGCAGGGAAATGTGTTCTCTTGTTCCGTCATGCAC
GAGGCACTGCACAATCACTACACCCAGAAGTCCCTGTCCCTCAGCCCTGGCAAAGGC
AGCGGCGGATCTGGAGGCGGCGGATCCCTCCCAGAGACCGGCGGATGA (SEQ ID NO:
23).

### b4) Construction of pFRL-rPS-FIX-C4-Fc-L2-P

The construction method is the same as for pFRL-rPS-FIX-C2-Fc-P. The PCR primers for amplifying the C4-Fc-L2-P gene include forward primers F5: 5'-CCCTGT CAGACTGCCTGGACCCGCTGTCGCTCCACCACCTGCACTCC-3' (SEQ ID NO: 37) and F6: 5'-TAGATCTGTCGCTCCACCACCTGCACTCCCCGCCCCTGTCAGACTGCCTGGACC-3' (SEQ ID NO: 38), and reverse primer R3. The C4-Fc-L2-P sequence was obtained by 2 rounds of PCR amplification.

Amino acid sequence of FIX-C4-Fc-L2-P:

MQRVNMIMAESPGLITICLLGYLLSAECTVFLDHENANKILNRPKRYNSGKLEEFVQGNL
ERECMEEKCSFEEAREVFENTERTTEFWKQYVDGDQCESNPCLNGGSCKDDINSYECWC
PFGFEGKNCELDVTCNIKNGRCEQFCKNSADNKVVCSCTEGYRLAENQKSCEPAVPFPC
GRVSVSQTSKLTRAETVFPDVDYVNSTEAETILDNITQSTQSFNDFTRVVGGEDAKPGQFP
WQVVLNGKVDAFCGGSIVNEKWIVTAAHCVETGVKITVVAGEHNIEETEHTEQKRNVIR
IIPHHNYNAAINKYNHDIALLELDEPLVLNSYVTPICIADKEYTNIFLKFGSGYVSGWGRV
FHKGRSALVLQYLRVPLVDRATCLRSTKFTIYNNMFCAGFHEGGRDSCQGDSGGPHVTE
VEGTSFLTGIISWGEECAMKGKYGIYTKVSRYVNWIKEKTKLTGGGGSGGGGSGGGGSG
GGGSGGGGSVAPPPALPAPVRLPGPAVAPPPALPAVAPPPALPADKTHTCPPCPAPELLGGPS
VFLFPPKPKDTLMISRTPEVTCVVVDVSHEDPEVKFNWYVDGVEVHNAKTKPREEQYAS
TYRVVSVLTVLHQDWLNGKEYKCKVSNKALPAPIEKTISKAKGQPREPQVYTLPPSRDEL

TKNQVSLTCLVKGFYPSDIAVEWESNGQPENNYKTTPPVLDSDGSFFLYSKLTVDKSRWQ
QGNVFSCSVMHEALHNHYTQKSLSLSPGGSGGSGGGGSLPETGG (SEQ ID NO: 24).

Nucleotide sequence of FIX-C4-Fc-L2-P

ATGCAGCGCGTCAATATGATTATGGCCGAGTCTCCAGGCCTCATTACCATTTGTCTGCT
GGGCTACCTGCTGTCCGCAGAGTGTACCGTCTTCCTCGACCACGAGAACGCAAATAA
GATTCTCAATAGGCCTAAGCGCTACAATTCCGGAAAGCTCGAAGAGTTTGTCCAGGGA
AATCTGGAGCGCGAGTGTATGGAGGAAAAGTGTAGCTTTGAAGAAGCTAGAGAGGTG
TTTGAGAATACTGAGCGCACTACTGAGTTTTGGAAGCAGTATGTGGATGGCGATCAGT
GCGAGTCCAATCCATGCCTGAATGGCGGATCTTGCAAGGATGATATTAATTCTTACGAG
TGTTGGTGCCCATTCGGCTTTGAGGGAAAGAATTGCGAACTGGATGTCACATGTAACA
TCAAGAACGGCAGATGCGAGCAGTTTTGTAAGAACAGCGCCGATAATAAGGTGGTGT
GTAGTTGTACTGAGGGATACCGCCTCGCAGAGAATCAGAAGTCCTGTGAGCCCGCTG
TGCCATTTCCTTGTGGCCGGGTCAGCGTCAGCCAGACTTCTAAGCTGACACGCGCCG
AGACCGTCTTTCCAGATGTGGATTATGTCAACTCTACTGAGGCCGAGACCATTCTCGAT
AATATTACTCAGTCTACACAGTCCTTTAACGACTTCACTCGGGTGGTGGGCGGAGAGG
ACGCAAAGCCTGGCCAGTTTCCTTGGCAGGTCGTGCTCAACGGAAAGGTGGACGCAT
TTTGTGGCGGATCTATCGTGAATGAGAAGTGGATTGTGACCGCCGCTCACTGCGTCGA
GACAGGCGTCAAGATTACCGTGGTCGCTGGAGAGCACAACATTGAGGAAACAGAGC
ACACCGAGCAGAAGCGCAATGTGATTCGCATCATCCCACACCACAACTACAACGCCG
CTATTAATAAGTACAACCACGACATCGCCCTGTTAGAACTAGATGAGCCTCTGGTCCTG
AATAGTTACGTCACTCCTATTTGTATCGCCGATAAAGAATACACTAACATCTTTCTCAA
GTTCGGCTCTGGCTATGTATCTGGATGGGGACGGGTGTTTCACAAGGGACGGTCCGCA
CTCGTGCTACAATACCTCCGGGTGCCACTGGTGGATCGGGCAACATGCCTCCGGTCTA
CCAAGTTTACTATATACAATAACATGTTTTGCGCCGGCTTTCACGAGGGCGGACGGGAT
AGTTGCCAGGGCGATAGTGGAGGCCCTCATGTCACAGAGGTGGAGGGAACTAGCTTT
CTGACAGGCATTATCTCTTGGGGCGAAGAGTGCGCTATGAAGGGAAAGTACGGCATCT
ATACTAAGGTCTCGCGTTACGTCAATTGGATCAAGGAAAAGACAAAGCTGACAGGCG
GCGGAGGGAGCGGCGGCGGAGGCTCTGGCGGCGGCGGATCTGGCGGAGGCGGCTCT
GGCGGAGGCGGATCTGTCGCTCCACCACCTGCACTCCCCGCCCCTGTCAGACTGCCT
GGACCCGCTGTCGCTCCACCACCTGCACTCCCCGCCGTGGCTCCTCCACCTGCTTTGC

CCGCTGATAAGACCCACACATGTCCACCTTGCCCAGCACCTGAGCTGCTGGGCGGAC
CTAGCGTGTTTCTGTTCCCACCAAAGCCAAAGGACACCCTCATGATTAGTAGAACCCC
AGAGGTGACTTGCGTCGTGGTCGATGTCAGTCACGAGGACCCCGAGGTGAAGTTTAA
TTGGTATGTAGATGGCGTCGAGGTGCACAACGCTAAGACCAAGCCACGCGAAGAGCA
GTACGCATCTACTTACCGGGTCGTCTCTGTGCTGACTGTGCTGCACCAGGATTGGCTC
AATGGCAAAGAGTACAAGTGTAAGGTCAGTAATAAGGCCCTCCCCGCACCTATTGAA
AAGACTATTAGCAAGGCAAAGGGCCAGCCTAGGGAGCCCCAGGTCTACACCCTGCCC
CCTTCCCGCGATGAGCTGACTAAGAATCAGGTCTCTCTCACTTGCCTCGTGAAGGGCT
TTTACCCATCCGACATTGCCGTGGAGTGGGAGTCCAACGGACAGCCAGAGAATAACT
ACAAGACAACTCCTCCCGTGCTCGATAGCGACGGTTCATTCTTCCTGTACTCCAAGCT
CACCGTTGATAAGTCTCGGTGGCAGCAGGGAAATGTGTTCTCTTGTTCCGTCATGCAC
GAGGCACTGCACAATCACTACACCCAGAAGTCCCTGTCCCTCAGCCCTGGCGGCAGC
GGCGGATCTGGAGGCGGCGGATCTCTCCCAGAGACCGGCGGATGA (SEQ ID NO: 25).

### b5) Construction of pFRL-rPS-FIX-C5-Fc-L2-P

Using FIX-C2-Fc-L2-P as a template, PCR amplification of FIX-C2 fragment was performed (forward primer F7: 5'-ATATAAGCTTCCGCCACCATGCAGCGC-3' (SEQ ID NO: 39) and reverse primer R4: 5'-ATATGGATCCAGCGGGCAAAGCAGGTGG AGGAGC-3' (SEQ ID NO: 40)). The obtained fragment was cloned into the vector pFRL-rPS-FIX-C2-Fc-L2-P through the HindIII/BamHI double restriction site to generate the bicistronic expression vector pFRL-rPS-FIX-C5-Fc-L2-P co-expressing FIX-C5-Fc-L2-P/Furin (C5 is actually a tandem of two C2).

Amino acid sequence of FIX-C5-Fc-L2-P:

MQRVNMIMAESPGLITICLLGYLLSAECTVFLDHENANKILNRPKRYNSGKLEEFVQGNL
ERECMEEKCSFEEAREVFENTERTTEFWKQYVDGDQCESNPCLNGGSCKDDINSYECWC
PFGFEGKNCELDVTCNIKNGRCEQFCKNSADNKVVCSCTEGYRLAENQKSCEPAVPFPC
GRVSVSQTSKLTRAETVFPDVDYVNSTEAETILDNITQSTQSFNDFTRVVGGEDAKPGQFP
WQVVLNGKVDAFCGGSIVNEKWIVTAAHCVETGVKITVVAGEHNIEETEHTEQKRNVIR
IIPHHNYNAAINKYNHDIALLELDEPLVLNSYVTPICIADKEYTNIFLKFGSGYVSGWGRV
FHKGRSALVLQYLRVPLVDRATCLRSTKFTIYNNMFCAGFHEGGRDSCQGDSGGPHVTE
VEGTSFLTGIISWGEECAMKGKYGIYTKVSRYVNWIKEKTKLTGGGGSGGGGSGGGGSG

GGGSGGGGSVAPPPALPAVAPPPALPAGSVAPPPALPAVAPPPALPADKTHTCPPCPAPELLG
GPSVFLFPPKPKDTLMISRTPEVTCVVVDVSHEDPEVKFNWYVDGVEVHNAKTKPREEQ
YASTYRVVSVLTVLHQDWLNGKEYKCKVSNKALPAPIEKTISKAKGQPREPQVYTLPPSR
DELTKNQVSLTCLVKGFYPSDIAVEWESNGQPENNYKTTPPVLDSDGSFFLYSKLTVDKSR
WQQGNVFSCSVMHEALHNHYTQKSLSLSPGGSGGSGGGGSLPETGG (SEQ ID NO: 26).

Nucleotide sequence of FIX-C5-Fc-L2-P:

ATGCAGCGCGTCAATATGATTATGGCCGAGTCTCCAGGCCTCATTACCATTTGTCTGCT
GGGCTACCTGCTGTCCGCAGAGTGTACCGTCTTCCTCGACCACGAGAACGCAAATAA
GATTCTCAATAGGCCTAAGCGCTACAATTCCGGAAAGCTCGAAGAGTTTGTCCAGGGA
AATCTGGAGCGCGAGTGTATGGAGGAAAAGTGTAGCTTTGAAGAAGCTAGAGAGGTG
TTTGAGAATACTGAGCGCACTACTGAGTTTTGGAAGCAGTATGTGGATGGCGATCAGT
GCGAGTCCAATCCATGCCTGAATGGCGGATCTTGCAAGGATGATATTAATTCTTACGAG
TGTTGGTGCCCATTCGGCTTTGAGGGAAAGAATTGCGAACTGGATGTCACATGTAACA
TCAAGAACGGCAGATGCGAGCAGTTTTGTAAGAACAGCGCCGATAATAAGGTGGTGT
GTAGTTGTACTGAGGGATACCGCCTCGCAGAGAATCAGAAGTCCTGTGAGCCCGCTG
TGCCATTTCCTTGTGGCCGGGTCAGCGTCAGCCAGACTTCTAAGCTGACACGCGCCG
AGACCGTCTTTCCAGATGTGGATTATGTCAACTCTACTGAGGCCGAGACCATTCTCGAT
AATATTACTCAGTCTACACAGTCCTTTAACGACTTCACTCGGGTGGTGGGCGGAGAGG
ACGCAAAGCCTGGCCAGTTTCCTTGGCAGGTCGTGCTCAACGGAAAGGTGGACGCAT
TTTGTGGCGGATCTATCGTGAATGAGAAGTGGATTGTGACCGCCGCTCACTGCGTCGA
GACAGGCGTCAAGATTACCGTGGTCGCTGGAGAGCACAACATTGAGGAAACAGAGC
ACACCGAGCAGAAGCGCAATGTGATTCGCATCATCCCACACCACAACTACAACGCCG
CTATTAATAAGTACAACCACGACATCGCCCTGTTAGAACTAGATGAGCCTCTGGTCCTG
AATAGTTACGTCACTCCTATTTGTATCGCCGATAAAGAATACACTAACATCTTTCTCAA
GTTCGGCTCTGGCTATGTATCTGGATGGGGACGGGTGTTTCACAAGGGACGGTCCGCA
CTCGTGCTACAATACCTCCGGGTGCCACTGGTGGATCGGGCAACATGCCTCCGGTCTA
CCAAGTTTACTATATACAATAACATGTTTTGCGCCGGCTTTCACGAGGGCGGACGGGAT
AGTTGCCAGGGCGATAGTGGAGGCCCTCATGTCACAGAGGTGGAGGGAACTAGCTTT
CTGACAGGCATTATCTCTTGGGGCGAAGAGTGCGCTATGAAGGGAAAGTACGGCATCT
ATACTAAGGTCTCGCGTTACGTCAATTGGATCAAGGAAAAGACAAAGCTGACAGGCG

GCGGAGGGAGCGGCGGCGGAGGCTCTGGCGGCGGCGGATCTGGCGGAGGCGGCTCT
GGCGGAGGCGGATCTGTCGCTCCACCACCTGCACTCCCCGCCGTGGCTCCTCCACCT
GCTTTGCCCGCTGGATCCGTCGCTCCACCACCTGCACTCCCCGCCGTGGCTCCTCCAC
CTGCTTTGCCCGCTGATAAGACCCACACATGTCCACCTTGCCCAGCACCTGAGCTGCT
GGGCGGACCTAGCGTGTTTCTGTTCCCACCAAAGCCAAAGGACACCCTCATGATTAGT
AGAACCCCAGAGGTGACTTGCGTCGTGGTCGATGTCAGTCACGAGGACCCCGAGGTG
AAGTTTAATTGGTATGTAGATGGCGTCGAGGTGCACAACGCTAAGACCAAGCCACGC
GAAGAGCAGTACGCATCTACTTACCGGGTCGTCTCTGTGCTGACTGTGCTGCACCAGG
ATTGGCTCAATGGCAAAGAGTACAAGTGTAAGGTCAGTAATAAGGCCCTCCCCGCACC
TATTGAAAAGACTATTAGCAAGGCAAAGGGCCAGCCTAGGGAGCCCCAGGTCTACAC
CCTGCCCCCTTCCCGCGATGAGCTGACTAAGAATCAGGTCTCTCTCACTTGCCTCGTG
AAGGGCTTTTACCCATCCGACATTGCCGTGGAGTGGGAGTCCAACGGACAGCCAGAG
AATAACTACAAGACAACTCCTCCCGTGCTCGATAGCGACGGTTCATTCTTCCTGTACTC
CAAGCTCACCGTTGATAAGTCTCGGTGGCAGCAGGGAAATGTGTTCTCTTGTTCCGTC
ATGCACGAGGCACTGCACAATCACTACACCCAGAAGTCCCTGTCCCTCAGCCCTGGC
GGCAGCGGCGGATCTGGAGGCGGCGGATCTCTCCCAGAGACCGGCGGATAA (SEQ ID
NO: 27).

### b6) Construction of pFRL-rPS-FIX-GS-Fc-L2-P

The construction method of the bicistronic expression vector pFRL-rPS-FIX-GS-Fc-L2-P co-expressing FIX-GS-Fc-L2-P/Furin is the same as that of pFRL-rPS-FIX-C2-Fc-L2-P. The primers for PCR amplification of the Fc-L2-P gene include forward primer F8: 5'-GATTTGGATCCGATAAGACCCACACATGTCCACCTTG-3' (SEQ ID NO: 41) and reverse primer R5: 5'-TAACC GGAATTCATTATCCGCCGGTCTCTGGGAGAGATCCGCCGCCTCCAGATCCGCCGCTGC CGCCAGGGCTGAGGGACAGGGACT-3' (SEQ ID NO: 42).

Amino acid sequence of FIX-GS-Fc-L2-P:

MQRVNMIMAESPGLITICLLGYLLSAECTVFLDHENANKILNRPKRYNSGKLEEFVQGNL
ERECMEEKCSFEEAREVFENTERTTEFWKQYVDGDQCESNPCLNGGSCKDDINSYECWC
PFGFEGKNCELDVTCNIKNGRCEQFCKNSADNKVVCSCTEGYRLAENQKSCEPAVPFPC

GRVSVSQTSKLTRAETVFPDVDYVNSTEAETILDNITQSTQSFNDFTRVVGGEDAKPGQFP
WQVVLNGKVDAFCGGSIVNEKWIVTAAHCVETGVKITVVAGEHNIEETEHTEQKRNVIR
IIPHHNYNAAINKYNHDIALLELDEPLVLNSYVTPICIADKEYTNIFLKFGSGYVSGWGRV
FHKGRSALVLQYLRVPLVDRATCLRSTKFTIYNNMFCAGFHEGGRDSCQGDSGGPHVTE
VEGTSFLTGIISWGEECAMKGKYGIYTKVSRYVNWIKEKTKLTGGGGSGGGGSGGGGSG
GGGSGGGGSDKTHTCPPCPAPELLGGPSVFLFPPKPKDTLMISRTPEVTCVVVDVSHEDPE
VKFNWYVDGVEVHNAKTKPREEQYASTYRVVSVLTVLHQDWLNGKEYKCKVSNKALP
APIEKTISKAKGQPREPQVYTLPPSRDELTKNQVSLTCLVKGFYPSDIAVEWESNGQPENN
YKTTPPVLDSDGSFFLYSKLTVDKSRWQQGNVFSCSVMHEALHNHYTQKSLSLSPGGSG
GSGGGGSLPETGG (SEQ ID NO: 28).

Nucleotide sequence of FIX-GS-Fc-L2-P:

ATGCAGCGCGTCAATATGATTATGGCCGAGTCTCCAGGCCTCATTACCATTTGTCTGCT
GGGCTACCTGCTGTCCGCAGAGTGTACCGTCTTCCTCGACCACGAGAACGCAAATAA
GATTCTCAATAGGCCTAAGCGCTACAATTCCGGAAAGCTCGAAGAGTTTGTCCAGGGA
AATCTGGAGCGCGAGTGTATGGAGGAAAAGTGTAGCTTTGAAGAAGCTAGAGAGGTG
TTTGAGAATACTGAGCGCACTACTGAGTTTTGGAAGCAGTATGTGGATGGCGATCAGT
GCGAGTCCAATCCATGCCTGAATGGCGGATCTTGCAAGGATGATATTAATTCTTACGAG
TGTTGGTGCCCATTCGGCTTTGAGGGAAAGAATTGCGAACTGGATGTCACATGTAACA
TCAAGAACGGCAGATGCGAGCAGTTTTGTAAGAACAGCGCCGATAATAAGGTGGTGT
GTAGTTGTACTGAGGGATACCGCCTCGCAGAGAATCAGAAGTCCTGTGAGCCCGCTG
TGCCATTTCCTTGTGGCCGGGTCAGCGTCAGCCAGACTTCTAAGCTGACACGCGCCG
AGACCGTCTTTCCAGATGTGGATTATGTCAACTCTACTGAGGCCGAGACCATTCTCGAT
AATATTACTCAGTCTACACAGTCCTTTAACGACTTCACTCGGGTGGTGGGCGGAGAGG
ACGCAAAGCCTGGCCAGTTTCCTTGGCAGGTCGTGCTCAACGGAAAGGTGGACGCAT
TTTGTGGCGGATCTATCGTGAATGAGAAGTGGATTGTGACCGCCGCTCACTGCGTCGA
GACAGGCGTCAAGATTACCGTGGTCGCTGGAGAGCACAACATTGAGGAAACAGAGC
ACACCGAGCAGAAGCGCAATGTGATTCGCATCATCCCACACCACAACTACAACGCCG
CTATTAATAAGTACAACCACGACATCGCCCTGTTAGAACTAGATGAGCCTCTGGTCCTG
AATAGTTACGTCACTCCTATTTGTATCGCCGATAAAGAATACACTAACATCTTTCTCAA
GTTCGGCTCTGGCTATGTATCTGGATGGGGACGGGTGTTTCACAAGGGACGGTCCGCA

CTCGTGCTACAATACCTCCGGGTGCCACTGGTGGATCGGGCAACATGCCTCCGGTCTA
CCAAGTTTACTATATACAATAACATGTTTTGCGCCGGCTTTCACGAGGGCGGACGGGAT
AGTTGCCAGGGCGATAGTGGAGGCCCTCATGTCACAGAGGTGGAGGGAACTAGCTTT
CTGACAGGCATTATCTCTTGGGGCGAAGAGTGCGCTATGAAGGGAAAGTACGGCATCT
ATACTAAGGTCTCGCGTTACGTCAATTGGATCAAGGAAAAGACAAAGCTGACAGGCG
GCGGAGGGAGCGGCGGCGGAGGCTCTGGCGGCGGCGGATCTGGCGGAGGCGGCTCT
GGCGGAGGCGGATCCGATAAGACCCACACATGTCCACCTTGCCCAGCACCTGAGCTG
CTGGGCGGACCTAGCGTGTTTCTGTTCCCACCAAAGCCAAAGGACACCCTCATGATTA
GTAGAACCCCAGAGGTGACTTGCGTCGTGGTCGATGTCAGTCACGAGGACCCCGAGG
TGAAGTTTAATTGGTATGTAGATGGCGTCGAGGTGCACAACGCTAAGACCAAGCCACG
CGAAGAGCAGTACGCATCTACTTACCGGGTCGTCTCTGTGCTGACTGTGCTGCACCAG
GATTGGCTCAATGGCAAAGAGTACAAGTGTAAGGTCAGTAATAAGGCCCTCCCCGCA
CCTATTGAAAAGACTATTAGCAAGGCAAAGGGCCAGCCTAGGGAGCCCCAGGTCTAC
ACCCTGCCCCCTTCCCGCGATGAGCTGACTAAGAATCAGGTCTCTCTCACTTGCCTCG
TGAAGGGCTTTTACCCATCCGACATTGCCGTGGAGTGGGAGTCCAACGGACAGCCAG
AGAATAACTACAAGACAACTCCTCCCGTGCTCGATAGCGACGGTTCATTCTTCCTGTA
CTCCAAGCTCACCGTTGATAAGTCTCGGTGGCAGCAGGGAAATGTGTTCTCTTGTTCC
GTCATGCACGAGGCACTGCACAATCACTACACCCAGAAGTCCCTGTCCCTCAGCCCT
GGCGGCAGCGGCGGATCTGGAGGCGGCGGATCTCTCCCAGAGACCGGCGGATAA
(SEQ ID NO: 29).

The structure diagram of the above FIX-L1-Fc-L2-P/Furin bicistronic expression vectors is shown in FIG. 1A.

Construction of GGCX and VKORC1 co-expressing vector

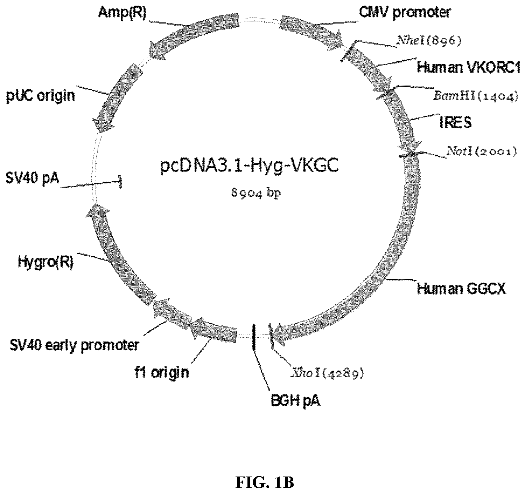

FIX is a vitamin K (VK)-dependent glycoprotein, and with the help of VK, the glutamate residue in its N-terminal Gla domain is carboxylated to form γ-carboxyglutamate, which gives FIX blood coagulation activity. During Gla modification, γ-glutamyl carboxylase (GGCX) and vitamin K epoxide reductase complex subunit 1 (VKORC 1) are two key enzymes. Therefore, the present invention constructed the plasmid pcDNA3.1-Hyg-VKGC to increase the expression level of functional FIX by co-transfection the plasmid with FIX. The pcDNA3.1-Hyg-VKGC plasmid structure is shown in FIG. 1B, and the construction method is as follows: gamma glutamyl carboxylase (GGCX), vitamin K epoxide reductase complex subunit 1 (VKORC1) and internal ribosome entry site (IRES) DNA were synthesized by full-gene synthesis; the IRES was cloned into the vector pcDNA3.1(+)/hygro through the NheI/XhoI double restriction site to construct the plasmid p3.1hyg-IRES; then, through different double restriction sites, VKORC1 gene (by NheI/BamHI) and GGCX gene (by NotI/XhoI) were cloned into p3.1hyg-IRES to construct the GGCX and VKORC1 co-expressing vector pcDNA3.1-Hyg-VKGC.

### Construction of stable cell lines

Each type of plasmids expressing the FIX fusion protein constructed above was transfected into the host cell CHO DG44 by electrotransfection. For electroporation, 1×10⁷ cells, 40 µg of FIX-L1-Fc-L2-P/Furin co-expressing plasmid and 20 µg of pcDNA3.1-Hyg-VKGC expression plasmid were gently mixed and added to the electroporation cuvettes (Bio-Rad, 4 mm). Bio-Rad (GENE PULSER XCELL) electroporator was used and the electroporation parameters were set as follows:

| Voltage(V) | Pulse length (ms) | Number of Pulses | Pulse interval (sec) | Cuvette (mm) |
|---|---|---|---|---|
| 290 | 20 | 1 | 0 | 4 |

After electroporation, the cells were transferred to recovery medium. After 24 hours of culture, the cells were seeded in fifteen 96-well plates, and the medium was changed to screening medium containing Opti-MEM I+50 nM MTX. After the cells were cultured for 4 to 5 days, medium containing 50 nM MTX was supplemented 2 to 3 times. When the cell confluence reached 50% or more, the clones were screened for high expression of target protein by Dot Blotting method, and the antibody used was mouse anti-human IgG Fc. The selected clones with relatively high expression levels were sequentially transferred to 24-well plates, 6-well plates, cell culture dishes and cell culture shake flasks for expansion.

In order to increase the yield of fusion protein, the cells were cultured under pressure with increasing MTX concentration. In this way, through the inhibition of DHFR gene by MTX, the co-amplification of DHFR gene and fusion protein gene was realized.

### Production of fusion protein

### 3.1 Cell thawing and passaging

Cells were thawed and cultured in culture flasks, and after the density increased, the cells were transferred to shake flasks containing CD DG44 Medium+5mg/L VK for culture and passage. Passage was performed when the cell density reached the range of 2.5-3.5×10⁶ cells/mL, and the passage density was between 0.8-1.0×10⁶ cells/mL. When cell seeds were expanded to 1.8-2 L and the density reached about 3.5×10⁶ cells/mL, the cells were cultured in tanks.

### 3.2 Fermentation

A 15L bioreactor (Applikon, Biobundle 15L) was used for fermentation, the inoculation volume was 8 L, and the cell density was 1.0×10⁶ cells/mL. The culture parameters were set as follows: 1) pH 6.8-7.4; 2) dissolved oxygen (DO) 40%; 3) oxygen supply mode: large bubble ventilation; 4) cooling strategy: culturing at 37°C in the early stage, and cooling to 33°C on the 5th day; 5) rotation speed: 80-140 rpm; 6) feeding strategy: feeding on the fourth day at an amount of 2%, 3%, 4%, 3%, 2%, 2%, 2%, 2%, and 2%, and the total feeding volume was 24%, and the cells were counted with a cell counter every day; 7) harvest: after culturing for about 13 days, harvesting the supernatant by centrifugation at 3000 g for 10 min.

### 3.3 Sample detection and purification

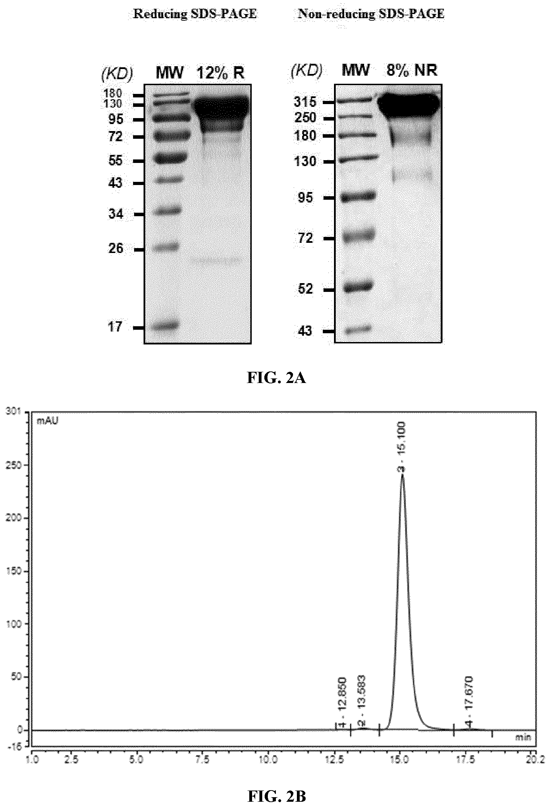

The supernatant of the fermentation broth was purified in three steps: affinity chromatography, mixed-mode chromatography and anion exchange chromatography. The purified fusion protein was detected by SDS-PAGE and SEC-HPLC. As shown in FIG. 2A, SDS-PAGE results show that the size of the interest proteins were basically the same as the theoretical molecular weight.

SEC-HPLC was performed on Agilent Advancebio SEC 300A, 2.7 µm, 7.8^{∗}300 mm column, and gradient elution was performed using 50 mM Tris+150 mM NaCl, pH 7.2 eluent, wherein the column temperature was 30°C, flow rate was 0.5 mL/min, sample injector temperature was 4.0 °C, and sample injector volume was 30 µg. FIG. 2B shows the purification results using FIX-C1-Fc-L2-P protein as an example, for which the retention time was 15.1 min, and the SEC purity after three-step purification was 95.0%.

### Example 2 In Vitro Activity Detection of FIX-L1-Fc-L2-P and Comparison

In this example, the *in vitro* activity of FIX-C1-Fc-L2-P, FIX-C2-Fc-L2-P, FIX-C3-Fc-L2-P, FIX-C4-Fc-L2-P and FIX-C5-Fc-L2-P prepared in Example 1 were tested and compared with the *in vitro* activity of FIX-GS-Fc-L2-P devoid of a rigid unit in the first linker L1.

The activity of human coagulation factor IX (hFIX) was measured by the one-stage method based on activated partial thromboplastin time (APTT), where ellagic acid was used as the activator, and the 8^{th} International Standard 2009 FIX Concentrate (the standard for WHO FIX activity) was used for APTT determination. The logarithms of both the activity of the known standard and the measured APTT time were used for linear fitting to plot the standard curve. The sample to be tested was diluted to fit the range of the standard curve, and mixed with FIX-deficient plasma to measure the APTT value. The biological activity of the sample to be tested was calculated based on the standard curve.

The aliquoted WHO FIX active standard or sample was equilibrated to room temperature, then 10 µl of which was pipetted and diluted to about 1 IU/ml with 5% FIX-deficient plasma, and then was diluted 10 times and 20 times (and further diluted 40 times and 80 times for the active standard) with 5% FIX-deficient plasma and vortexed to mix well. The diluted standard or sample was placed on the analysis sample rack of the automatic coagulometer, and the reagents used were placed in the corresponding reagent positions. Then the corresponding measurement program on the automatic coagulation analyzer was employed to determine the coagulation time, and the measurement was repeated twice for each dilution. The logarithm of the standard activity was served as the abscissa and the logarithm of the coagulation time as the ordinate to plot the standard curve. The measured coagulation time of the diluted test product was substituted into the standard curve to calculate the activity result, which was then multiplied by the dilution factor to obtain the corresponding activity.

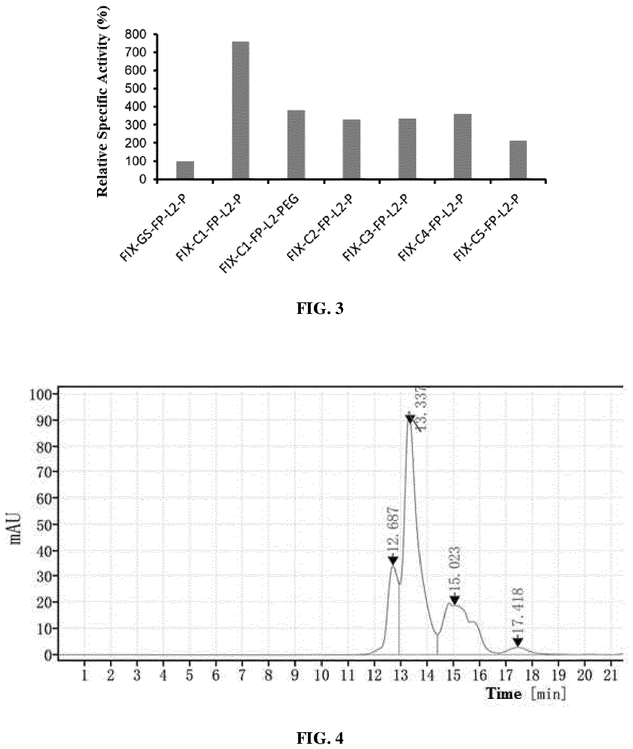

It was unexpectedly found that, as shown in FIG. 3, the specific activities of the FIX-C1-Fc-L2-P, FIX-C2-Fc-L2-P, FIX-C3-Fc-L2-P, FIX-C4-Fc-L2-P and FIX-C5-Fc-L2-P molecules in which the first linker L1 contained a rigid unit were much higher than that of the FIX-GS-Fc-L2-P in which the first linker L1 was devoid of a rigid unit, indicating that the first linker L1 containing the rigid unit can significantly increase the specific activity of the molecule.

In addition, unexpectedly, among all the fusion proteins containing the rigid unit in L1, FIX-C1-Fc-L2-P had significantly higher activity, which was nearly double or above than other fusion proteins in terms of specific activity (FIG. 3). Therefore, in the subsequent examples, FIX-C1-Fc-L2-P was selected for in-depth study.

### Example 3 Preparation of PEG-conjugated Fusion Protein FIX-C1-Fc-L2-PEG

FIX-C1-Fc-L2-P protein contains LPETGG at C-terminus, which can be specifically recognized by Sortase A. Under the action of Sortase A, the amide bond between T and G was cut, and T reacted with the sulfhydryl group of C at Sortase position 184 to form a thioester bond intermediate, which was then attacked by GGGAA-PEG with poly-Gly at the N-terminal. Finally, PEG of a specific molecular weight can be linked to the C-terminal of the protein.

PEGlation (pegylation) reaction system: 40KD-GGGAA-PEG was dissolved in buffer and adjusted to pH 7.5. Then the protein and 40KD-GGGAA-PEG were added to the 50 mM Tris 150 mM NaCl pH 7.5 buffer system at a feeding ratio of 1:20, and then Sortase A and 10 mM CaCl₂ were added. After 30 minutes, EDTA was added to terminate the reaction.

The conjugation rate of the conjugate (FIX-C1-Fc-L2-PEG) was tested by SEC-HPLC, which was performed on TOSOH TSKgel UItraSW Aggregate 7.8^{∗}300 mm 3 µm column, and gradient elution was performed using 50 mM Tris+150 mM NaCl, pH 7.2 elution buffer, where the column temperature was 25 °C, flow rate was 0.5 mL/min, sample injector temperature was 4.0 °C, sample injector volume was 50 µg, the retention time of the conjugated protein was 12-14.5 min, and the retention time of the substrate was 15 min. FIG. 4 shows the detection results of FIX-C1-Fc-L2-PEG, wherein the proportion of PEG-conjugated product reached 70% at 30 min.

### Purification and detection of PEG-conjugated product

The PEG-conjugated products were subjected to anion exchange chromatography to remove unreacted GGGAA-PEG, Sortase A and unconjugated substrate (FIX-C1-Fc-L2-P), and the residual amount of unconjugated substrate was controlled below 3%.

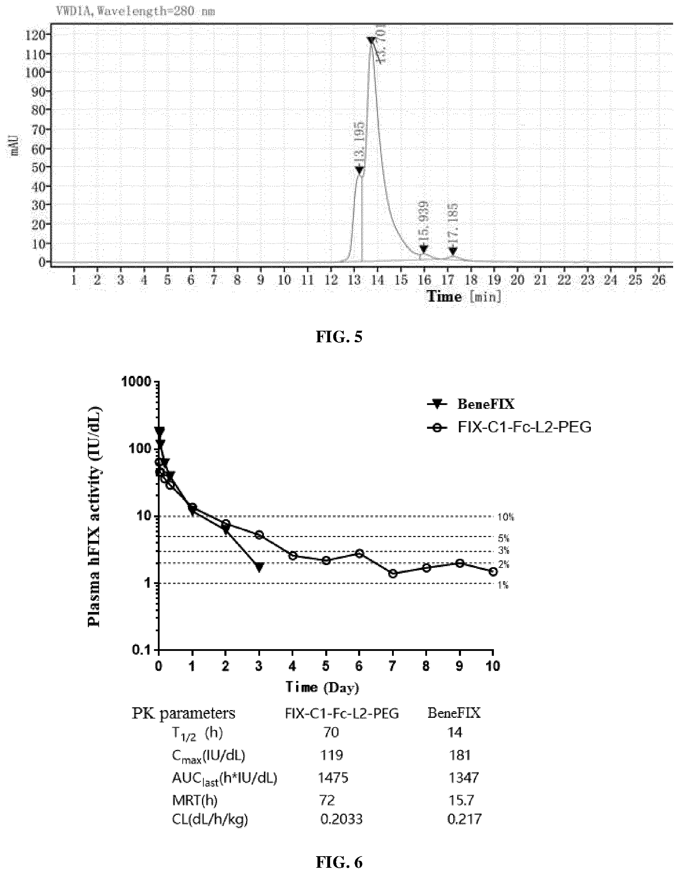

The conjugated samples were separated by anion exchange chromatography, and peaks were collected at different stages. The samples collected at each peak were detected by SDS-HPLC and SEC, and then the samples with a ratio of unconjugated substrate less than 5% were combined to detect the purity by SEC. The SEC detection result of FIX-C1-Fc-L2-PEG is shown in FIG. 5, wherein the SEC purity of the PEGylated product was 97.8%. Among them, the double-conjugated product accounted for 15.7%, with a retention time of 13.20 min, the single-conjugated product accounted for 82.2% with a retention time of 13.70 min, and the unreacted substrate FIX-C1-Fc-L2-P accounted for 1.24% with a retention time of 15.94 min.

### Example 4 FIX-C1-Fc-L2-PEG Conjugate Maintaining Excellent In Vitro Activity

This example further tested the *in vitro* activity of the FIX-C 1-Fc-L2-PEG conjugate. The activity of human coagulation factor IX (hFIX) was measured by the one-stage method based on activated partial thromboplastin time (APTT), where ellagic acid was used as the activator, and the 8th International Standard 2009 FIX Concentrate (the standard for WHO FIX activity) was used for APTT determination. The logarithms of the activity of the known standard and the measured APTT time were used for linear fitting to plot the standard curve. The sample to be tested was diluted to fit the range of the standard curve, and mixed with FIX-deficient plasma to measure the APTT value. The biological activity of the sample to be tested was calculated based on the standard curve.

The aliquoted WHO FIX active standard or sample was equilibrated to room temperature, then 10 µl of which was pipetted and diluted to about 1 IU/ml with 5% FIX-deficient plasma, and then was diluted 10 times and 20 times (and further diluted 40 times and 80 times for the active standard) with 5% FIX-deficient plasma and vortexed to mix well. The diluted standard or sample was placed on the analysis sample rack of the automatic coagulometer, and the reagents used were placed in the corresponding reagent positions. Then the corresponding measurement program on the automatic coagulation analyzer was employed to determine the coagulation time, and the measurement was repeated twice for each dilution. The logarithm of the standard activity was served as the abscissa and the logarithm of the coagulation time as the ordinate to plot the standard curve. The measured coagulation time of the diluted test product was substituted into the standard curve to calculate the activity result, which was then multiplied by the dilution factor to obtain the corresponding activity.

As shown in FIG. 3, the specific activity of the conjugate FIX-C1-Fc-L2-PEG was lower than that of FIX-C1-Fc-L2-P, which reflects that PEG conjugation will reduce the *in vitro* specific activity of the molecule to a certain extent, and the above results are consistent with the common understanding in the field. However, it is also worth noting that the *in vitro* activity of FIX-C1-Fc-L2-PEG was still maintained at a relatively high level, not only much higher than that of FIX-GS-Fc-L2-P without PEG conjugation, but also comparable to that of other FIX-L1-Fc-L2-P fusion proteins without PEG conjugation, except for FIX-C1-Fc-L2-P.

### Example 5 FIX-C1-Fc-L2-PEG showing a significantly extended half-life in vivo

This example further tested the activity of the FIX-C 1-Fc-L2-PEG conjugate in mice. Specifically, FIX-C1-Fc-L2-PEG was administered to HB mice, and blood sample was collected from the orbit at different time points. Then the plasma was separated for detecting the hFIX activity in the plasma by the one-stage method, and the pharmacokinetics parameters were calculated by the software WinNonlin 5.2.1.

The experimental animals used in this example are shown in the following table:

| Name | Gender | Grade | Source |
|---|---|---|---|
| HB | ♂ and ♀ | SPF | Shanghai Model Organisms Center, Inc. |

The test samples used in this example are as follows:

| Name | Batch No. | Concentration (IU/ml) |
|---|---|---|
| FIX-C1-Fc-L2-PEG | 20181226 | 180 |
| BeneFIX (recombinant human coagulation factor IX injection) | N64048 | 50 |

This experiment was divided into 2 groups. The control drug BeneFIX and the FIX-C1-Fc-L2-PEG conjugate sample were administered through the tail vein at 300 IU/kg. The blood collection time points were: 1) BeneFIX: 0.083, 0.5, 1, 4, 8, 24, 48, and 72h; 2) FIX-C1-Fc-L2-PEG: 0.083, 0.5, 1, 4, 8, 24, 48, 72, 96, 120, 144, 168, 192, 216, and 240h. For each blood collection time point in each group, 5 HB animals (2 males, 3 females) were subjected to orbital blood collection and hFIX activity detection.

As shown in FIG. 6, BeneFIX can maintain plasma hFIX level at above 5% for 2 days after administration, but only 1%-2% plasma hFIX level 3 days after administration; while FIX-C1-Fc-L2-PEG still maintained plasma hFIX level at above 5% for 3 days after administration, and plasma hFIX level above 2% 6 days after administration, especially plasma hFIX level can still be maintained at 1%-2% 10 days after administration. The experimental results show that the elimination phase half-life (T1/2) of FIX-C1-Fc-L2-PEG reaches 70h, much greater than the T1/2 of the control drug BeneFIX (14h; consistent with the half-life data reported in literature) and the T1/2 of Alprolix (46h) reported in literature (Peters et al. Prolonged activity of factor IX as a monomeric Fc fusion protein. Blood 2010; 115(10): 2057-64.). The maximum blood drug concentration (Cmax) was 119 IU/dL, the drug-time area under the curve (AUClast) was 1475 h^{∗}IU/dL, the clearance rate (CL) was 0.2033 dL/h/kg, and the mean residence time (MRT) was 72h, achieving excellent pharmacodynamic effect. It can be seen that the PEG site-directed conjugate prepared by fusing hFIX with Fc significantly increases the half-life of the hFIX molecule, and has significantly better long-acting effects *in vivo* than existing drugs, which has good application prospects.

## Claims

1. A fusion protein of coagulation factor IX (FIX), comprising a coagulation factor IX active moiety and a fusion partner (FP) capable of extending the half-life of the fusion protein, wherein the coagulation factor IX active moiety and the fusion partner are linked directly or through a first linker L1;
preferably, the first linker L1 is a peptide containing 1, 2, 3, 4, 5, 6, 7, 8, 9, 10, or more amino acids;
more preferably, the first linker L1 comprises a flexible peptide and/or a rigid unit, and the flexible peptide is a peptide containing glycine (Gly, G), serine (Ser, S), alanine (Ala, A) and/or threonine (Thr, T), for example, the flexible peptide is (GS)ₘ(GGS)ₙ(GGGS)ₒ(GGGGS)ₚ, where m, n, o and p are independently selected from integers from 0 to 50, such as 0, 1, 2, 3, 4, 5, 6, 7, 8, 9, 10, 11, 12, 13, 14, 15, 16, 17, 18, 19 or 20, preferably m, n, o and p are not all 0.

2. The fusion protein according to claim 1, wherein the coagulation factor IX active moiety is derived from human, such as full-length or truncated human coagulation factor IX; the full-length or truncated human coagulation factor IX may contain one or more amino acid mutations, provided that it still retains the FIX activity, for example, the coagulation factor IX active moiety comprises the amino acid sequence shown in SEQ ID NO: 1, or has at least 85%, 90%, 95% or higher identity to the amino acid sequence shown in SEQ ID NO: 1.

3. The fusion protein according to claim 1 or 2, wherein the rigid unit is a peptide containing 1, 2, 3, 4, 5, 6, 7, 8, 9, 10 or more proline (Pro, P):
preferably, the rigid unit comprises an amino acid sequence selected from the group consisting of:
VAPPPALPAPVRLPGPA (SEQ ID NO: 3),
VAPPPALPAVAPPPALPA (SEQ ID NO: 4),
VAPPPALPAVAPPPALPAVAPPPALPAPVRLPGPA (SEQ ID NO: 5),
VAPPPALPAPVRLPGPAVAPPPALPAVAPPPALPA (SEQ ID NO: 6), and
VAPPPALPAVAPPPALPAGSVAPPPALPAVAPPPALPA (SEQ ID NO: 7);
preferably, the first linker L1 is selected from the group consisting of:
GGGGSGGGGSGGGGSGGGGSGGGGSVAPPPALPAPVRLPGPA (SEQ ID NO: 8),
GGGGSGGGGSGGGGSGGGGSGGGGSVAPPPALPAVAPPPALPA (SEQ ID NO: 9), 
GGGGSGGGGSGGGGSGGGGSGGGGSVAPPPALPAVAPPPALPAVAPPPALPAPVRL
PGPA (SEQ ID NO: 10),
 
GGGGSGGGGSGGGGSGGGGSGGGGSVAPPPALPAPVRLPGPAVAPPPALPAVAPPP
ALPA (SEQ ID NO: 11),
 and

GGGGSGGGGSGGGGSGGGGSGGGGSVAPPPALPAVAPPPALPAGSVAPPPALPAVA
PPPALPA (SEQ ID NO: 12).

4. The fusion protein according to any one of claims 1 to 3, wherein the fusion partner is a full-length, truncated or variant form of immunoglobulin Fc fragment, albumin, transferrin or XTEN; the fusion partner is, for example, derived from human, for example, the Fc fragment is derived from a human immunoglobulin Fc fragment;
preferably, the Fc fragment is composed of one to four domains selected from the group consisting of CH1 domain, CH2 domain, CH3 domain and CH4 domain;
preferably, the Fc fragment is derived from the Fc fragment of IgG, IgA, IgD, IgE, or IgM, more preferably IgG Fc fragment;
more preferably, the Fc fragment is derived from the Fc fragment of IgG1, IgG2, IgG3, or IgG4, and
more preferably, the IgG Fc fragment has reduced ADCC effect and/or CDC effect and/or enhanced binding affinity to FcRn.

5. The fusion protein according to any one of claims 1 to 4, wherein the fusion partner is also linked directly or via a second linker L2 to a peptide P containing a Sortase recognition site, and wherein the Sortase is, for example, Sortase A or Sortase B;
preferably, the amino acid sequence of P contains the core recognition site LPXTG of Sortase A, wherein X is any amino acid, and the amino acid sequence of P is, such as LPETG, LPETGG or LPETGGG;
more preferably, the P further contains an affinity tag linked to the Sortase recognition site, and the amino acid sequence of P is, for example, LPETGGHHHHHH or LPETGGWSHPQFEK.

6. The fusion protein according to claim 5, wherein the second linker L2 is a peptide containing 1, 2, 3, 4, 5, 6, 7, 8, 9, 10 or more amino acids;
preferably, the second linker L2 is a flexible peptide fragment containing glycine (Gly, G), serine (Ser, S), alanine (Ala, A) and/or threonine (Thr, T), such as (GS)_{w}(GGS)ₓ(GGGS)_{y}(GGGGS)_{z}, where w, x, y and z are independently selected from integers from 0 to 50, such as 0, 1, 2, 3, 4, 5, 6, 7, 8, 9, 10, 11, 12, 13, 14, 15, 16, 17, 18, 19 or 20, preferably w, x, y and z are not all 0;
preferably, the second linker L2 is selected from the group consisting of GGGGS (SEQ ID NO: 14), GGGGSGGGGS (SEQ ID NO: 15) and GSGGSGGGGS (SEQ ID NO: 16).

7. The fusion protein according to claim 6, which has a structure of FIX-L1-FP-L2-P, wherein FIX, L1, L2, FP, and P have the same definitions as in claim 6, and wherein either or both of L1 and L2 may not be present.

8. The fusion protein according to any one of claims 1 to 7, comprising the sequence shown in SEQ ID NO: 18, 20, 22, 24, 26 or 28, or encoded by the nucleic acid sequence shown in SEQ ID NO: 19, 21, 23, 25, 27, or 29.

9. A conjugate, wherein a hydrophilic polymer is attached to the end of the fusion protein according to any one of claims 1 to 8;
preferably, the hydrophilic polymer is attached to the Sortase recognition site via a transpeptidation reaction;
in particular, the fusion protein may be in a form of monomer or dimer.

10. The conjugate according to claim 9, wherein the hydrophilic polymer is selected from polysaccharide and polyalkylene glycol, such as polypropylene glycol and polyethylene glycol;
the polyalkylene glycol may be end-capped, for example, capped with alkoxy group such as methoxy group, and/or the polyalkylene glycol is linear or branched; the molecular weight of the polyalkylene glycol may be ≥1, ≥10, ≥20, ≥30, ≥40, ≥50, ≥60, ≥70, ≥80, ≥90, ≥100, ≥110, ≥120, ≥130, ≥140, ≥150, or ≥160 kDa, for example, 5, 10, 20, 30, 40, 50, 60, 70, 80, 90, or 100 kDa, or a value between any two of the values; and preferably the molecular weight of the hydrophilic polymer is 20 kDa or 40 kDa.

11. A method for producing the conjugate according to claim 9 or 10, comprising contacting the fusion protein of any one of claims 1 to 8 with Sortase (such as Sortase A or Sortase B) and the hydrophilic polymer, wherein one end of the hydrophilic polymer has an amino group that can be amidated by Sortase.

12. The method according to claim 11, wherein the N-terminus of the hydrophilic polymer contains poly-Gly, such as GGGAA.

13. A pharmaceutical composition comprising an effective amount of the fusion protein according to any one of claims 1 to 8 and/or the conjugate according to claim 9 or 10, and optionally a pharmaceutically acceptable carrier.

14. The pharmaceutical composition according to claim 13 for use in the prevention and/or treatment of a hemorrhagic disease, wherein the hemorrhagic disease is preferably selected from hemorrhagic diseases in patients with congenital or acquired deficiency of FIX, and spontaneous or surgical bleeding in patients with hemophilia B.

15. The pharmaceutical composition according to claim 13 or 14, wherein the pharmaceutical composition is in a form of liquid preparation or lyophilized preparation.

16. Use of the fusion protein according to any one of claims 1 to 8, the conjugate according to claim 9 or 10, or the pharmaceutical composition according to any one of claims 13 to 15 for the manufacture of a medicament for the prevention and/or treatment of a hemorrhagic disease, wherein the hemorrhagic disease is preferably selected from hemorrhagic diseases in patients with congenital or acquired deficiency of FIX, and spontaneous or surgical bleeding in patients with hemophilia B.

17. A method for preventing and/or treating a hemorrhagic disease, comprising administering the fusion protein according to any one of claims 1 to 8, the conjugate according to claim 9 or 10, or the pharmaceutical composition according to any one of claims 13 to 15 to a subject in need thereof, wherein the hemorrhagic disease is preferably selected from hemorrhagic diseases in patients with congenital or acquired deficiency of FIX, and spontaneous or surgical bleeding in patients with hemophilia B.

18. A kit comprising the fusion protein according to any one of claims 1 to 8, the conjugate according to claim 9 or 10, or the pharmaceutical composition according to any one of claims 13 to 15, and optionally instructions for use.

19. A nucleic acid molecule encoding the fusion protein according to any one of claims 1 to 8.

20. An expression vector comprising the nucleic acid molecule according to claim 19.


SEQUENCE LISTING

<110>  JIANGSU GENSCIENCES INC.

<120>  IMPROVED FIX FUSION PROTEIN AND CONJUGATE AND USE THEREOF

<130>  PCT/CN2020/097911

<150>  CN 201910591280.0
<151>  2019-07-02

<150>  PCT/CN2020/097911
<151>  2020-06-24

<160>  42

<170>  PatentIn version 3.2

<210>  1
<211>  461
<212>  PRT
<213>  Homo sapiens

<400>  1

<210>  2
<211>  1386
<212>  DNA
<213>  Homo sapiens

<400>  2
atgcagcgcg tgaacatgat catggcagaa tcaccaggcc tcatcaccat ctgcctttta      60

ggatatctac tcagtgctga atgtacagtt tttcttgatc atgaaaacgc caacaaaatt     120

ctgaatcggc caaagaggta taattcaggt aaattggaag agtttgttca agggaacctt     180

gagagagaat gtatggaaga aaagtgtagt tttgaagaag cacgagaagt ttttgaaaac     240

actgaaagaa caactgaatt ttggaagcag tatgttgatg gagatcagtg tgagtccaat     300

ccatgtttaa atggcggcag ttgcaaggat gacattaatt cctatgaatg ttggtgtccc     360

tttggatttg aaggaaagaa ctgtgaatta gatgtaacat gtaacattaa gaatggcaga     420

tgcgagcagt tttgtaaaaa tagtgctgat aacaaggtgg tttgctcctg tactgaggga     480

tatcgacttg cagaaaacca gaagtcctgt gaaccagcag tgccatttcc atgtggaaga     540

gtttctgttt cacaaacttc taagctcacc cgtgctgaga ctgtttttcc tgatgtggac     600

tatgtaaatt ctactgaagc tgaaaccatt ttggataaca tcactcaaag cacccaatca     660

tttaatgact tcactcgggt tgttggtgga gaagatgcca aaccaggtca attcccttgg     720

caggttgttt tgaatggtaa agttgatgca ttctgtggag gctctatcgt taatgaaaaa     780

tggattgtaa ctgctgccca ctgtgttgaa actggtgtta aaattacagt tgtcgcaggt     840

gaacataata ttgaggagac agaacataca gagcaaaagc gaaatgtgat tcgaattatt     900

cctcaccaca actacaatgc agctattaat aagtacaacc atgacattgc ccttctggaa     960

ctggacgaac ccttagtgct aaacagctac gttacaccta tttgcattgc tgacaaggaa    1020

tacacgaaca tcttcctcaa atttggatct ggctatgtaa gtggctgggg aagagtcttc    1080

cacaaaggga gatcagcttt agttcttcag taccttagag ttccacttgt tgaccgagcc    1140

acatgtcttc gatctacaaa gttcaccatc tataacaaca tgttctgtgc tggcttccat    1200

gaaggaggta gagattcatg tcaaggagat agtgggggac cccatgttac tgaagtggaa    1260

gggaccagtt tcttaactgg aattattagc tggggtgaag agtgtgcaat gaaaggcaaa   1320

tatggaatat ataccaaggt atcccggtat gtcaactgga ttaaggaaaa aacaaagctc   1380

acttaa                                                              1386

<210>  3
<211>  17
<212>  PRT
<213>  Artificial sequence

<220>
<223>  Rigid unit

<400>  3

Val Ala Pro Pro Pro Ala Leu Pro Ala Pro Val Arg Leu Pro Gly Pro
1               5                   10                  15

Ala

<210>  4
<211>  18
<212>  PRT
<213>  Artificial sequence

<220>
<223>  Rigid unit

<400>  4

Val Ala Pro Pro Pro Ala Leu Pro Ala Val Ala Pro Pro Pro Ala Leu
1               5                   10                  15

Pro Ala

<210>  5
<211>  35
<212>  PRT
<213>  Artificial sequence

<220>
<223>  Rigid unit

<400>  5

Val Ala Pro Pro Pro Ala Leu Pro Ala Val Ala Pro Pro Pro Ala Leu
1               5                   10                  15

Pro Ala Val Ala Pro Pro Pro Ala Leu Pro Ala Pro Val Arg Leu Pro
            20                  25                  30

Gly Pro Ala
        35

<210>  6
<211>  35
<212>  PRT
<213>  Artificial sequence

<220>
<223>  Rigid unit

<400>  6

Val Ala Pro Pro Pro Ala Leu Pro Ala Pro Val Arg Leu Pro Gly Pro
1               5                   10                  15

Ala Val Ala Pro Pro Pro Ala Leu Pro Ala Val Ala Pro Pro Pro Ala
            20                  25                  30

Leu Pro Ala
        35

<210>  7
<211>  38
<212>  PRT
<213>  Artificial sequence

<220>
<223>  Rigid unit

<400>  7

Val Ala Pro Pro Pro Ala Leu Pro Ala Val Ala Pro Pro Pro Ala Leu
1               5                   10                  15

Pro Ala Gly Ser Val Ala Pro Pro Pro Ala Leu Pro Ala Val Ala Pro
            20                  25                  30

Pro Pro Ala Leu Pro Ala
        35

<210>  8
<211>  42
<212>  PRT
<213>  Artificial sequence

<220>
<223>  First linker L1: C1

<400>  8

Gly Gly Gly Gly Ser Gly Gly Gly Gly Ser Gly Gly Gly Gly Ser Gly
1               5                   10                  15

Gly Gly Gly Ser Gly Gly Gly Gly Ser Val Ala Pro Pro Pro Ala Leu
            20                  25                  30

Pro Ala Pro Val Arg Leu Pro Gly Pro Ala
        35                  40

<210>  9
<211>  43
<212>  PRT
<213>  Artificial sequence

<220>
<223>  First linker L1: C2

<400>  9

Gly Gly Gly Gly Ser Gly Gly Gly Gly Ser Gly Gly Gly Gly Ser Gly
1               5                   10                  15

Gly Gly Gly Ser Gly Gly Gly Gly Ser Val Ala Pro Pro Pro Ala Leu
            20                  25                  30

Pro Ala Val Ala Pro Pro Pro Ala Leu Pro Ala
        35                  40

<210>  10
<211>  60
<212>  PRT
<213>  Artificial sequence

<220>
<223>  First linker L1: C3

<400>  10

Gly Gly Gly Gly Ser Gly Gly Gly Gly Ser Gly Gly Gly Gly Ser Gly
1               5                   10                  15

Gly Gly Gly Ser Gly Gly Gly Gly Ser Val Ala Pro Pro Pro Ala Leu
            20                  25                  30

Pro Ala Val Ala Pro Pro Pro Ala Leu Pro Ala Val Ala Pro Pro Pro
        35                  40                  45

Ala Leu Pro Ala Pro Val Arg Leu Pro Gly Pro Ala
    50                  55                  60

<210>  11
<211>  60
<212>  PRT
<213>  Artificial sequence

<220>
<223>  First linker L1: C4

<400>  11

Gly Gly Gly Gly Ser Gly Gly Gly Gly Ser Gly Gly Gly Gly Ser Gly
1               5                   10                  15

Gly Gly Gly Ser Gly Gly Gly Gly Ser Val Ala Pro Pro Pro Ala Leu
            20                  25                  30

Pro Ala Pro Val Arg Leu Pro Gly Pro Ala Val Ala Pro Pro Pro Ala
        35                  40                  45

Leu Pro Ala Val Ala Pro Pro Pro Ala Leu Pro Ala
    50                  55                  60

<210>  12
<211>  63
<212>  PRT
<213>  Artificial sequence

<220>
<223>  First linker L1: C5

<400>  12

Gly Gly Gly Gly Ser Gly Gly Gly Gly Ser Gly Gly Gly Gly Ser Gly
1               5                   10                  15

Gly Gly Gly Ser Gly Gly Gly Gly Ser Val Ala Pro Pro Pro Ala Leu
            20                  25                  30

Pro Ala Val Ala Pro Pro Pro Ala Leu Pro Ala Gly Ser Val Ala Pro
        35                  40                  45

Pro Pro Ala Leu Pro Ala Val Ala Pro Pro Pro Ala Leu Pro Ala
    50                  55                  60

<210>  13
<211>  25
<212>  PRT
<213>  Artificial sequence

<220>
<223>  First linker L1: GS

<400>  13

Gly Gly Gly Gly Ser Gly Gly Gly Gly Ser Gly Gly Gly Gly Ser Gly
1               5                   10                  15

Gly Gly Gly Ser Gly Gly Gly Gly Ser
            20                  25

<210>  14
<211>  5
<212>  PRT

<213>  Artificial sequence

<220>
<223>  Second linker L2

<400>  14

Gly Gly Gly Gly Ser
1               5


<210>  15
<211>  10
<212>  PRT
<213>  Artificial sequence

<220>
<223>  Second linker L2

<400>  15

Gly Gly Gly Gly Ser Gly Gly Gly Gly Ser
1               5                   10


<210>  16
<211>  10
<212>  PRT
<213>  Artificial sequence

<220>
<223>  Second linker L2

<400>  16

Gly Ser Gly Gly Ser Gly Gly Gly Gly Ser
1               5                   10


<210>  17
<211>  715
<212>  PRT
<213>  Artificial sequence

<220>
<223>  PACE/Furin 1-715

<400>  17

Met Glu Leu Arg Pro Trp Leu Leu Trp Val Val Ala Ala Thr Gly Thr
1               5                   10                  15


Leu Val Leu Leu Ala Ala Asp Ala Gln Gly Gln Lys Val Phe Thr Asn
            20                  25                  30


Thr Trp Ala Val Arg Ile Pro Gly Gly Pro Ala Val Ala Asn Ser Val
        35                  40                  45


Ala Arg Lys His Gly Phe Leu Asn Leu Gly Gln Ile Phe Gly Asp Tyr
    50                  55                  60

Tyr His Phe Trp His Arg Gly Val Thr Lys Arg Ser Leu Ser Pro His
65                  70                  75                  80

Arg Pro Arg His Ser Arg Leu Gln Arg Glu Pro Gln Val Gln Trp Leu
                85                  90                  95

Glu Gln Gln Val Ala Lys Arg Arg Thr Lys Arg Asp Val Tyr Gln Glu
            100                 105                 110

Pro Thr Asp Pro Lys Phe Pro Gln Gln Trp Tyr Leu Ser Gly Val Thr
        115                 120                 125

Gln Arg Asp Leu Asn Val Lys Ala Ala Trp Ala Gln Gly Tyr Thr Gly
    130                 135                 140

His Gly Ile Val Val Ser Ile Leu Asp Asp Gly Ile Glu Lys Asn His
145                 150                 155                 160

Pro Asp Leu Ala Gly Asn Tyr Asp Pro Gly Ala Ser Phe Asp Val Asn
                165                 170                 175

Asp Gln Asp Pro Asp Pro Gln Pro Arg Tyr Thr Gln Met Asn Asp Asn
            180                 185                 190

Arg His Gly Thr Arg Cys Ala Gly Glu Val Ala Ala Val Ala Asn Asn
        195                 200                 205

Gly Val Cys Gly Val Gly Val Ala Tyr Asn Ala Arg Ile Gly Gly Val
    210                 215                 220

Arg Met Leu Asp Gly Glu Val Thr Asp Ala Val Glu Ala Arg Ser Leu
225                 230                 235                 240

Gly Leu Asn Pro Asn His Ile His Ile Tyr Ser Ala Ser Trp Gly Pro
                245                 250                 255

Glu Asp Asp Gly Lys Thr Val Asp Gly Pro Ala Arg Leu Ala Glu Glu
            260                 265                 270

Ala Phe Phe Arg Gly Val Ser Gln Gly Arg Gly Gly Leu Gly Ser Ile
        275                 280                 285

Phe Val Trp Ala Ser Gly Asn Gly Gly Arg Glu His Asp Ser Cys Asn
    290                 295                 300

Cys Asp Gly Tyr Thr Asn Ser Ile Tyr Thr Leu Ser Ile Ser Ser Ala

305                 310                 315                 320

Thr Gln Phe Gly Asn Val Pro Trp Tyr Ser Glu Ala Cys Ser Ser Thr
                325                 330                 335

Leu Ala Thr Thr Tyr Ser Ser Gly Asn Gln Asn Glu Lys Gln Ile Val
            340                 345                 350

Thr Thr Asp Leu Arg Gln Lys Cys Thr Glu Ser His Thr Gly Thr Ser
        355                 360                 365

Ala Ser Ala Pro Leu Ala Ala Gly Ile Ile Ala Leu Thr Leu Glu Ala
    370                 375                 380

Asn Lys Asn Leu Thr Trp Arg Asp Met Gln His Leu Val Val Gln Thr
385                 390                 395                 400

Ser Lys Pro Ala His Leu Asn Ala Asn Asp Trp Ala Thr Asn Gly Val
                405                 410                 415

Gly Arg Lys Val Ser His Ser Tyr Gly Tyr Gly Leu Leu Asp Ala Gly
            420                 425                 430

Ala Met Val Ala Leu Ala Gln Asn Trp Thr Thr Val Ala Pro Gln Arg
        435                 440                 445

Lys Cys Ile Ile Asp Ile Leu Thr Glu Pro Lys Asp Ile Gly Lys Arg
    450                 455                 460

Leu Glu Val Arg Lys Thr Val Thr Ala Cys Leu Gly Glu Pro Asn His
465                 470                 475                 480

Ile Thr Arg Leu Glu His Ala Gln Ala Arg Leu Thr Leu Ser Tyr Asn
                485                 490                 495

Arg Arg Gly Asp Leu Ala Ile His Leu Val Ser Pro Met Gly Thr Arg
            500                 505                 510

Ser Thr Leu Leu Ala Ala Arg Pro His Asp Tyr Ser Ala Asp Gly Phe
        515                 520                 525

Asn Asp Trp Ala Phe Met Thr Thr His Ser Trp Asp Glu Asp Pro Ser
    530                 535                 540

Gly Glu Trp Val Leu Glu Ile Glu Asn Thr Ser Glu Ala Asn Asn Tyr
545                 550                 555                 560

Gly Thr Leu Thr Lys Phe Thr Leu Val Leu Tyr Gly Thr Ala Pro Glu
                565                 570                 575

Gly Leu Pro Val Pro Pro Glu Ser Ser Gly Cys Lys Thr Leu Thr Ser
            580                 585                 590

Ser Gln Ala Cys Val Val Cys Glu Glu Gly Phe Ser Leu His Gln Lys
        595                 600                 605

Ser Cys Val Gln His Cys Pro Pro Gly Phe Ala Pro Gln Val Leu Asp
    610                 615                 620

Thr His Tyr Ser Thr Glu Asn Asp Val Glu Thr Ile Arg Ala Ser Val
625                 630                 635                 640

Cys Ala Pro Cys His Ala Ser Cys Ala Thr Cys Gln Gly Pro Ala Leu
                645                 650                 655

Thr Asp Cys Leu Ser Cys Pro Ser His Ala Ser Leu Asp Pro Val Glu
            660                 665                 670

Gln Thr Cys Ser Arg Gln Ser Gln Ser Ser Arg Glu Ser Pro Pro Gln
        675                 680                 685

Gln Gln Pro Pro Arg Leu Pro Pro Glu Val Glu Ala Gly Gln Arg Leu
    690                 695                 700

Arg Ala Gly Leu Leu Pro Ser His Leu Pro Glu
705                 710                 715

<210>  18
<211>  746
<212>  PRT
<213>  Artificial sequence

<220>
<223>  FIX-C1-Fc-L2-P

<400>  18

Met Gln Arg Val Asn Met Ile Met Ala Glu Ser Pro Gly Leu Ile Thr
1               5                   10                  15

Ile Cys Leu Leu Gly Tyr Leu Leu Ser Ala Glu Cys Thr Val Phe Leu
            20                  25                  30

Asp His Glu Asn Ala Asn Lys Ile Leu Asn Arg Pro Lys Arg Tyr Asn
        35                  40                  45

Ser Gly Lys Leu Glu Glu Phe Val Gln Gly Asn Leu Glu Arg Glu Cys

50                  55                  60

Met Glu Glu Lys Cys Ser Phe Glu Glu Ala Arg Glu Val Phe Glu Asn
65                  70                  75                  80

Thr Glu Arg Thr Thr Glu Phe Trp Lys Gln Tyr Val Asp Gly Asp Gln
                85                  90                  95

Cys Glu Ser Asn Pro Cys Leu Asn Gly Gly Ser Cys Lys Asp Asp Ile
            100                 105                 110

Asn Ser Tyr Glu Cys Trp Cys Pro Phe Gly Phe Glu Gly Lys Asn Cys
        115                 120                 125

Glu Leu Asp Val Thr Cys Asn Ile Lys Asn Gly Arg Cys Glu Gln Phe
    130                 135                 140

Cys Lys Asn Ser Ala Asp Asn Lys Val Val Cys Ser Cys Thr Glu Gly
145                 150                 155                 160

Tyr Arg Leu Ala Glu Asn Gln Lys Ser Cys Glu Pro Ala Val Pro Phe
                165                 170                 175

Pro Cys Gly Arg Val Ser Val Ser Gln Thr Ser Lys Leu Thr Arg Ala
            180                 185                 190

Glu Thr Val Phe Pro Asp Val Asp Tyr Val Asn Ser Thr Glu Ala Glu
        195                 200                 205

Thr Ile Leu Asp Asn Ile Thr Gln Ser Thr Gln Ser Phe Asn Asp Phe
    210                 215                 220

Thr Arg Val Val Gly Gly Glu Asp Ala Lys Pro Gly Gln Phe Pro Trp
225                 230                 235                 240

Gln Val Val Leu Asn Gly Lys Val Asp Ala Phe Cys Gly Gly Ser Ile
                245                 250                 255

Val Asn Glu Lys Trp Ile Val Thr Ala Ala His Cys Val Glu Thr Gly
            260                 265                 270

Val Lys Ile Thr Val Val Ala Gly Glu His Asn Ile Glu Glu Thr Glu
        275                 280                 285

His Thr Glu Gln Lys Arg Asn Val Ile Arg Ile Ile Pro His His Asn
    290                 295                 300

Tyr Asn Ala Ala Ile Asn Lys Tyr Asn His Asp Ile Ala Leu Leu Glu
305                 310                 315                 320

Leu Asp Glu Pro Leu Val Leu Asn Ser Tyr Val Thr Pro Ile Cys Ile
                325                 330                 335

Ala Asp Lys Glu Tyr Thr Asn Ile Phe Leu Lys Phe Gly Ser Gly Tyr
            340                 345                 350

Val Ser Gly Trp Gly Arg Val Phe His Lys Gly Arg Ser Ala Leu Val
        355                 360                 365

Leu Gln Tyr Leu Arg Val Pro Leu Val Asp Arg Ala Thr Cys Leu Arg
    370                 375                 380

Ser Thr Lys Phe Thr Ile Tyr Asn Asn Met Phe Cys Ala Gly Phe His
385                 390                 395                 400

Glu Gly Gly Arg Asp Ser Cys Gln Gly Asp Ser Gly Gly Pro His Val
                405                 410                 415

Thr Glu Val Glu Gly Thr Ser Phe Leu Thr Gly Ile Ile Ser Trp Gly
            420                 425                 430

Glu Glu Cys Ala Met Lys Gly Lys Tyr Gly Ile Tyr Thr Lys Val Ser
        435                 440                 445

Arg Tyr Val Asn Trp Ile Lys Glu Lys Thr Lys Leu Thr Gly Gly Gly
    450                 455                 460

Gly Ser Gly Gly Gly Gly Ser Gly Gly Gly Gly Ser Gly Gly Gly Gly
465                 470                 475                 480

Ser Gly Gly Gly Gly Ser Val Ala Pro Pro Pro Ala Leu Pro Ala Pro
                485                 490                 495

Val Arg Leu Pro Gly Pro Ala Asp Lys Thr His Thr Cys Pro Pro Cys
            500                 505                 510

Pro Ala Pro Glu Leu Leu Gly Gly Pro Ser Val Phe Leu Phe Pro Pro
        515                 520                 525

Lys Pro Lys Asp Thr Leu Met Ile Ser Arg Thr Pro Glu Val Thr Cys
    530                 535                 540

Val Val Val Asp Val Ser His Glu Asp Pro Glu Val Lys Phe Asn Trp
545                 550                 555                 560

Tyr Val Asp Gly Val Glu Val His Asn Ala Lys Thr Lys Pro Arg Glu
                565                 570                 575

Glu Gln Tyr Ala Ser Thr Tyr Arg Val Val Ser Val Leu Thr Val Leu
            580                 585                 590

His Gln Asp Trp Leu Asn Gly Lys Glu Tyr Lys Cys Lys Val Ser Asn
        595                 600                 605

Lys Ala Leu Pro Ala Pro Ile Glu Lys Thr Ile Ser Lys Ala Lys Gly
    610                 615                 620

Gln Pro Arg Glu Pro Gln Val Tyr Thr Leu Pro Pro Ser Arg Asp Glu
625                 630                 635                 640

Leu Thr Lys Asn Gln Val Ser Leu Thr Cys Leu Val Lys Gly Phe Tyr
                645                 650                 655

Pro Ser Asp Ile Ala Val Glu Trp Glu Ser Asn Gly Gln Pro Glu Asn
            660                 665                 670

Asn Tyr Lys Thr Thr Pro Pro Val Leu Asp Ser Asp Gly Ser Phe Phe
        675                 680                 685

Leu Tyr Ser Lys Leu Thr Val Asp Lys Ser Arg Trp Gln Gln Gly Asn
    690                 695                 700

Val Phe Ser Cys Ser Val Met His Glu Ala Leu His Asn His Tyr Thr
705                 710                 715                 720

Gln Lys Ser Leu Ser Leu Ser Pro Gly Lys Gly Ser Gly Gly Ser Gly
                725                 730                 735

Gly Gly Gly Ser Leu Pro Glu Thr Gly Gly
            740                 745

<210>  19
<211>  2241
<212>  DNA
<213>  Artificial sequence

<220>
<223>  FIX-C1-Fc-L2-P

<400>  19
atgcagcgcg tcaatatgat tatggccgag tctccaggcc tcattaccat ttgtctgctg      60

ggctacctgc tgtccgcaga gtgtaccgtc ttcctcgacc acgagaacgc aaataagatt     120

ctcaataggc ctaagcgcta caattccgga aagctcgaag agtttgtcca gggaaatctg     180

gagcgcgagt gtatggagga aaagtgtagc tttgaagaag ctagagaggt gtttgagaat 240
actgagcgca ctactgagtt ttggaagcag tatgtggatg gcgatcagtg cgagtccaat 300
ccatgcctga atggcggatc ttgcaaggat gatattaatt cttacgagtg ttggtgccca 360
ttcggctttg agggaaagaa ttgcgaactg gatgtcacat gtaacatcaa gaacggcaga 420
tgcgagcagt tttgtaagaa cagcgccgat aataaggtgg tgtgtagttg tactgaggga 480
taccgcctcg cagagaatca gaagtcctgt gagcccgctg tgccatttcc ttgtggccgg 540
gtcagcgtca gccagacttc taagctgaca cgcgccgaga ccgtctttcc agatgtggat 600
tatgtcaact ctactgaggc cgagaccatt ctcgataata ttactcagtc tacacagtcc 660
tttaacgact tcactcgggt ggtgggcgga gaggacgcaa agcctggcca gtttccttgg 720
caggtcgtgc tcaacggaaa ggtggacgca ttttgtggcg gatctatcgt gaatgagaag 780
tggattgtga ccgccgctca ctgcgtcgag acaggcgtca agattaccgt ggtcgctgga 840
gagcacaaca ttgaggaaac agagcacacc gagcagaagc gcaatgtgat tcgcatcatc 900
ccacaccaca actacaacgc cgctattaat aagtacaacc acgacatcgc cctgttagaa 960
ctagatgagc ctctggtcct gaatagttac gtcactccta tttgtatcgc cgataaagaa 1020
tacactaaca tctttctcaa gttcggctct ggctatgtat ctggatgggg acgggtgttt 1080
cacaagggac ggtccgcact cgtgctacaa tacctccggg tgccactggt ggatcgggca 1140
acatgcctcc ggtctaccaa gtttactata tacaataaca tgttttgcgc cggctttcac 1200
gagggcggac gggatagttg ccagggcgat agtggaggcc ctcatgtcac agaggtggag 1260
ggaactagct ttctgacagg cattatctct tggggcgaag agtgcgctat gaagggaaag 1320
tacggcatct atactaaggt ctcgcgttac gtcaattgga tcaaggaaaa gacaaagctg 1380
acaggcggcg gagggagcgg cggcggaggc tctggcggcg gcggatctgg cggaggcggc 1440
tctggcggag gcggatccgt cgctccacca cctgcactcc ccgcccctgt cagactgcct 1500
ggacccgctg ataagaccca cacatgtcca ccttgcccag cacctgagct gctgggcgga 1560
cctagcgtgt ttctgttccc accaaagcca aaggacaccc tcatgattag tagaacccca 1620
gaggtgactt gcgtcgtggt cgatgtcagt cacgaggacc ccgaggtgaa gtttaattgg 1680
tatgtagatg gcgtcgaggt gcacaacgct aagaccaagc cacgcgaaga gcagtacgca 1740
tctacttacc gggtcgtctc tgtgctgact gtgctgcacc aggattggct caatggcaaa 1800
gagtacaagt gtaaggtcag taataaggcc ctccccgcac ctattgaaaa gactattagc 1860
aaggcaaagg gccagcctag ggagccccag gtctacaccc tgcccccttc ccgcgatgag 1920
ctgactaaga atcaggtctc tctcacttgc ctcgtgaagg gcttttaccc atccgacatt 1980
gccgtggagt gggagtccaa cggacagcca gagaataact acaagacaac tcctcccgtg 2040

ctcgatagcg acggttcatt cttcctgtac tccaagctca ccgttgataa gtctcggtgg 2100

cagcagggaa atgtgttctc ttgttccgtc atgcacgagg cactgcacaa tcactacacc 2160

cagaagtccc tgtccctcag ccctggcaaa ggcagcggcg gatctggagg cggcggatcc 2220

ctcccagaga ccggcggatg a 2241

<210> 20
<211> 746
<212> PRT
<213> Artificial sequence

<220>
<223> FIX-C2-Fc-L2-P

<400> 20

Met Gln Arg Val Asn Met Ile Met Ala Glu Ser Pro Gly Leu Ile Thr
1               5                   10                  15

Ile Cys Leu Leu Gly Tyr Leu Leu Ser Ala Glu Cys Thr Val Phe Leu
            20                  25                  30

Asp His Glu Asn Ala Asn Lys Ile Leu Asn Arg Pro Lys Arg Tyr Asn
        35                  40                  45

Ser Gly Lys Leu Glu Glu Phe Val Gln Gly Asn Leu Glu Arg Glu Cys
    50                  55                  60

Met Glu Glu Lys Cys Ser Phe Glu Glu Ala Arg Glu Val Phe Glu Asn
65                  70                  75                  80

Thr Glu Arg Thr Thr Glu Phe Trp Lys Gln Tyr Val Asp Gly Asp Gln
                85                  90                  95

Cys Glu Ser Asn Pro Cys Leu Asn Gly Gly Ser Cys Lys Asp Asp Ile
            100                 105                 110

Asn Ser Tyr Glu Cys Trp Cys Pro Phe Gly Phe Glu Gly Lys Asn Cys
        115                 120                 125

Glu Leu Asp Val Thr Cys Asn Ile Lys Asn Gly Arg Cys Glu Gln Phe
    130                 135                 140

Cys Lys Asn Ser Ala Asp Asn Lys Val Val Cys Ser Cys Thr Glu Gly
145                 150                 155                 160

Tyr Arg Leu Ala Glu Asn Gln Lys Ser Cys Glu Pro Ala Val Pro Phe
                165                 170                 175

Pro Cys Gly Arg Val Ser Val Ser Gln Thr Ser Lys Leu Thr Arg Ala
        180                 185                 190

Glu Thr Val Phe Pro Asp Val Asp Tyr Val Asn Ser Thr Glu Ala Glu
        195                 200                 205

Thr Ile Leu Asp Asn Ile Thr Gln Ser Thr Gln Ser Phe Asn Asp Phe
    210                 215                 220

Thr Arg Val Val Gly Gly Glu Asp Ala Lys Pro Gly Gln Phe Pro Trp
225                 230                 235                 240

Gln Val Val Leu Asn Gly Lys Val Asp Ala Phe Cys Gly Gly Ser Ile
                245                 250                 255

Val Asn Glu Lys Trp Ile Val Thr Ala Ala His Cys Val Glu Thr Gly
            260                 265                 270

Val Lys Ile Thr Val Val Ala Gly Glu His Asn Ile Glu Glu Thr Glu
        275                 280                 285

His Thr Glu Gln Lys Arg Asn Val Ile Arg Ile Ile Pro His His Asn
    290                 295                 300

Tyr Asn Ala Ala Ile Asn Lys Tyr Asn His Asp Ile Ala Leu Leu Glu
305                 310                 315                 320

Leu Asp Glu Pro Leu Val Leu Asn Ser Tyr Val Thr Pro Ile Cys Ile
                325                 330                 335

Ala Asp Lys Glu Tyr Thr Asn Ile Phe Leu Lys Phe Gly Ser Gly Tyr
            340                 345                 350

Val Ser Gly Trp Gly Arg Val Phe His Lys Gly Arg Ser Ala Leu Val
        355                 360                 365

Leu Gln Tyr Leu Arg Val Pro Leu Val Asp Arg Ala Thr Cys Leu Arg
    370                 375                 380

Ser Thr Lys Phe Thr Ile Tyr Asn Asn Met Phe Cys Ala Gly Phe His
385                 390                 395                 400

Glu Gly Gly Arg Asp Ser Cys Gln Gly Asp Ser Gly Gly Pro His Val
                405                 410                 415

Thr Glu Val Glu Gly Thr Ser Phe Leu Thr Gly Ile Ile Ser Trp Gly
            420                 425                 430

Glu Glu Cys Ala Met Lys Gly Lys Tyr Gly Ile Tyr Thr Lys Val Ser
        435                 440                 445

Arg Tyr Val Asn Trp Ile Lys Glu Lys Thr Lys Leu Thr Gly Gly Gly
    450                 455                 460

Gly Ser Gly Gly Gly Gly Ser Gly Gly Gly Gly Ser Gly Gly Gly Gly
465                 470                 475                 480

Ser Gly Gly Gly Gly Ser Val Ala Pro Pro Pro Ala Leu Pro Ala Val
                485                 490                 495

Ala Pro Pro Pro Ala Leu Pro Ala Asp Lys Thr His Thr Cys Pro Pro
            500                 505                 510

Cys Pro Ala Pro Glu Leu Leu Gly Gly Pro Ser Val Phe Leu Phe Pro
        515                 520                 525

Pro Lys Pro Lys Asp Thr Leu Met Ile Ser Arg Thr Pro Glu Val Thr
    530                 535                 540

Cys Val Val Val Asp Val Ser His Glu Asp Pro Glu Val Lys Phe Asn
545                 550                 555                 560

Trp Tyr Val Asp Gly Val Glu Val His Asn Ala Lys Thr Lys Pro Arg
                565                 570                 575

Glu Glu Gln Tyr Ala Ser Thr Tyr Arg Val Val Ser Val Leu Thr Val
            580                 585                 590

Leu His Gln Asp Trp Leu Asn Gly Lys Glu Tyr Lys Cys Lys Val Ser
        595                 600                 605

Asn Lys Ala Leu Pro Ala Pro Ile Glu Lys Thr Ile Ser Lys Ala Lys
    610                 615                 620

Gly Gln Pro Arg Glu Pro Gln Val Tyr Thr Leu Pro Pro Ser Arg Asp
625                 630                 635                 640

Glu Leu Thr Lys Asn Gln Val Ser Leu Thr Cys Leu Val Lys Gly Phe
                645                 650                 655

Tyr Pro Ser Asp Ile Ala Val Glu Trp Glu Ser Asn Gly Gln Pro Glu
            660                 665                 670

Asn Asn Tyr Lys Thr Thr Pro Pro Val Leu Asp Ser Asp Gly Ser Phe
        675                 680                 685

Phe Leu Tyr Ser Lys Leu Thr Val Asp Lys Ser Arg Trp Gln Gln Gly
    690                 695                 700

Asn Val Phe Ser Cys Ser Val Met His Glu Ala Leu His Asn His Tyr
705                 710                 715                 720

Thr Gln Lys Ser Leu Ser Leu Ser Pro Gly Gly Ser Gly Gly Ser Gly
                725                 730                 735

Gly Gly Gly Ser Leu Pro Glu Thr Gly Gly
            740                 745

<210>  21
<211>  2241
<212>  DNA
<213>  Artificial sequence

<220>
<223>  FIX-C2-Fc-L2-P

<400>  21
atgcagcgcg tcaatatgat tatggccgag tctccaggcc tcattaccat ttgtctgctg      60

ggctacctgc tgtccgcaga gtgtaccgtc ttcctcgacc acgagaacgc aaataagatt     120

ctcaataggc ctaagcgcta caattccgga aagctcgaag agtttgtcca gggaaatctg     180

gagcgcgagt gtatggagga aaagtgtagc tttgaagaag ctagagaggt gtttgagaat     240

actgagcgca ctactgagtt ttggaagcag tatgtggatg gcgatcagtg cgagtccaat     300

ccatgcctga atggcggatc ttgcaaggat gatattaatt cttacgagtg ttggtgccca     360

ttcggctttg agggaaagaa ttgcgaactg gatgtcacat gtaacatcaa gaacggcaga     420

tgcgagcagt tttgtaagaa cagcgccgat aataaggtgg tgtgtagttg tactgaggga     480

taccgcctcg cagagaatca gaagtcctgt gagcccgctg tgccatttcc ttgtggccgg     540

gtcagcgtca gccagacttc taagctgaca cgcgccgaga ccgtctttcc agatgtggat     600

tatgtcaact ctactgaggc cgagaccatt ctcgataata ttactcagtc tacacagtcc     660

tttaacgact tcactcgggt ggtgggcgga gaggacgcaa agcctggcca gtttccttgg     720

caggtcgtgc tcaacggaaa ggtggacgca ttttgtggcg gatctatcgt gaatgagaag     780

tggattgtga ccgccgctca ctgcgtcgag acaggcgtca agattaccgt ggtcgctgga     840

gagcacaaca ttgaggaaac agagcacacc gagcagaagc gcaatgtgat tcgcatcatc     900

ccacaccaca actacaacgc cgctattaat aagtacaacc acgacatcgc cctgttagaa     960

ctagatgagc ctctggtcct gaatagttac gtcactccta tttgtatcgc cgataaagaa    1020

tacactaaca tctttctcaa gttcggctct ggctatgtat ctggatgggg acgggtgttt    1080

cacaagggac ggtccgcact cgtgctacaa tacctccggg tgccactggt ggatcgggca 1140

acatgcctcc ggtctaccaa gtttactata tacaataaca tgttttgcgc cggctttcac 1200

gagggcggac gggatagttg ccagggcgat agtggaggcc ctcatgtcac agaggtggag 1260

ggaactagct ttctgacagg cattatctct tggggcgaag agtgcgctat gaagggaaag 1320

tacggcatct atactaaggt ctcgcgttac gtcaattgga tcaaggaaaa gacaaagctg 1380

acaggcggcg gagggagcgg cggcggaggc tctggcggcg gcggatctgg cggaggcggc 1440

tctggcggag gcggatccgt cgctccacca cctgcactcc ccgccgtggc tcctccacct 1500

gctttgcccg ctgataagac ccacacatgt ccaccttgcc cagcacctga gctgctgggc 1560

ggacctagcg tgtttctgtt cccaccaaag ccaaaggaca ccctcatgat tagtagaacc 1620

ccagaggtga cttgcgtcgt ggtcgatgtc agtcacgagg accccgaggt gaagtttaat 1680

tggtatgtag atggcgtcga ggtgcacaac gctaagacca agccacgcga agagcagtac 1740

gcatctactt accgggtcgt ctctgtgctg actgtgctgc accaggattg gctcaatggc 1800

aaagagtaca agtgtaaggt cagtaataag gccctccccg cacctattga aaagactatt 1860

agcaaggcaa agggccagcc tagggagccc caggtctaca ccctgccccc ttcccgcgat 1920

gagctgacta agaatcaggt ctctctcact tgcctcgtga agggctttta cccatccgac 1980

attgccgtgg agtgggagtc caacggacag ccagagaata actacaagac aactcctccc 2040

gtgctcgata gcgacggttc attcttcctg tactccaagc tcaccgttga taagtctcgg 2100

tggcagcagg gaaatgtgtt ctcttgttcc gtcatgcacg aggcactgca caatcactac 2160

acccagaagt ccctgtccct cagccctggc ggcagcggcg gatctggagg cggcggatct 2220

ctcccagaga ccggcggata a 2241

<210> 22
<211> 764
<212> PRT
<213> Artificial sequence

<220>
<223> FIX-C3-Fc-L2-P

<400> 22

Met Gln Arg Val Asn Met Ile Met Ala Glu Ser Pro Gly Leu Ile Thr
1               5                   10                  15

Ile Cys Leu Leu Gly Tyr Leu Leu Ser Ala Glu Cys Thr Val Phe Leu
            20                  25                  30

Asp His Glu Asn Ala Asn Lys Ile Leu Asn Arg Pro Lys Arg Tyr Asn
        35                  40                  45

Ser Gly Lys Leu Glu Glu Phe Val Gln Gly Asn Leu Glu Arg Glu Cys
    50                  55                  60

Met Glu Glu Lys Cys Ser Phe Glu Glu Ala Arg Glu Val Phe Glu Asn
65                  70                  75                  80

Thr Glu Arg Thr Thr Glu Phe Trp Lys Gln Tyr Val Asp Gly Asp Gln
                85                  90                  95

Cys Glu Ser Asn Pro Cys Leu Asn Gly Gly Ser Cys Lys Asp Asp Ile
            100                 105                 110

Asn Ser Tyr Glu Cys Trp Cys Pro Phe Gly Phe Glu Gly Lys Asn Cys
        115                 120                 125

Glu Leu Asp Val Thr Cys Asn Ile Lys Asn Gly Arg Cys Glu Gln Phe
    130                 135                 140

Cys Lys Asn Ser Ala Asp Asn Lys Val Val Cys Ser Cys Thr Glu Gly
145                 150                 155                 160

Tyr Arg Leu Ala Glu Asn Gln Lys Ser Cys Glu Pro Ala Val Pro Phe
                165                 170                 175

Pro Cys Gly Arg Val Ser Val Ser Gln Thr Ser Lys Leu Thr Arg Ala
            180                 185                 190

Glu Thr Val Phe Pro Asp Val Asp Tyr Val Asn Ser Thr Glu Ala Glu
        195                 200                 205

Thr Ile Leu Asp Asn Ile Thr Gln Ser Thr Gln Ser Phe Asn Asp Phe
    210                 215                 220

Thr Arg Val Val Gly Gly Glu Asp Ala Lys Pro Gly Gln Phe Pro Trp
225                 230                 235                 240

Gln Val Val Leu Asn Gly Lys Val Asp Ala Phe Cys Gly Gly Ser Ile
                245                 250                 255

Val Asn Glu Lys Trp Ile Val Thr Ala Ala His Cys Val Glu Thr Gly
            260                 265                 270

Val Lys Ile Thr Val Val Ala Gly Glu His Asn Ile Glu Glu Thr Glu
        275                 280                 285

His Thr Glu Gln Lys Arg Asn Val Ile Arg Ile Ile Pro His His Asn
    290                 295                 300

Tyr Asn Ala Ala Ile Asn Lys Tyr Asn His Asp Ile Ala Leu Leu Glu
305                 310                 315                 320

Leu Asp Glu Pro Leu Val Leu Asn Ser Tyr Val Thr Pro Ile Cys Ile
                325                 330                 335

Ala Asp Lys Glu Tyr Thr Asn Ile Phe Leu Lys Phe Gly Ser Gly Tyr
            340                 345                 350

Val Ser Gly Trp Gly Arg Val Phe His Lys Gly Arg Ser Ala Leu Val
        355                 360                 365

Leu Gln Tyr Leu Arg Val Pro Leu Val Asp Arg Ala Thr Cys Leu Arg
    370                 375                 380

Ser Thr Lys Phe Thr Ile Tyr Asn Asn Met Phe Cys Ala Gly Phe His
385                 390                 395                 400

Glu Gly Gly Arg Asp Ser Cys Gln Gly Asp Ser Gly Gly Pro His Val
                405                 410                 415

Thr Glu Val Glu Gly Thr Ser Phe Leu Thr Gly Ile Ile Ser Trp Gly
            420                 425                 430

Glu Glu Cys Ala Met Lys Gly Lys Tyr Gly Ile Tyr Thr Lys Val Ser
        435                 440                 445

Arg Tyr Val Asn Trp Ile Lys Glu Lys Thr Lys Leu Thr Gly Gly Gly
    450                 455                 460

Gly Ser Gly Gly Gly Gly Ser Gly Gly Gly Gly Ser Gly Gly Gly Gly
465                 470                 475                 480

Ser Gly Gly Gly Gly Ser Val Ala Pro Pro Pro Ala Leu Pro Ala Val
                485                 490                 495

Ala Pro Pro Pro Ala Leu Pro Ala Val Ala Pro Pro Pro Ala Leu Pro
            500                 505                 510

Ala Pro Val Arg Leu Pro Gly Pro Ala Asp Lys Thr His Thr Cys Pro
        515                 520                 525

Pro Cys Pro Ala Pro Glu Leu Leu Gly Gly Pro Ser Val Phe Leu Phe
    530                 535                 540

Pro Pro Lys Pro Lys Asp Thr Leu Met Ile Ser Arg Thr Pro Glu Val
545                 550                 555                 560

Thr Cys Val Val Val Asp Val Ser His Glu Asp Pro Glu Val Lys Phe
                565                 570                 575

Asn Trp Tyr Val Asp Gly Val Glu Val His Asn Ala Lys Thr Lys Pro
            580                 585                 590

Arg Glu Glu Gln Tyr Ala Ser Thr Tyr Arg Val Val Ser Val Leu Thr
        595                 600                 605

Val Leu His Gln Asp Trp Leu Asn Gly Lys Glu Tyr Lys Cys Lys Val
    610                 615                 620

Ser Asn Lys Ala Leu Pro Ala Pro Ile Glu Lys Thr Ile Ser Lys Ala
625                 630                 635                 640

Lys Gly Gln Pro Arg Glu Pro Gln Val Tyr Thr Leu Pro Pro Ser Arg
                645                 650                 655

Asp Glu Leu Thr Lys Asn Gln Val Ser Leu Thr Cys Leu Val Lys Gly
            660                 665                 670

Phe Tyr Pro Ser Asp Ile Ala Val Glu Trp Glu Ser Asn Gly Gln Pro
        675                 680                 685

Glu Asn Asn Tyr Lys Thr Thr Pro Pro Val Leu Asp Ser Asp Gly Ser
    690                 695                 700

Phe Phe Leu Tyr Ser Lys Leu Thr Val Asp Lys Ser Arg Trp Gln Gln
705                 710                 715                 720

Gly Asn Val Phe Ser Cys Ser Val Met His Glu Ala Leu His Asn His
                725                 730                 735

Tyr Thr Gln Lys Ser Leu Ser Leu Ser Pro Gly Lys Gly Ser Gly Gly
            740                 745                 750

Ser Gly Gly Gly Gly Ser Leu Pro Glu Thr Gly Gly
        755                 760

<210>  23
<211>  2295
<212>  DNA
<213>  Artificial sequence

<220>
<223>  FIX-C3-Fc-L2-P

<400>  23

atgcagcgcg tcaatatgat tatggccgag tctccaggcc tcattaccat ttgtctgctg 60

ggctacctgc tgtccgcaga gtgtaccgtc ttcctcgacc acgagaacgc aaataagatt 120

ctcaataggc ctaagcgcta caattccgga aagctcgaag agtttgtcca gggaaatctg 180

gagcgcgagt gtatggagga aaagtgtagc tttgaagaag ctagagaggt gtttgagaat 240

actgagcgca ctactgagtt ttggaagcag tatgtggatg gcgatcagtg cgagtccaat 300

ccatgcctga atggcggatc ttgcaaggat gatattaatt cttacgagtg ttggtgccca 360

ttcggctttg agggaaagaa ttgcgaactg gatgtcacat gtaacatcaa gaacggcaga 420

tgcgagcagt tttgtaagaa cagcgccgat aataaggtgg tgtgtagttg tactgaggga 480

taccgcctcg cagagaatca gaagtcctgt gagcccgctg tgccatttcc ttgtggccgg 540

gtcagcgtca gccagacttc taagctgaca cgcgccgaga ccgtctttcc agatgtggat 600

tatgtcaact ctactgaggc cgagaccatt ctcgataata ttactcagtc tacacagtcc 660

tttaacgact tcactcgggt ggtgggcgga gaggacgcaa agcctggcca gtttccttgg 720

caggtcgtgc tcaacggaaa ggtggacgca ttttgtggcg gatctatcgt gaatgagaag 780

tggattgtga ccgccgctca ctgcgtcgag acaggcgtca agattaccgt ggtcgctgga 840

gagcacaaca ttgaggaaac agagcacacc gagcagaagc gcaatgtgat tcgcatcatc 900

ccacaccaca actacaacgc cgctattaat aagtacaacc acgacatcgc cctgttagaa 960

ctagatgagc ctctggtcct gaatagttac gtcactccta tttgtatcgc cgataaagaa 1020

tacactaaca tctttctcaa gttcggctct ggctatgtat ctggatgggg acgggtgttt 1080

cacaagggac ggtccgcact cgtgctacaa tacctccggg tgccactggt ggatcgggca 1140

acatgcctcc ggtctaccaa gtttactata tacaataaca tgttttgcgc cggctttcac 1200

gagggcggac gggatagttg ccagggcgat agtggaggcc ctcatgtcac agaggtggag 1260

ggaactagct ttctgacagg cattatctct tggggcgaag agtgcgctat gaagggaaag 1320

tacggcatct atactaaggt ctcgcgttac gtcaattgga tcaaggaaaa gacaaagctg 1380

acaggcggcg gagggagcgg cggcggaggc tctggcggcg gcggatctgg cggaggcggc 1440

tctggcggag gcggatctgt cgctccacca cctgcactcc ccgccgtggc tcctccacct 1500

gctttgcccg ctgtcgctcc accacctgca ctccccgccc ctgtcagact gcctggaccc 1560

gctgataaga cccacacatg tccaccttgc ccagcacctg agctgctggg cggacctagc 1620

gtgtttctgt tcccaccaaa gccaaaggac accctcatga ttagtagaac cccagaggtg 1680

acttgcgtcg tggtcgatgt cagtcacgag gaccccgagg tgaagtttaa ttggtatgta 1740

gatggcgtcg aggtgcacaa cgctaagacc aagccacgcg aagagcagta cgcatctact 1800

taccgggtcg tctctgtgct gactgtgctg caccaggatt ggctcaatgg caaagagtac 1860

aagtgtaagg tcagtaataa ggccctcccc gcacctattg aaaagactat tagcaaggca 1920

aagggccagc ctagggagcc ccaggtctac accctgcccc cttcccgcga tgagctgact 1980

aagaatcagg tctctctcac ttgcctcgtg aagggctttt acccatccga cattgccgtg 2040

gagtgggagt ccaacggaca gccagagaat aactacaaga caactcctcc cgtgctcgat 2100

agcgacggtt cattcttcct gtactccaag ctcaccgttg ataagtctcg gtggcagcag 2160

ggaaatgtgt tctcttgttc cgtcatgcac gaggcactgc acaatcacta cacccagaag 2220

tccctgtccc tcagccctgg caaaggcagc ggcggatctg gaggcggcgg atccctccca 2280

gagaccggcg gatga 2295

<210> 24
<211> 763
<212> PRT
<213> Artificial sequence

<220>
<223> FIX-C4-Fc-L2-P

<400> 24

Met Gln Arg Val Asn Met Ile Met Ala Glu Ser Pro Gly Leu Ile Thr
1               5                   10                  15

Ile Cys Leu Leu Gly Tyr Leu Leu Ser Ala Glu Cys Thr Val Phe Leu
            20                  25                  30

Asp His Glu Asn Ala Asn Lys Ile Leu Asn Arg Pro Lys Arg Tyr Asn
        35                  40                  45

Ser Gly Lys Leu Glu Glu Phe Val Gln Gly Asn Leu Glu Arg Glu Cys
    50                  55                  60

Met Glu Glu Lys Cys Ser Phe Glu Glu Ala Arg Glu Val Phe Glu Asn
65                  70                  75                  80

Thr Glu Arg Thr Thr Glu Phe Trp Lys Gln Tyr Val Asp Gly Asp Gln
                85                  90                  95

Cys Glu Ser Asn Pro Cys Leu Asn Gly Gly Ser Cys Lys Asp Asp Ile
            100                 105                 110

Asn Ser Tyr Glu Cys Trp Cys Pro Phe Gly Phe Glu Gly Lys Asn Cys
        115                 120                 125

Glu Leu Asp Val Thr Cys Asn Ile Lys Asn Gly Arg Cys Glu Gln Phe
    130                 135                 140

Cys Lys Asn Ser Ala Asp Asn Lys Val Val Cys Ser Cys Thr Glu Gly

145                 150                 155                 160

Tyr Arg Leu Ala Glu Asn Gln Lys Ser Cys Glu Pro Ala Val Pro Phe
                165                 170                 175

Pro Cys Gly Arg Val Ser Val Ser Gln Thr Ser Lys Leu Thr Arg Ala
            180                 185                 190

Glu Thr Val Phe Pro Asp Val Asp Tyr Val Asn Ser Thr Glu Ala Glu
        195                 200                 205

Thr Ile Leu Asp Asn Ile Thr Gln Ser Thr Gln Ser Phe Asn Asp Phe
    210                 215                 220

Thr Arg Val Val Gly Gly Glu Asp Ala Lys Pro Gly Gln Phe Pro Trp
225                 230                 235                 240

Gln Val Val Leu Asn Gly Lys Val Asp Ala Phe Cys Gly Gly Ser Ile
                245                 250                 255

Val Asn Glu Lys Trp Ile Val Thr Ala Ala His Cys Val Glu Thr Gly
            260                 265                 270

Val Lys Ile Thr Val Val Ala Gly Glu His Asn Ile Glu Glu Thr Glu
        275                 280                 285

His Thr Glu Gln Lys Arg Asn Val Ile Arg Ile Ile Pro His His Asn
    290                 295                 300

Tyr Asn Ala Ala Ile Asn Lys Tyr Asn His Asp Ile Ala Leu Leu Glu
305                 310                 315                 320

Leu Asp Glu Pro Leu Val Leu Asn Ser Tyr Val Thr Pro Ile Cys Ile
                325                 330                 335

Ala Asp Lys Glu Tyr Thr Asn Ile Phe Leu Lys Phe Gly Ser Gly Tyr
            340                 345                 350

Val Ser Gly Trp Gly Arg Val Phe His Lys Gly Arg Ser Ala Leu Val
        355                 360                 365

Leu Gln Tyr Leu Arg Val Pro Leu Val Asp Arg Ala Thr Cys Leu Arg
    370                 375                 380

Ser Thr Lys Phe Thr Ile Tyr Asn Asn Met Phe Cys Ala Gly Phe His
385                 390                 395                 400

Glu Gly Gly Arg Asp Ser Cys Gln Gly Asp Ser Gly Gly Pro His Val
                405                 410                 415

Thr Glu Val Glu Gly Thr Ser Phe Leu Thr Gly Ile Ile Ser Trp Gly
            420                 425                 430

Glu Glu Cys Ala Met Lys Gly Lys Tyr Gly Ile Tyr Thr Lys Val Ser
        435                 440                 445

Arg Tyr Val Asn Trp Ile Lys Glu Lys Thr Lys Leu Thr Gly Gly Gly
    450                 455                 460

Gly Ser Gly Gly Gly Gly Ser Gly Gly Gly Gly Ser Gly Gly Gly Gly
465                 470                 475                 480

Ser Gly Gly Gly Gly Ser Val Ala Pro Pro Pro Ala Leu Pro Ala Pro
                485                 490                 495

Val Arg Leu Pro Gly Pro Ala Val Ala Pro Pro Pro Ala Leu Pro Ala
            500                 505                 510

Val Ala Pro Pro Pro Ala Leu Pro Ala Asp Lys Thr His Thr Cys Pro
        515                 520                 525

Pro Cys Pro Ala Pro Glu Leu Leu Gly Gly Pro Ser Val Phe Leu Phe
    530                 535                 540

Pro Pro Lys Pro Lys Asp Thr Leu Met Ile Ser Arg Thr Pro Glu Val
545                 550                 555                 560

Thr Cys Val Val Val Asp Val Ser His Glu Asp Pro Glu Val Lys Phe
                565                 570                 575

Asn Trp Tyr Val Asp Gly Val Glu Val His Asn Ala Lys Thr Lys Pro
            580                 585                 590

Arg Glu Glu Gln Tyr Ala Ser Thr Tyr Arg Val Val Ser Val Leu Thr
        595                 600                 605

Val Leu His Gln Asp Trp Leu Asn Gly Lys Glu Tyr Lys Cys Lys Val
    610                 615                 620

Ser Asn Lys Ala Leu Pro Ala Pro Ile Glu Lys Thr Ile Ser Lys Ala
625                 630                 635                 640

Lys Gly Gln Pro Arg Glu Pro Gln Val Tyr Thr Leu Pro Pro Ser Arg
                645                 650                 655

Asp Glu Leu Thr Lys Asn Gln Val Ser Leu Thr Cys Leu Val Lys Gly
            660                 665                 670

Phe Tyr Pro Ser Asp Ile Ala Val Glu Trp Glu Ser Asn Gly Gln Pro
        675                 680                 685

Glu Asn Asn Tyr Lys Thr Thr Pro Pro Val Leu Asp Ser Asp Gly Ser
    690                 695                 700

Phe Phe Leu Tyr Ser Lys Leu Thr Val Asp Lys Ser Arg Trp Gln Gln
705                 710                 715                 720

Gly Asn Val Phe Ser Cys Ser Val Met His Glu Ala Leu His Asn His
                725                 730                 735

Tyr Thr Gln Lys Ser Leu Ser Leu Ser Pro Gly Gly Ser Gly Gly Ser
            740                 745                 750

Gly Gly Gly Gly Ser Leu Pro Glu Thr Gly Gly
        755                 760

<210>  25
<211>  2292
<212>  DNA
<213>  Artificial sequence

<220>
<223>  FIX-C4-Fc-L2-P

<400>  25
atgcagcgcg tcaatatgat tatggccgag tctccaggcc tcattaccat ttgtctgctg      60

ggctacctgc tgtccgcaga gtgtaccgtc ttcctcgacc acgagaacgc aaataagatt     120

ctcaataggc ctaagcgcta caattccgga aagctcgaag agtttgtcca gggaaatctg     180

gagcgcgagt gtatggagga aaagtgtagc tttgaagaag ctagagaggt gtttgagaat     240

actgagcgca ctactgagtt ttggaagcag tatgtggatg gcgatcagtg cgagtccaat     300

ccatgcctga atggcggatc ttgcaaggat gatattaatt cttacgagtg ttggtgccca     360

ttcggctttg agggaaagaa ttgcgaactg gatgtcacat gtaacatcaa gaacggcaga     420

tgcgagcagt tttgtaagaa cagcgccgat aataaggtgg tgtgtagttg tactgaggga     480

taccgcctcg cagagaatca gaagtcctgt gagcccgctg tgccatttcc ttgtggccgg     540

gtcagcgtca gccagacttc taagctgaca cgcgccgaga ccgtctttcc agatgtggat     600

tatgtcaact ctactgaggc cgagaccatt ctcgataata ttactcagtc tacacagtcc     660

tttaacgact tcactcgggt ggtgggcgga gaggacgcaa agcctggcca gtttccttgg     720

caggtcgtgc tcaacggaaa ggtggacgca ttttgtggcg gatctatcgt gaatgagaag     780

tggattgtga ccgccgctca ctgcgtcgag acaggcgtca agattaccgt ggtcgctgga 840

gagcacaaca ttgaggaaac agagcacacc gagcagaagc gcaatgtgat tcgcatcatc 900

ccacaccaca actacaacgc cgctattaat aagtacaacc acgacatcgc cctgttagaa 960

ctagatgagc ctctggtcct gaatagttac gtcactccta tttgtatcgc cgataaagaa 1020

tacactaaca tctttctcaa gttcggctct ggctatgtat ctggatgggg acgggtgttt 1080

cacaagggac ggtccgcact cgtgctacaa tacctccggg tgccactggt ggatcgggca 1140

acatgcctcc ggtctaccaa gtttactata tacaataaca tgttttgcgc cggctttcac 1200

gagggcggac gggatagttg ccagggcgat agtggaggcc ctcatgtcac agaggtggag 1260

ggaactagct ttctgacagg cattatctct tggggcgaag agtgcgctat gaagggaaag 1320

tacggcatct atactaaggt ctcgcgttac gtcaattgga tcaaggaaaa gacaaagctg 1380

acaggcggcg gagggagcgg cggcggaggc tctggcggcg gcggatctgg cggaggcggc 1440

tctggcggag gcggatctgt cgctccacca cctgcactcc ccgcccctgt cagactgcct 1500

ggacccgctg tcgctccacc acctgcactc cccgccgtgg ctcctccacc tgctttgccc 1560

gctgataaga cccacacatg tccaccttgc ccagcacctg agctgctggg cggacctagc 1620

gtgtttctgt tcccaccaaa gccaaaggac accctcatga ttagtagaac cccagaggtg 1680

acttgcgtcg tggtcgatgt cagtcacgag gaccccgagg tgaagtttaa ttggtatgta 1740

gatggcgtcg aggtgcacaa cgctaagacc aagccacgcg aagagcagta cgcatctact 1800

taccgggtcg tctctgtgct gactgtgctg caccaggatt ggctcaatgg caaagagtac 1860

aagtgtaagg tcagtaataa ggccctcccc gcacctattg aaaagactat tagcaaggca 1920

aagggccagc ctagggagcc ccaggtctac accctgcccc cttcccgcga tgagctgact 1980

aagaatcagg tctctctcac ttgcctcgtg aagggctttt acccatccga cattgccgtg 2040

gagtgggagt ccaacggaca gccagagaat aactacaaga caactcctcc cgtgctcgat 2100

agcgacggtt cattcttcct gtactccaag ctcaccgttg ataagtctcg gtggcagcag 2160

ggaaatgtgt tctcttgttc cgtcatgcac gaggcactgc acaatcacta cacccagaag 2220

tccctgtccc tcagccctgg cggcagcggc ggatctggag gcggcggatc tctcccagag 2280

accggcggat ga 2292

<210> 26
<211> 766
<212> PRT
<213> Artificial sequence

<220>
<223> FIX-C5-Fc-L2-P

<400> 26

Met Gln Arg Val Asn Met Ile Met Ala Glu Ser Pro Gly Leu Ile Thr
1               5                   10                  15

Ile Cys Leu Leu Gly Tyr Leu Leu Ser Ala Glu Cys Thr Val Phe Leu
            20                  25                  30

Asp His Glu Asn Ala Asn Lys Ile Leu Asn Arg Pro Lys Arg Tyr Asn
        35                  40                  45

Ser Gly Lys Leu Glu Glu Phe Val Gln Gly Asn Leu Glu Arg Glu Cys
    50                  55                  60

Met Glu Glu Lys Cys Ser Phe Glu Glu Ala Arg Glu Val Phe Glu Asn
65                  70                  75                  80

Thr Glu Arg Thr Thr Glu Phe Trp Lys Gln Tyr Val Asp Gly Asp Gln
                85                  90                  95

Cys Glu Ser Asn Pro Cys Leu Asn Gly Gly Ser Cys Lys Asp Asp Ile
            100                 105                 110

Asn Ser Tyr Glu Cys Trp Cys Pro Phe Gly Phe Glu Gly Lys Asn Cys
        115                 120                 125

Glu Leu Asp Val Thr Cys Asn Ile Lys Asn Gly Arg Cys Glu Gln Phe
    130                 135                 140

Cys Lys Asn Ser Ala Asp Asn Lys Val Val Cys Ser Cys Thr Glu Gly
145                 150                 155                 160

Tyr Arg Leu Ala Glu Asn Gln Lys Ser Cys Glu Pro Ala Val Pro Phe
                165                 170                 175

Pro Cys Gly Arg Val Ser Val Ser Gln Thr Ser Lys Leu Thr Arg Ala
            180                 185                 190

Glu Thr Val Phe Pro Asp Val Asp Tyr Val Asn Ser Thr Glu Ala Glu
        195                 200                 205

Thr Ile Leu Asp Asn Ile Thr Gln Ser Thr Gln Ser Phe Asn Asp Phe
    210                 215                 220

Thr Arg Val Val Gly Gly Glu Asp Ala Lys Pro Gly Gln Phe Pro Trp
225                 230                 235                 240

Gln Val Val Leu Asn Gly Lys Val Asp Ala Phe Cys Gly Gly Ser Ile
                245                 250                 255

Val Asn Glu Lys Trp Ile Val Thr Ala Ala His Cys Val Glu Thr Gly
            260                 265                 270

Val Lys Ile Thr Val Val Ala Gly Glu His Asn Ile Glu Glu Thr Glu
        275                 280                 285

His Thr Glu Gln Lys Arg Asn Val Ile Arg Ile Ile Pro His His Asn
    290                 295                 300

Tyr Asn Ala Ala Ile Asn Lys Tyr Asn His Asp Ile Ala Leu Leu Glu
305                 310                 315                 320

Leu Asp Glu Pro Leu Val Leu Asn Ser Tyr Val Thr Pro Ile Cys Ile
                325                 330                 335

Ala Asp Lys Glu Tyr Thr Asn Ile Phe Leu Lys Phe Gly Ser Gly Tyr
            340                 345                 350

Val Ser Gly Trp Gly Arg Val Phe His Lys Gly Arg Ser Ala Leu Val
        355                 360                 365

Leu Gln Tyr Leu Arg Val Pro Leu Val Asp Arg Ala Thr Cys Leu Arg
    370                 375                 380

Ser Thr Lys Phe Thr Ile Tyr Asn Asn Met Phe Cys Ala Gly Phe His
385                 390                 395                 400

Glu Gly Gly Arg Asp Ser Cys Gln Gly Asp Ser Gly Gly Pro His Val
                405                 410                 415

Thr Glu Val Glu Gly Thr Ser Phe Leu Thr Gly Ile Ile Ser Trp Gly
            420                 425                 430

Glu Glu Cys Ala Met Lys Gly Lys Tyr Gly Ile Tyr Thr Lys Val Ser
        435                 440                 445

Arg Tyr Val Asn Trp Ile Lys Glu Lys Thr Lys Leu Thr Gly Gly Gly
    450                 455                 460

Gly Ser Gly Gly Gly Gly Ser Gly Gly Gly Gly Ser Gly Gly Gly Gly
465                 470                 475                 480

Ser Gly Gly Gly Gly Ser Val Ala Pro Pro Pro Ala Leu Pro Ala Val
                485                 490                 495

Ala Pro Pro Pro Ala Leu Pro Ala Gly Ser Val Ala Pro Pro Pro Ala

500                 505                 510

Leu Pro Ala Val Ala Pro Pro Pro Ala Leu Pro Ala Asp Lys Thr His
        515                 520                 525

Thr Cys Pro Pro Cys Pro Ala Pro Glu Leu Leu Gly Gly Pro Ser Val
    530                 535                 540

Phe Leu Phe Pro Pro Lys Pro Lys Asp Thr Leu Met Ile Ser Arg Thr
545                 550                 555                 560

Pro Glu Val Thr Cys Val Val Val Asp Val Ser His Glu Asp Pro Glu
                565                 570                 575

Val Lys Phe Asn Trp Tyr Val Asp Gly Val Glu Val His Asn Ala Lys
            580                 585                 590

Thr Lys Pro Arg Glu Glu Gln Tyr Ala Ser Thr Tyr Arg Val Val Ser
        595                 600                 605

Val Leu Thr Val Leu His Gln Asp Trp Leu Asn Gly Lys Glu Tyr Lys
    610                 615                 620

Cys Lys Val Ser Asn Lys Ala Leu Pro Ala Pro Ile Glu Lys Thr Ile
625                 630                 635                 640

Ser Lys Ala Lys Gly Gln Pro Arg Glu Pro Gln Val Tyr Thr Leu Pro
                645                 650                 655

Pro Ser Arg Asp Glu Leu Thr Lys Asn Gln Val Ser Leu Thr Cys Leu
            660                 665                 670

Val Lys Gly Phe Tyr Pro Ser Asp Ile Ala Val Glu Trp Glu Ser Asn
        675                 680                 685

Gly Gln Pro Glu Asn Asn Tyr Lys Thr Thr Pro Pro Val Leu Asp Ser
    690                 695                 700

Asp Gly Ser Phe Phe Leu Tyr Ser Lys Leu Thr Val Asp Lys Ser Arg
705                 710                 715                 720

Trp Gln Gln Gly Asn Val Phe Ser Cys Ser Val Met His Glu Ala Leu
                725                 730                 735

His Asn His Tyr Thr Gln Lys Ser Leu Ser Leu Ser Pro Gly Gly Ser
            740                 745                 750

Gly Gly Ser Gly Gly Gly Gly Ser Leu Pro Glu Thr Gly Gly
        755                 760                 765

<210>  27
<211>  2301
<212>  DNA
<213>  Artificial sequence

<220>
<223>  FIX-C5-Fc-L2-P

<400>  27
atgcagcgcg tcaatatgat tatggccgag tctccaggcc tcattaccat ttgtctgctg      60

ggctacctgc tgtccgcaga gtgtaccgtc ttcctcgacc acgagaacgc aaataagatt     120

ctcaataggc ctaagcgcta caattccgga aagctcgaag agtttgtcca gggaaatctg     180

gagcgcgagt gtatggagga aaagtgtagc tttgaagaag ctagagaggt gtttgagaat     240

actgagcgca ctactgagtt ttggaagcag tatgtggatg gcgatcagtg cgagtccaat     300

ccatgcctga atggcggatc ttgcaaggat gatattaatt cttacgagtg ttggtgccca     360

ttcggctttg agggaaagaa ttgcgaactg gatgtcacat gtaacatcaa gaacggcaga     420

tgcgagcagt tttgtaagaa cagcgccgat aataaggtgg tgtgtagttg tactgaggga     480

taccgcctcg cagagaatca gaagtcctgt gagcccgctg tgccatttcc ttgtggccgg     540

gtcagcgtca gccagacttc taagctgaca cgcgccgaga ccgtctttcc agatgtggat     600

tatgtcaact ctactgaggc cgagaccatt ctcgataata ttactcagtc tacacagtcc     660

tttaacgact tcactcgggt ggtgggcgga gaggacgcaa agcctggcca gtttccttgg     720

caggtcgtgc tcaacggaaa ggtggacgca ttttgtggcg gatctatcgt gaatgagaag     780

tggattgtga ccgccgctca ctgcgtcgag acaggcgtca agattaccgt ggtcgctgga     840

gagcacaaca ttgaggaaac agagcacacc gagcagaagc gcaatgtgat tcgcatcatc     900

ccacaccaca actacaacgc cgctattaat aagtacaacc acgacatcgc cctgttagaa     960

ctagatgagc ctctggtcct gaatagttac gtcactccta tttgtatcgc cgataaagaa    1020

tacactaaca tctttctcaa gttcggctct ggctatgtat ctggatgggg acgggtgttt    1080

cacaagggac ggtccgcact cgtgctacaa tacctccggg tgccactggt ggatcgggca    1140

acatgcctcc ggtctaccaa gtttactata tacaataaca tgttttgcgc cggctttcac    1200

gagggcggac gggatagttg ccagggcgat agtggaggcc ctcatgtcac agaggtggag    1260

ggaactagct ttctgacagg cattatctct tggggcgaag agtgcgctat gaagggaaag    1320

tacggcatct atactaaggt ctcgcgttac gtcaattgga tcaaggaaaa gacaaagctg    1380

acaggcggcg gagggagcgg cggcggaggc tctggcggcg gcggatctgg cggaggcggc    1440

tctggcggag gcggatctgt cgctccacca cctgcactcc ccgccgtggc tcctccacct    1500

gctttgcccg ctggatccgt cgctccacca cctgcactcc ccgccgtggc tcctccacct 1560

gctttgcccg ctgataagac ccacacatgt ccaccttgcc cagcacctga gctgctgggc 1620

ggacctagcg tgtttctgtt cccaccaaag ccaaaggaca ccctcatgat tagtagaacc 1680

ccagaggtga cttgcgtcgt ggtcgatgtc agtcacgagg accccgaggt gaagtttaat 1740

tggtatgtag atggcgtcga ggtgcacaac gctaagacca agccacgcga agagcagtac 1800

gcatctactt accgggtcgt ctctgtgctg actgtgctgc accaggattg gctcaatggc 1860

aaagagtaca agtgtaaggt cagtaataag gccctccccg cacctattga aaagactatt 1920

agcaaggcaa agggccagcc tagggagccc caggtctaca ccctgccccc ttcccgcgat 1980

gagctgacta agaatcaggt ctctctcact tgcctcgtga agggctttta cccatccgac 2040

attgccgtgg agtgggagtc caacggacag ccagagaata actacaagac aactcctccc 2100

gtgctcgata gcgacggttc attcttcctg tactccaagc tcaccgttga taagtctcgg 2160

tggcagcagg gaaatgtgtt ctcttgttcc gtcatgcacg aggcactgca caatcactac 2220

acccagaagt ccctgtccct cagccctggc ggcagcggcg gatctggagg cggcggatct 2280

ctcccagaga ccggcggata a 2301

<210> 28
<211> 728
<212> PRT
<213> Artificial sequence

<220>
<223> FIX-GS-Fc-L2-P

<400> 28

Met Gln Arg Val Asn Met Ile Met Ala Glu Ser Pro Gly Leu Ile Thr
1               5                   10                  15

Ile Cys Leu Leu Gly Tyr Leu Leu Ser Ala Glu Cys Thr Val Phe Leu
            20                  25                  30

Asp His Glu Asn Ala Asn Lys Ile Leu Asn Arg Pro Lys Arg Tyr Asn
        35                  40                  45

Ser Gly Lys Leu Glu Glu Phe Val Gln Gly Asn Leu Glu Arg Glu Cys
    50                  55                  60

Met Glu Glu Lys Cys Ser Phe Glu Glu Ala Arg Glu Val Phe Glu Asn
65                  70                  75                  80

Thr Glu Arg Thr Thr Glu Phe Trp Lys Gln Tyr Val Asp Gly Asp Gln
                85                  90                  95

Cys Glu Ser Asn Pro Cys Leu Asn Gly Gly Ser Cys Lys Asp Asp Ile
        100                 105                 110

Asn Ser Tyr Glu Cys Trp Cys Pro Phe Gly Phe Glu Gly Lys Asn Cys
    115                 120                 125

Glu Leu Asp Val Thr Cys Asn Ile Lys Asn Gly Arg Cys Glu Gln Phe
    130                 135                 140

Cys Lys Asn Ser Ala Asp Asn Lys Val Val Cys Ser Cys Thr Glu Gly
145                 150                 155                 160

Tyr Arg Leu Ala Glu Asn Gln Lys Ser Cys Glu Pro Ala Val Pro Phe
                165                 170                 175

Pro Cys Gly Arg Val Ser Val Ser Gln Thr Ser Lys Leu Thr Arg Ala
        180                 185                 190

Glu Thr Val Phe Pro Asp Val Asp Tyr Val Asn Ser Thr Glu Ala Glu
    195                 200                 205

Thr Ile Leu Asp Asn Ile Thr Gln Ser Thr Gln Ser Phe Asn Asp Phe
    210                 215                 220

Thr Arg Val Val Gly Gly Glu Asp Ala Lys Pro Gly Gln Phe Pro Trp
225                 230                 235                 240

Gln Val Val Leu Asn Gly Lys Val Asp Ala Phe Cys Gly Gly Ser Ile
                245                 250                 255

Val Asn Glu Lys Trp Ile Val Thr Ala Ala His Cys Val Glu Thr Gly
        260                 265                 270

Val Lys Ile Thr Val Val Ala Gly Glu His Asn Ile Glu Glu Thr Glu
    275                 280                 285

His Thr Glu Gln Lys Arg Asn Val Ile Arg Ile Ile Pro His His Asn
    290                 295                 300

Tyr Asn Ala Ala Ile Asn Lys Tyr Asn His Asp Ile Ala Leu Leu Glu
305                 310                 315                 320

Leu Asp Glu Pro Leu Val Leu Asn Ser Tyr Val Thr Pro Ile Cys Ile
                325                 330                 335

Ala Asp Lys Glu Tyr Thr Asn Ile Phe Leu Lys Phe Gly Ser Gly Tyr
        340                 345                 350

Val Ser Gly Trp Gly Arg Val Phe His Lys Gly Arg Ser Ala Leu Val
        355                 360                 365

Leu Gln Tyr Leu Arg Val Pro Leu Val Asp Arg Ala Thr Cys Leu Arg
    370                 375                 380

Ser Thr Lys Phe Thr Ile Tyr Asn Asn Met Phe Cys Ala Gly Phe His
385                 390                 395                 400

Glu Gly Gly Arg Asp Ser Cys Gln Gly Asp Ser Gly Gly Pro His Val
                405                 410                 415

Thr Glu Val Glu Gly Thr Ser Phe Leu Thr Gly Ile Ile Ser Trp Gly
            420                 425                 430

Glu Glu Cys Ala Met Lys Gly Lys Tyr Gly Ile Tyr Thr Lys Val Ser
        435                 440                 445

Arg Tyr Val Asn Trp Ile Lys Glu Lys Thr Lys Leu Thr Gly Gly Gly
    450                 455                 460

Gly Ser Gly Gly Gly Gly Ser Gly Gly Gly Gly Ser Gly Gly Gly Gly
465                 470                 475                 480

Ser Gly Gly Gly Gly Ser Asp Lys Thr His Thr Cys Pro Pro Cys Pro
                485                 490                 495

Ala Pro Glu Leu Leu Gly Gly Pro Ser Val Phe Leu Phe Pro Pro Lys
            500                 505                 510

Pro Lys Asp Thr Leu Met Ile Ser Arg Thr Pro Glu Val Thr Cys Val
        515                 520                 525

Val Val Asp Val Ser His Glu Asp Pro Glu Val Lys Phe Asn Trp Tyr
    530                 535                 540

Val Asp Gly Val Glu Val His Asn Ala Lys Thr Lys Pro Arg Glu Glu
545                 550                 555                 560

Gln Tyr Ala Ser Thr Tyr Arg Val Val Ser Val Leu Thr Val Leu His
                565                 570                 575

Gln Asp Trp Leu Asn Gly Lys Glu Tyr Lys Cys Lys Val Ser Asn Lys
            580                 585                 590

Ala Leu Pro Ala Pro Ile Glu Lys Thr Ile Ser Lys Ala Lys Gly Gln
        595                 600                 605

Pro Arg Glu Pro Gln Val Tyr Thr Leu Pro Pro Ser Arg Asp Glu Leu
    610                 615                 620

Thr Lys Asn Gln Val Ser Leu Thr Cys Leu Val Lys Gly Phe Tyr Pro
625                 630                 635                 640

Ser Asp Ile Ala Val Glu Trp Glu Ser Asn Gly Gln Pro Glu Asn Asn
                645                 650                 655

Tyr Lys Thr Thr Pro Pro Val Leu Asp Ser Asp Gly Ser Phe Phe Leu
            660                 665                 670

Tyr Ser Lys Leu Thr Val Asp Lys Ser Arg Trp Gln Gln Gly Asn Val
        675                 680                 685

Phe Ser Cys Ser Val Met His Glu Ala Leu His Asn His Tyr Thr Gln
    690                 695                 700

Lys Ser Leu Ser Leu Ser Pro Gly Gly Ser Gly Gly Ser Gly Gly Gly
705                 710                 715                 720

Gly Ser Leu Pro Glu Thr Gly Gly
                725

<210>  29
<211>  2187
<212>  DNA
<213>  Artificial sequence

<220>
<223>  FIX-GS-Fc-L2-P

<400>  29
atgcagcgcg tcaatatgat tatggccgag tctccaggcc tcattaccat ttgtctgctg      60

ggctacctgc tgtccgcaga gtgtaccgtc ttcctcgacc acgagaacgc aaataagatt     120

ctcaataggc ctaagcgcta caattccgga aagctcgaag agtttgtcca gggaaatctg     180

gagcgcgagt gtatggagga aaagtgtagc tttgaagaag ctagagaggt gtttgagaat     240

actgagcgca ctactgagtt ttggaagcag tatgtggatg gcgatcagtg cgagtccaat     300

ccatgcctga atggcggatc ttgcaaggat gatattaatt cttacgagtg ttggtgccca     360

ttcggctttg agggaaagaa ttgcgaactg gatgtcacat gtaacatcaa gaacggcaga     420

tgcgagcagt tttgtaagaa cagcgccgat aataaggtgg tgtgtagttg tactgaggga     480

taccgcctcg cagagaatca gaagtcctgt gagcccgctg tgccatttcc ttgtggccgg     540

gtcagcgtca gccagacttc taagctgaca cgcgccgaga ccgtctttcc agatgtggat     600

tatgtcaact ctactgaggc cgagaccatt ctcgataata ttactcagtc tacacagtcc 660

tttaacgact tcactcgggt ggtgggcgga gaggacgcaa agcctggcca gtttccttgg 720

caggtcgtgc tcaacggaaa ggtggacgca ttttgtggcg gatctatcgt gaatgagaag 780

tggattgtga ccgccgctca ctgcgtcgag acaggcgtca agattaccgt ggtcgctgga 840

gagcacaaca ttgaggaaac agagcacacc gagcagaagc gcaatgtgat tcgcatcatc 900

ccacaccaca actacaacgc cgctattaat aagtacaacc acgacatcgc cctgttagaa 960

ctagatgagc ctctggtcct gaatagttac gtcactccta tttgtatcgc cgataaagaa 1020

tacactaaca tctttctcaa gttcggctct ggctatgtat ctggatgggg acgggtgttt 1080

cacaagggac ggtccgcact cgtgctacaa tacctccggg tgccactggt ggatcgggca 1140

acatgcctcc ggtctaccaa gtttactata tacaataaca tgttttgcgc cggctttcac 1200

gagggcggac gggatagttg ccagggcgat agtggaggcc ctcatgtcac agaggtggag 1260

ggaactagct ttctgacagg cattatctct tggggcgaag agtgcgctat gaagggaaag 1320

tacggcatct atactaaggt ctcgcgttac gtcaattgga tcaaggaaaa gacaaagctg 1380

acaggcggcg gagggagcgg cggcggaggc tctggcggcg gcggatctgg cggaggcggc 1440

tctggcggag gcggatccga taagacccac acatgtccac cttgcccagc acctgagctg 1500

ctgggcggac ctagcgtgtt tctgttccca ccaaagccaa aggacaccct catgattagt 1560

agaaccccag aggtgacttg cgtcgtggtc gatgtcagtc acgaggaccc cgaggtgaag 1620

tttaattggt atgtagatgg cgtcgaggtg cacaacgcta agaccaagcc acgcgaagag 1680

cagtacgcat ctacttaccg ggtcgtctct gtgctgactg tgctgcacca ggattggctc 1740

aatggcaaag agtacaagtg taaggtcagt aataaggccc tccccgcacc tattgaaaag 1800

actattagca aggcaaaggg ccagcctagg gagccccagg tctacaccct gcccccttcc 1860

cgcgatgagc tgactaagaa tcaggtctct ctcacttgcc tcgtgaaggg cttttaccca 1920

tccgacattg ccgtggagtg ggagtccaac ggacagccag agaataacta caagacaact 1980

cctcccgtgc tcgatagcga cggttcattc ttcctgtact ccaagctcac cgttgataag 2040

tctcggtggc agcagggaaa tgtgttctct tgttccgtca tgcacgaggc actgcacaat 2100

cactacaccc agaagtccct gtccctcagc cctggcggca gcggcggatc tggaggcggc 2160

ggatctctcc cagagaccgg cggataa 2187

<210> 30
<211> 37
<212> DNA
<213> Artificial sequence

<220>
<223> F1

<400>  30
tcaggaagat ctcgcgaatt aattctgtgg aatgtgt                                37


<210>  31
<211>  36
<212>  DNA
<213>  Artificial sequence

<220>
<223>  R1

<400>  31
tatcctagat ctgctggcac gacaggtttc ccgact                                 36


<210>  32
<211>  87
<212>  DNA
<213>  Artificial sequence

<220>
<223>  F2

<400>  32
ttcgcggatc cgtcgctcca ccacctgcac tccccgccgt ggctcctcca cctgctttgc      60

ccgctgataa gacccacaca tgtccac                                           87


<210>  33
<211>  87
<212>  DNA
<213>  Artificial sequence

<220>
<223>  R2

<400>  33
taaccggaat tcattatccg ccggtctctg ggagagatcc gccgcctcca gatccgccgc      60

tgccgccagg gctgagggac agggact                                           87


<210>  34
<211>  48
<212>  DNA
<213>  Artificial sequence

<220>
<223>  F3

<400>  34
tggctcctcc acctgctttg cccgctgtcg ctccaccacc tgcactcc                    48


<210>  35
<211>  57
<212>  DNA
<213>  Artificial sequence

<220>
<223>  F4

<400>  35
tagatctgtc gctccaccac ctgcactccc cgccgtggct cctccacctg ctttgcc          57


<210>  36
<211>  31
<212>  DNA
<213>  Artificial sequence

<220>
<223>  R3

<400>  36
tgaattctta tcatccgccg gtctctggga g                                      31


<210>  37
<211>  47
<212>  DNA
<213>  Artificial sequence

<220>
<223>  F5

<400>  37
ccctgtcaga ctgcctggac ccgctgtcgc tccaccacct gcactcc                     47


<210>  38
<211>  54
<212>  DNA
<213>  Artificial sequence

<220>
<223>  F6

<400>  38
tagatctgtc gctccaccac ctgcactccc cgcccctgtc agactgcctg gacc             54


<210>  39
<211>  27
<212>  DNA
<213>  Artificial sequence

<220>
<223>  F7

<400>  39
atataagctt ccgccaccat gcagcgc                                           27


<210>  40
<211>  34
<212>  DNA
<213>  Artificial sequence

<220>
<223>  R4

<400>  40
atatggatcc agcgggcaaa gcaggtggag gagc                                   34

<210>  41
<211>  37
<212>  DNA
<213>  Artificial sequence

<220>
<223>  F8

<400>  41
gatttggatc cgataagacc cacacatgtc caccttg                                37


<210>  42
<211>  87
<212>  DNA
<213>  Artificial sequence

<220>
<223>  R5

<400>  42
taaccggaat tcattatccg ccggtctctg ggagagatcc gccgcctcca gatccgccgc      60

tgccgccagg gctgagggac agggact                                           87